# EUROPEAN PATENT APPLICATION

(11) **EP 2 374 790 A1**
(43) Date of publication of application: **12.10.2011**
(21) Application number: 09826157.1
(22) Date of filing: 13.11.2009
(51) Int. Cl.: C07C 233/11, A61K 31/165, A61P 25/08, C07C 233/58, C07C 235/34, C07C 255/57, C07C 317/44, C07C 317/46, C07C 323/62

(54) **BIPHENYLACETAMIDE DERIVATIVE**

(30) Priority: 14.11.2008 JP 2008292682
(71) Applicant: Dainippon Sumitomo Pharma Co., Ltd., Osaka 541-8524 (JP)
(72) Inventor: IWAMA, Seiji, Suita-shi Osaka 564-0053 (JP); TANAKA, Tomoyuki, Suita-shi Osaka 564-0053 (JP); YAJIMA, Nana, Suita-shi Osaka 564-0053 (JP)
(74) Representative: Webster, Jeremy Mark
(86) International application number: PCT/JP2009/069355
(87) International publication number: WO 2010/055911

(57) **Abstract**

The present invention provides a compound of formula (I) or a salt thereof, wherein R¹, R² and R³ are independently selected from the group consisting of hydrogen atom, fluorine atom, chlorine atom, bromine atom, C₁₋₆ alkyl, C₁₋₆ alkoxy substituted with fluorine atom, and others; R⁴ and R⁵ are independently selected from the group consisting of hydrogen atom, fluorine atom, chlorine atom, C₁₋₆ alkyl, C₁₋₆ alkoxy substituted with fluorine atom, and others; R⁶ and R⁷ are independently selected from the group consisting of hydrogen atom, fluorine atom, methyl, ethyl, hydroxy group, and others; and R⁸ and R⁹ are independently selected from the group consisting of hydrogen atom, C₁₋₆ alkyl, and others, which is useful as an agent for treating or preventing various types of epilepsy including partial seizures and/or generalized seizures.

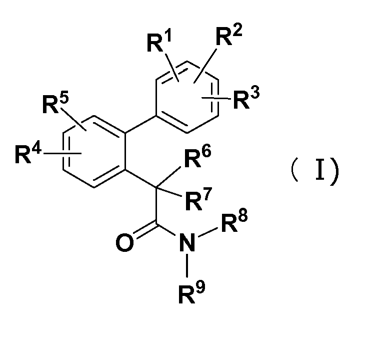

## Description

### TECHNICAL FIELD

The present invention relates to novel biphenylacetamide derivatives useful as a medicament for treating epilepsy.

### BACKGROUND ART

Epilepsy is a chronic disease caused by hyperexcitability of brain neurons, and the disease which comprises recurrent symptoms of paroxysmal abnormality in movement, consciousness and sensation, and behavior disorder. One third of the cases are intractable epilepsy resistant to conventional medicaments. Epileptic seizures are categorized into partial seizures and generalized seizures.
Partial seizures exhibit a behavioral abnormality and an aberrant electroencephalographic-pattern which originate at the localized part in either side of cerebral hemisphere. Partial seizures are further categorized into simple partial seizure, complex partial seizure, and secondarily generalized tonic-clonic seizure.
On the other hand, generalized seizures show synchronously in both cerebral hemispheres, i.e. it does not originate at a localized part of the cerebral hemisphere. The clinical symptom of generalized seizures is a characteristic motor symptom in whole body accompanied by loss of consciousness. When this seizures start, the aberration of electroencephalograms occurs synchronously at both cerebral hemispheres. Generalized seizures include, for example, absence seizure, atypical absence seizure, myoclonic seizure, tonic seizure, clonic seizure, tonic-clonic seizure, atonic seizure, west syndrome, and Lennox-Gastaut syndrome.

The above-mentioned seizures of epilepsy are usually treated by drug therapy. Various antiepileptic drugs have been used in patients thereof, but there are no antiepileptic drugs, except for valproic acid, which have a broad spectrum of efficacy against partial seizures and generalized seizures (especially, atypical absence seizure and myoclonic seizure). However, valproic acid is not a sufficient medicament from the viewpoints of efficacy and side effects, and as a result, there are still many refractory patients with some types of seizures to existing drugs. Accordingly, there is a great unmet medical need to provide a medicament which exhibits efficacy against a wide range of partial seizures and generalized seizures, shows efficacy for treating refractory epilepsy, and has improvement in safety.

The mechanism of actions in many existing antiepileptic drugs remains unclear exactly, thus it has been thought that the antiepileptic drugs act via various mechanisms in a complex way. In practice, some antiepileptic drugs are used for treating several neurological disorders and psychiatric disorders even though they were prepared as an antiepileptic drug in the first place (Pawel, D.Z et al., Non-epilepsy use of antiepileptic drugs, Pharmacological Reports, 2006, 58, 1-12). For example, except for lithium, there are no specific medicaments for stably treating bipolar disorder which exhibits dysthymia with recurrent manic states and depressive states (i.e. manic depression), but some antiepileptic drugs are allowed as a mood stabilizer and then they have also been used for treating bipolar mania. Carbamazepine had been used at first (Brambilla, P., Barrale, F., Soares, J.C., Perspectives on the use of anticonvulsants in the treatment of bipolar disorder, International Journal of Neuropsychopharmacology, 2001, 4, 421-446), and now, valproic acid has been used. Besides lithium, valproic acid is clinically used as a first-line agent for treating bipolar manic state (Angel, I and Horovitz, T., Bipolar disorder and valproic acid). However, there are no suitable medicaments for treating bipolar depression, and therefore it is desired to provide a novel medicament useful for treating both mania and depression.

Patent Reference 1 discloses biphenyl alkylamines having antiarrhythmic effects. The chemical structure thereof is depicted by the following formula: 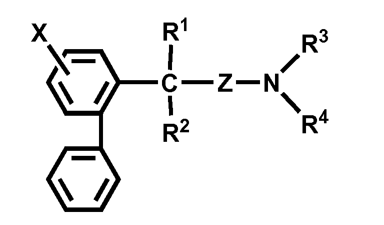 wherein
X is hydrogen or halogen;
R¹ is hydrogen, hydroxy group, CN, CONH₂ or COOR⁵, wherein R⁵ is hydrogen or lower alkyl;
R² is hydrogen or C₁₋₃ alkyl;
Z is -(CH₂)ₙ- or 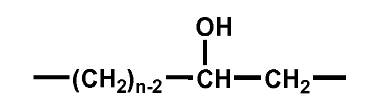 wherein n is 2, 3 or 4; and
R³ and R⁴ are independently selected from the group consisting of hydrogen, lower alkyl, lower alkenyl, phenylalkyl, and nitrogen atom, or R³ and R⁴ may be combined to form a ring structure selected from the group consisting of pyrrolidino, piperidino and morpholino.
The compound of Patent Reference 1 has at least three carbon atoms between the biphenyl and the amino group, and thus the chemical structure thereof is different from that of the compound of formula (I) hereinafter set forth.

Patent Reference 2 discloses benzene derivatives useful as an Hsp90 family protein inhibitor. The chemical structure thereof is depicted by the following formula: 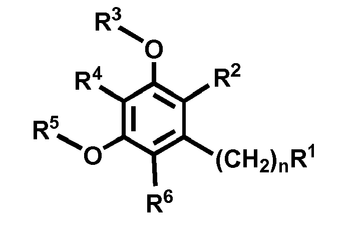 wherein
n is an integer of 0 to 10,
R¹ is hydrogen atom, hydroxy, cyano, carboxy, nitro, halogen, substituted or unsubstituted lower alkyl, -CONR⁷R⁸ or other substituents, wherein R⁷ and R⁸ are independently selected from the group consisting of hydrogen atom, substituted or unsubstituted lower alkyl and other substituents;
R² is substituted or unsubstituted lower alkyl, substituted or unsubstituted lower alkenyl, substituted or unsubstituted lower alkynyl, substituted or unsubstituted aryl, or other substituents;
R³ and R⁵ are independently selected from the group consisting of hydrogen atom, substituted or unsubstituted lower alkyl and other substituents; and
R⁴ and R⁶ are independently selected from the group consisting of hydrogen atom, hydroxy, halogen, cyano, substituted or unsubstituted aryl and other substituents.
The compound of Patent Reference 2 has hydroxy group or alkoxy group as an essential substituent on the benzene ring, and thus the chemical structure thereof is different from that of the compound of formula (I) hereinafter set forth.
[Patent Reference 1] US 4,277,471 B
[Patent Reference 2] EU 1,704,856 B

### DISCLOSURE OF INVENTION

### (Problems to Be Solved by Invention)

A problem to be solved by the present invention is to provide a novel compound useful as an antiepileptic drug.

### (Means to Solve the Problem)

The present inventors have extensively studied to solve the above problem and then have found that a novel compound of the following formula (A) exhibits a potent anticonvulsant action. Based upon the new findings, the present invention has been completed. The present invention provides biphenylacetamide derivatives of the following formula (A) (hereinafter, optionally referred to as "the compound of the present invention").

### Term 1

A compound of the following formula (A): 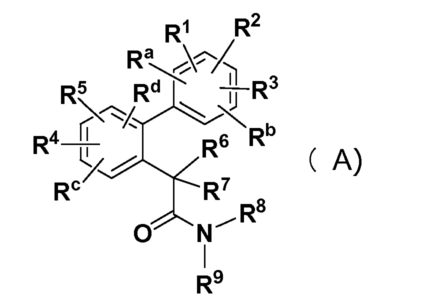 or a hydrate or a solvate thereof wherein
R¹, R² , R³, R^{a} and R^{b} are each bound to any one different carbon atom having a replaceable hydrogen atom in the benzene ring to which they are attached, and are independently selected from the group consisting of hydrogen atom, fluorine atom, chlorine atom, bromine atom, C₁₋₆ alkyl, C₁₋₆ alkoxy substituted with 1-3 fluorine atoms, C₁₋₆ alkyl-S(O)ₙ- and cyano, wherein said alkyl and alkylS(O)ₙ- may be substituted with 1-5 fluorine atoms, provided that when any two of R¹, R², R³, R^{a} and R^{b} are each 2'-methyl and 3'-methyl, at least one of the other substituents is other than hydrogen atom,
R⁴, R⁵, R^{c} and R^{d} are each bound to any one different carbon atom having a replaceable hydrogen atom in the benzene ring to which they are attached, and are independently selected from the group consisting of hydrogen atom, fluorine atom, chlorine atom, C₁₋₆ alkyl, C₁₋₆ alkoxy substituted with 1-3 fluorine atoms, C₁₋₆ alkylS(O)ₙ-, cyano and nitro, wherein said alkyl and alkyl - S(O)ₙ- may be substituted with 1-5 fluorine atoms,
R⁶ and R⁷ are independently selected from the group consisting of hydrogen atom, fluorine atom, methyl, ethyl, hydroxy group and C₁₋₆ alkoxy, provided that when one of R⁶ and R⁷ is hydroxy group, the other is not fluorine atom, hydroxy group or C₁₋₆ alkoxy, or R⁶ and R⁷ may be combined to form C₃₋₆ cycloalkyl with the carbon atom which they are attached to, wherein said methyl, ethyl, alkoxy and cycloalkyl may be substituted with 1-5 fluorine atoms,
R⁸ and R⁹ are independently selected from the group consisting of hydrogen atom, C₁₋₆ alkyl, C₃₋₇ cycloalkyl-C₁₋₃ alkyl and C₃₋₆ cycloalkyl, wherein said alkyl, cycloalkyl-alkyl and cycloalkyl may be substituted with 1-5 fluorine atoms,
n is an integer of 0 to 2,
provided that when R¹, R², R³, R⁴, R⁵, R^{a}, R^{b}, R^{c} and R^{d} are all hydrogen atoms, and R⁶ and R⁷ are both hydrogen atoms, at least one of R⁸ and R⁹ is hydrogen atom, and
when R¹, R², R³, R⁴, R⁵, R^{a}, R^{b}, R^{c} and R^{d} are all hydrogen atoms, and at least one of R⁶ and R⁷ is alkoxy, R⁸ and R⁹ are independently selected from the group consisting of hydrogen atom, C₁₋₃ alkyl, C₃₋₇ cycloalkyl-C₁₋₃ alkyl and C₃₋₆ cycloalkyl.

### Term 2

A compound of the following formula (I): 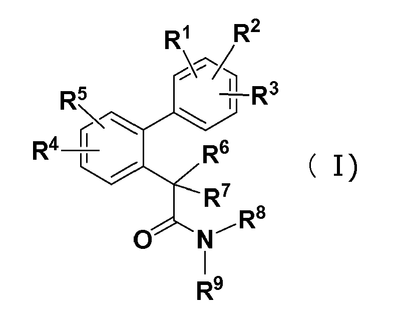 or a hydrate or a solvate thereof wherein
R¹, R² and R³ are each bound to any one different carbon atom having a replaceable hydrogen atom in the benzene ring to which they are attached, and are independently selected from the group consisting of hydrogen atom, fluorine atom, chlorine atom, bromine atom, C₁₋₆ alkyl, C₁₋₆ alkoxy substituted with 1-3 fluorine atoms, C₁₋₆ alkyl-S(O)ₙ- and cyano, wherein said alkyl and alkylS(O)ₙ- may be substituted with 1-5 fluorine atoms, provided that when any two of R¹, R² and R³ are each 2'-methyl and 3'-methyl, at least one of the other substituents is other than hydrogen atom,
R⁴ and R⁵ are each bound to any one different carbon atom having a replaceable hydrogen atom in the benzene ring to which they are attached, and are independently selected from the group consisting of hydrogen atom, fluorine atom, chlorine atom, C₁₋₆ alkyl, C₁₋₆ alkoxy substituted with 1-3 fluorine atoms, C₁₋₆ alkyl-S(O)ₙ-, cyano and nitro, wherein said alkyl and alkyl -S(O)ₙ- may be substituted with 1-5 fluorine atoms,
R⁶ and R⁷ are independently selected from the group consisting of hydrogen atom, fluorine atom, methyl, ethyl, hydroxy group and C₁₋₆ alkoxy, provided that when one of R⁶ and R⁷ is hydroxy group, the other is not fluorine atom, hydroxy group or C₁₋₆ alkoxy, or R⁶ and R⁷ may be combined to form C₃₋₆ cycloalkyl with the carbon atom which they are attached to, wherein said methyl, ethyl, alkoxy and cycloalkyl may be substituted with 1-5 fluorine atoms,
R⁸ and R⁹ are independently selected from the group consisting of hydrogen atom, C₁₋₆ alkyl, C₃₋₇ cycloalkyl-C₁₋₃ alkyl and C₃₋₆ cycloalkyl, wherein said alkyl, cycloalkyl-alkyl and cycloalkyl may be substituted with 1-5 fluorine atoms,
n is an integer of 0 to 2,
provided that when R¹, R², R³, R⁴ and R⁵ are all hydrogen atoms, and R⁶ and R⁷ are both hydrogen atoms, at least one of R⁸ and R⁹ is hydrogen atom, and
when R¹, R², R³, R⁴ and R⁵ are all hydrogen atoms, and at least one of R⁶ and R⁷ is alkoxy, R⁸ and R⁹ are independently selected from the group consisting of hydrogen atom, C₁₋₃ alkyl, C₃₋₇ cycloalkyl-C₁₋₃ alkyl and C₃₋₆ cycloalkyl.

### Term 3

The compound of term 2 or a hydrate or a solvate thereof wherein R⁸ and R⁹ are independently selected from the group consisting of hydrogen atom, C₁₋₃ alkyl, C₃₋₇ cycloalkyl-C₁₋₃ alkyl and C₃₋₆ cycloalkyl, wherein said alkyl, cycloalkyl-alkyl and cycloalkyl may be substituted with 1-5 fluorine atoms, provided that when R¹, R², R³, R⁴ and R⁵ are all hydrogen atoms, at least one of R⁸ and R⁹ is hydrogen atom.

### Term 4

The compound of term 2 or term 3, or a hydrate or a solvate thereof wherein R¹, R² and R³ are independently selected from the group consisting of hydrogen atom, fluorine atom, chlorine atom, bromine atom, trifluoromethyl, trifluoromethoxy, C₁₋₃ alkyl-S(O)ₙ- and cyano, wherein said alkyl-S(O)ₙ- may be substituted with 1-5 fluorine atoms.

### Term 5

The compound of term 2 or term 3, or a hydrate or a solvate thereof wherein R¹, R² and R³ are independently selected from the group consisting of hydrogen atom, fluorine atom, chlorine atom, bromine atom, trifluoromethyl and trifluoromethoxy.

### Term 6

The compound of any one of terms 2 to 5 or a hydrate or a solvate thereof wherein R⁹ is hydrogen atom.

### Term 7

The compound of any one of terms 2 to 6 or a hydrate or a solvate thereof wherein R⁴ and R⁵ are independently selected from the group consisting of hydrogen atom, fluorine atom, chlorine atom, C₁₋₃ alkyl, trifluoromethyl and trifluoromethoxy.

### Term 8

The compound of any one of terms 2 to 7 or a hydrate or a solvate thereof wherein R⁶ and R⁷ are independently selected from the group consisting of hydrogen atom, methyl, ethyl, hydroxy group and C₁₋₃ alkoxy, provided that when one of R⁶ and R⁷ is hydroxy group, the other is not hydroxy group or C₁₋₃ alkoxy.

### Term 9

The compound of term 2 or a hydrate or a solvate thereof wherein
R¹, R² and R³ are independently selected from the group consisting of hydrogen atom, fluorine atom, chlorine atom, bromine atom, trifluoromethyl and trifluoromethoxy,
R⁴ and R⁵ are independently selected from the group consisting of hydrogen atom, fluorine atom, chlorine atom, trifluoromethyl and trifluoromethoxy,
R⁶ and R⁷ are independently selected from the group consisting of hydrogen atom, methyl, hydroxy group and methoxy provided that when one of R⁶ and R⁷ is hydroxy group, the other is not hydroxy group or methoxy,
R⁸ is hydrogen atom, methyl or ethyl, and
R⁹ is hydrogen atom.

### Term 10

The compound of term 2 or term 9, or a hydrate or a solvate thereof wherein R¹, R², R³, R⁴ and R⁵ are not all hydrogen atom at the same time.

### Term 11

The compound of term 2 or a hydrate or solvate thereof wherein the compound is selected from:
N,N-dimethyl-2-[4'-(trifluoromethyl)biphenyl-2-yl]-acetamide (Example 3),
2-[3'-(trifluoromethyl)biphenyl-2-yl]acetamide (Example 4),
N-methyl-2-[3'-(trifluoromethyl)biphenyl-2-yl]-acetamide (Example 5),
N-methyl-2-[2'-(trifluoromethyl)biphenyl-2-yl]-acetamide (Example 7),
N-ethyl-2-[2'-(trifluoromethyl)biphenyl-2-yl]acetamide (Example 8),
2-(3',4'-difluorobiphenyl-2-yl)acetamide (Example 21),
2-(4'-fluorobiphenyl-2-yl)acetamide (Example 22),
2-(4'-fluorobiphenyl-2-yl)-N-methylacetamide (Example 23),
2-(3'-fluorobiphenyl-2-yl)-N-methylacetamide (Example 26),
2-(2'-fluorobiphenyl-2-yl)-N-methylacetamide (Example 28),
2-(2'-fluorobiphenyl-2-yl)-N,N-dimethylacetamide (Example 30),
N-methyl-2-(3'-methylbiphenyl-2-yl)-acetamide (Example 31),
2-(5-fluorobiphenyl-2-yl)acetamide (Example 39),
2-(4-fluorobiphenyl-2-yl)-N-methylacetamide (Example 45),
2-(2',4-difluorobiphenyl-2-yl)acetamide (Example 46),
2-(2',4-difluorobiphenyl-2-yl)-N-methylacetamide (Example 47),
2-(3'-chloro-4-fluorobiphenyl-2-yl)acetamide (Example 48),
2-(4'-bromobiphenyl-2-yl)acetamide (Example 49),
2-(biphenyl-2-yl)acetamide (Example 50),
2-(3'-bromobiphenyl-2-yl)acetamide (Example 52),
2-(4'-bromobiphenyl-2-yl)-N-methylacetamide (Example 53),
2-(biphenyl-2-yl)-N-methylacetamide (Example 54),
2-(biphenyl-2-yl)propanamide (Example 56),
2-(2'-fluorobiphenyl-2-yl)-N-methyl propanamide (Example 58),
2-(biphenyl-2-yl)-2-hydroxyacetamide (Example 59),
2-(2'-fluorobiphenyl-2-yl)-2-hydroxyacetamide (Example 60),
2-(biphenyl-2-yl)-2-methoxyacetamide (Example 61),
2-(biphenyl-2-yl)-2-methoxy-N-methylacetamide (Example 62),
2-(biphenyl-2-yl)-2-methoxy-N,N-dimethylacetamide (Example 63),
2-(2',3',5'-trichlorobiphenyl-2-yl)acetamide (Example 64),
2-(4'-chloro-2'-fluorobiphenyl-2-yl)acetamide (Example 68),
2-[3'-fluoro-4'-(trifluoromethoxy)biphenyl-2-yl]-acetamide (Example 70),
2-[3'-fluoro-5'-(trifluoromethyl)biphenyl-2-yl]-acetamide (Example 73),
2-[4'-chloro-3'-(trifluoromethyl)biphenyl-2-yl]-acetamide (Example 75),
2-[4'-(trifluoromethoxy)biphenyl-2-yl]acetamide (Example 76),
2-(2',4'-dichlorobiphenyl-2-yl)acetamide (Example 78),
2-[3'-(trifluoromethoxy)biphenyl-2-yl]acetamide (Example 79),
2-[2'-fluoro-5'-(trifluoromethyl)biphenyl-2-yl]-acetamide (Example 83),
2-[2'-chloro-4'-(trifluoromethyl)biphenyl-2-yl]-acetamide (Example 84),
2-(2'-chloro-3'-fluorobiphenyl-2-yl)acetamide (Example 86),
2-(5'-chloro-2'-fluorobiphenyl-2-yl)acetamide (Example 87),
2-(2',3',4'-trifluorobiphenyl-2-yl)acetamide (Example 89),
2-[2'-fluoro-5'-(trifluoromethoxy)biphenyl-2-yl]-acetamide (Example 93),
2-(2',5'-dichlorobiphenyl-2-yl)acetamide (Example 95),
N-methyl-2-[3'-(trifluoromethoxy)biphenyl-2-yl]-acetamide (Example 99),
2-(2'-fluoro-5-nitrobiphenyl-2-yl)acetamide (Example 113),
2-(3-fluorobiphenyl-2-yl)acetamide (Example 118),
2-(3-fluorobiphenyl-2-yl)-N-methylacetamide (Example 119),
2-(5-chlorobiphenyl-2-yl)-N-methylacetamide (Example 124),
2-(5-chloro-2'-fluorobiphenyl-2-yl)acetamide (Example 125),
2-(5-chloro-2'-fluorobiphenyl-2-yl)-N-methylacetamide (Example 126),
2-[4-chloro-4'-(trifluoromethyl)biphenyl-2-yl]-acetamide (Example 142),
2-[3'-fluoro-5-(trifluoromethyl)biphenyl-2-yl]-acetamide (Example 149),
2-(3',5'-dichloro-5-fluorobiphenyl-2-yl)acetamide (Example 150),
2-(3',5'-difluorobiphenyl-2-yl)acetamide (Example 153),
1-(2'-fluorobiphenyl-2-yl)-N-methyl-cyclopropanecarboxamide (Example 157),
1-(3'-fluorobiphenyl-2-yl)-N-methyl-cyclopropanecarboxamide (Example 159),
2-fluoro-2-(3'-fluorobiphenyl-2-yl)acetamide (Example 160),
2-fluoro-2-(2'-fluorobiphenyl-2-yl)-N-methylacetamide (Example 165),
2-fluoro-2-(4'-fluorobiphenyl-2-yl)-N-methylacetamide (Example 166),
2-(3'-chlorobiphenyl-2-yl)-2-fluoroacetamide (Example 167),
2,2-difluoro-2-(3'-fluorobiphenyl-2-yl)acetamide (Example 170),
2-(3'-chlorobiphenyl-2-yl)-2,2-difluoroacetamide (Example 171),
2-(biphenyl-2-yl)-2,2-difluoroacetamide (Example 174),
2-(3'-fluorobiphenyl-2-yl)propanamide (Example 175),
2-(5-fluorobiphenyl-2-yl)-2-hydroxyacetamide (Example 181),
2-(3',5-difluorobiphenyl-2-yl)-2-hydroxyacetamide (Example 182),
2-(3',5'-dichloro-5-fluorobiphenyl-2-yl)-2-hydroxyacetamide (Example 184),
2-(3',4-difluorobiphenyl-2-yl)-2-hydroxyacetamide (Example 188),
2-(3',5'-dichloro-4-fluorobiphenyl-2-yl)-2-hydroxyacetamide (Example 189),
2-(5'-chloro-2'-fluorobiphenyl-2-yl)-2-hydroxyacetamide (Example 194),
2-(2'-fluorobiphenyl-2-yl)-2-methoxyacetamide (Example 200),
2-(2'-fluorobiphenyl-2-yl)-2-methoxy-N-methylacetamide (Example 201),
2-(2'-fluorobiphenyl-2-yl)-2-methoxy-N,N-dimethylacetamide (Example 202),
2-(4'-fluorobiphenyl-2-yl)-2-methoxyacetamide (Example 203),
2-(4'-fluorobiphenyl-2-yl)-2-methoxy-N,N-dimethylacetamide (Example 205), and
2-(3'-chlorobiphenyl-2-yl)-2-methoxyacetamide (Example 206).

### Term 12

The compound of term 2 or a hydrate or solvate thereof wherein the compound is selected from:
2-(2'-fluorobiphenyl-2-yl)acetamide (Example 1),
N-methyl-2-[4'-(trifluoromethyl)biphenyl-2-yl]-acetamide (Example 2),
2-(3'-chlorobiphenyl-2-yl)acetamide (Example 13),
2-(2'-chlorobiphenyl-2-yl)acetamide (Example 16),
2-(2',3'-difluorobiphenyl-2-yl)acetamide (Example 20),
2-(3'-fluorobiphenyl-2-yl)acetamide (Example 25),
2-(2',5-difluorobiphenyl-2-yl)acetamide (Example 41),
2-(4-fluorobiphenyl-2-yl)acetamide (Example 44),
2-(biphenyl-2-yl)-N-methyl propanamide (Example 57),
2-(biphenyl-2-yl)-2-hydroxyacetamide (Example 59),
2-(2'-fluorobiphenyl-2-yl)-2-hydroxyacetamide (Example 60),
2-(2',3',5'-trifluorobiphenyl-2-yl)acetamide (Example 67),
2-(3',5'-dichlorobiphenyl-2-yl)acetamide (Example 71),
2-(3'-chloro-5'-fluorobiphenyl-2-yl)acetamide (Example 80),
2-(2'-chloro-5'-fluorobiphenyl-2-yl)acetamide (Example 88),
2-(3',4',5'-trifluorobiphenyl-2-yl)acetamide (Example 90),
2-[5'-chloro-2'-(trifluoromethyl)biphenyl-2-yl]-acetamide (Example 91),
2-(4,4'-dichlorobiphenyl-2-yl)acetamide (Example 109),
2-(4,4'-difluorobiphenyl-2-yl)acetamide (Example 114),
2-(4,4'-difluorobiphenyl-2-yl)-N-methylacetamide (Example 115),
2-(3,3'-difluorobiphenyl-2-yl)acetamide (Example 140),
2-(2',5'-difluorobiphenyl-2-yl)acetamide (Example 152),
2-(biphenyl-2-yl)-2-fluoroacetamide (Example 162),
2-fluoro-2-(2'-fluorobiphenyl-2-yl)acetamide (Example 164),
2-(3'-fluorobiphenyl-2-yl)-2-hydroxyacetamide (Example 178),
2-(3',5'-dichlorobiphenyl-2-yl)-2-hydroxyacetamide (Example 183),
2-(2'-chloro-5'-fluorobiphenyl-2-yl)-2-hydroxyacetamide (Example 185),
2-(4-fluorobiphenyl-2-yl)-2-hydroxyacetamide (Example 186),
2-(2'-chlorobiphenyl-2-yl)-2-hydroxyacetamide (Example 190),
2-(3'-chloro-5'-fluorobiphenyl-2-yl)-2-hydroxyacetamide (Example 191), and
2-hydroxy-2-(3',4',5'-trifluorobiphenyl-2-yl)acetamide (Example 193).

### Term 13

A pharmaceutical composition comprising the compound of any one of terms 1 to 12 or a hydrate or a solvate thereof, and a pharmaceutically acceptable carrier.

### Term 14

An antiepileptic drug comprising the compound of any one of terms 1 to 12 or a hydrate or a solvate thereof as an active ingredient.

### Term 15

A mood-stabilizing drug for bipolar disorder comprising the compound of any one of terms 1 to 12 or a hydrate or a solvate thereof as an active ingredient.

### Term 16

A method for treating epilepsy comprising administering to an epilepsy patient in need thereof, a therapeutically effective amount of the compound of any one of terms 1 to 12 or a hydrate or a solvate thereof.

### Term 17

Use of the compound of any one of terms 1 to 12 or a hydrate or a solvate thereof for the preparation of an antiepileptic drug.

Preferred examples of R¹ - R⁹ and n of formula (I) in term 2 are illustrated as follows.
Preferred R¹ - R³ are independently selected from the group consisting of hydrogen atom, fluorine atom, chlorine atom, bromine atom, trifluoromethyl and trifluoromethoxy. More preferably, R¹ - R³ are independently selected from the group consisting of hydrogen atom, fluorine atom, chlorine atom, trifluoromethyl and trifluoromethoxy, or the group consisting of hydrogen atom, fluorine atom and chlorine atom.
Preferred R⁴ and R⁵ are independently selected from the group consisting of hydrogen atom, fluorine atom, chlorine atom, trifluoromethyl and trifluoromethoxy. More preferably, R⁴ and R⁵ are independently selected from the group consisting of hydrogen atom, fluorine atom, and chlorine atom, or R⁴ and R⁵ are both hydrogen atom.
Preferred R⁶ and R⁷ are independently selected from the group consisting of hydrogen atom, fluorine atom, methyl, hydroxy group and methoxy. More preferably, R⁶ and R⁷ are independently selected from the group consisting of hydrogen atom, methyl, hydroxy group and methoxy, or the group consisting of hydrogen atom and hydroxy group; or R⁶ and R⁷ are both hydrogen atom.
Preferred R⁸ is hydrogen atom, methyl or ethyl, and preferred R⁹ is hydrogen atom. More preferably, R⁸ is hydrogen atom or methyl, and R⁹ is hydrogen atom.
Preferred n is 2.
Using the preferred examples of R¹ - R⁹ and n as shown above, the compound in any one of the above-mentioned terms may be restricted by selecting one of the preferred examples or by combining several ones, and then such restricted compound of formula (I) is also an aspect of the present invention.

### (Effect of Invention)

The compounds of the present invention equally exhibit potent anticonvulsant activity in several animal models for evaluating antiepileptic drugs candidates. Accordingly, the compounds of the present invention are useful as an antiepileptic drug having a broad therapeutic spectrum, for example, a drug for treating and/or preventing partial seizures (e.g. simple partial seizure, complex partial seizure, and secondarily generalized tonic-clonic seizure) and generalized seizures (e.g. absence seizure, myoclonic seizure, clonic seizure, tonic seizure, tonic-clonic seizure, atonic seizure, west syndrome, and Lennox-Gastaut syndrome). Furthermore, the compounds of the present invention are also expected to prevent or treat intractable epilepsy which cannot be cured by existing drugs. Moreover, the compound of the present invention at the same dosage amount as that required to exhibit an anticonvulsant activity shows an improved efficacy in animal models of both manic state and depressive state which are a pathological background of bipolar disorder, and thereby the compounds of the present invention are expected as a suitable mood stabilizing drug for treating bipolar disorder.

### BEST MODE FOR CARRYING OUT INVENTION

The compound of the present invention may exist in the form of a hydrate and/or a solvate thereof. Thus, the compound of the present invention also encompasses a hydrate and a solvate thereof.

The compound of the present invention may exist as several stereoisomers because the compound may include one or more asymmetric carbon atoms, or have geometric isomerism or axial chirality. Thus, the compound of formula (A) or (I) of the present invention also encompasses stereoisomers thereof, a mixture of the stereoisomers thereof, and a racemate thereof.

Regarding the compound of formula (A) of the present invention, R¹, R², R³, R^{a} and R^{b} are each bound to any one of five carbon atoms having a replaceable hydrogen atom in the benzene ring to which they are attached; and R⁴, R⁵, R^{c} and R^{d} are each bound to any one of four carbon atoms having a replaceable hydrogen atom in the benzene ring to which they are attached.

Regarding the compound of formula (1) of the present invention, R¹, R², and R³ are each bound to any one of three carbon atoms selected from five carbon atoms having a replaceable hydrogen atom in the benzene ring to which they are attached; and R⁴ and R⁵ are each bound to any one of two carbon atoms selected from four carbon atoms having a replaceable hydrogen atom in the benzene ring to which they are attached.
Regarding the compound of formula (1) of the present invention, the carbon atoms which may be substituted with R¹, R² and R³ are numbered as in the following formula (I). For example, "any two of R¹, R² and R³ are each 2'-methyl and 3'-methyl" means that two methyl are each substituted at the position of 2' and 3' in the following formula (I).

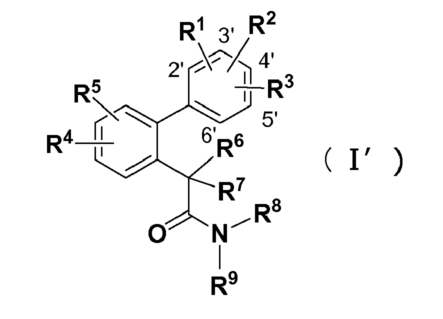

The terms used herein are defined as follows.
The term "alkyl" used herein means a straight or branched saturated hydrocarbon. For example, "C₁₋₃ alkyl" means an alkyl having 1 to 3 carbon atoms, and "C₁₋₆ alkyl" means an alkyl having 1 to 6 carbon atoms. Representative examples of "C₁₋₃ alkyl" include methyl, ethyl, propyl, and isopropyl. Besides such groups, "C₁₋₆ alkyl" includes butyl, isobutyl, s-butyl, t-butyl, pentyl, isopentyl, neopentyl, and hexyl.

The term "C₃₋₆ cycloalkyl" used herein means a monocyclic saturated hydrocarbon having 3 to 6 carbon atoms. Representative examples of "C₃₋₆ cycloalkyl" include cyclopropyl, cyclobutyl, cyclopentyl, and cyclohexyl.

The term C₃₋₇ cycloalkyl-C₁₋₃ alkyl" used herein means alkyl having 1 to 3 carbons wherein a monocyclic saturated hydrocarbon having 3 to 7 carbons is attached to any one carbon of the straight or branched saturated hydrocarbon. Representative examples of "C₃₋₇ cycloalkyl-C₁₋₃ alkyl" include cyclopropylmethyl, cyclobutylmethyl, 1-cyclopropylethyl, 2-cyclopropylethyl, 1-cyclopropylpropyl, 2-cyclopropylpropyl, 1-cyclobutylethyl, 2-cyclobutylethyl, cyclopentylmethyl, and cyclohexylmethyl.

The term "C₁₋₆ alkoxy" used herein means a straight or branched alkoxy having 1 to 6 carbon atoms. Representative examples of "C₁₋₆ alkoxy" include methoxy, ethoxy, propoxy, isopropoxy, butoxy, isobutoxy, s-butoxy, t-butoxy, pentyloxy, and hexyloxy.

The term "C₁₋₆ alkoxy substituted with 1-3 fluorine atoms" used herein means the above-defined alkoxy wherein any one to three replaceable hydrogen atoms of the alkoxy are substituted with fluorine atoms. Representative examples of "C₁₋₆ alkoxy substituted with 1-3 fluorine atoms" include difluoromethoxy, trifluoromethoxy, 2,2,2-trifluoroethoxy, 3,3,3-trifluoropropoxy, 4-fluorobutoxy, and 4,4,4-trifluorobutoxy.

The term "C₁₋₆ alkyl-S(O)ₙ-" used herein means -S(O)ₙ-group attached to a straight or branched alkyl having 1 to 6 carbon atoms. For example, "C₁₋₆ alkyl-S(O)ₙ-" means alkylthio when n is 0 (e.g. methylthio and ethylthio), alkylsulfinyl when n is 1 (e.g. methylsulfinyl and ethylsulfinyl), and alkylsulfonyl when n is 2 (e.g. methanesulfonyl and ethanesulfonyl).

The phrase "alkyl (including methyl and ethyl), alkylS(O)ₙ-, alkoxy, cycloalkyl and cycloalkyl-alkyl which may be substituted with 1-5 fluorine atoms" used herein means that above-defined alkyl, alkoxy, cycloalkyl and cycloalkyl-alkyl wherein any one to five replaceable hydrogen atoms on any carbon atoms thereof are substituted with fluorine atoms. Representative examples of the phrase include trifluoromethyl, fluoroethyl, trifluoroethyl, trifluoromethanesulfonyl, trifluoromethoxy, 2-fluorocyclopropyl, and (2-fluorocyclopropyl)methyl. When alkyl has only one carbon, the number of fluorine atoms which may be substituted is 3 or less.

R¹ - R³ R^{a}, and R^{b} include hydrogen atom, fluorine atom, chlorine atom, bromine atom, C₁₋₆ alkyl, C₁₋₆ alkoxy substituted with 1-3 fluorine atoms, C₁₋₆ alkyl-S(O)ₙ- and cyano, wherein said alkyl and alkyl-S(O)ₙ- may be substituted with 1-5 fluorine atoms wherein n is an integer of 0 to 2. Preferably, the groups include hydrogen atom, fluorine atom, chlorine atom, bromine atom, trifluoromethyl, trifluoromethoxy, methylthio, methanesulfonyl and cyano. More preferably, the groups include hydrogen atom, fluorine atom, chlorine atom, trifluoromethyl, trifluoromethoxy and cyano. Even more preferably, the groups include hydrogen atom, fluorine atom, chlorine atom and trifluoromethyl. The especially preferred groups include hydrogen atom, fluorine atom and trifluoromethyl, or hydrogen atom, fluorine atom and chlorine atom.

R⁴, R⁵, R^{c}, and R^{d} include hydrogen atom, fluorine atom, chlorine atom, C₁₋₆ alkyl, C₁₋₆ alkoxy substituted with 1-3 fluorine atoms, C₁₋₆ alkyl-S(O)ₙ-, cyano and nitro, wherein said alkyl and alkyl-S(O)ₙ- may be substituted with 1-5 fluorine atoms wherein n is an integer of 0 to 2. Preferably, the groups include hydrogen atom, fluorine atom, chlorine atom, methyl, ethyl, trifluoromethyl, trifluoromethoxy, methylthio, methanesulfonyl and cyano. More preferably, the groups include hydrogen atom, fluorine atom, chlorine atom, methyl, ethyl, trifluoromethyl, trifluoromethoxy and cyano. Even more preferably, the groups include hydrogen atom, fluorine atom, chlorine atom, trifluoromethyl and trifluoromethoxy. The especially preferred groups include hydrogen atom and fluorine atom.

R⁶ and R⁷ include hydrogen atom, fluorine atom, methyl, ethyl, hydroxy group and C₁₋₆ alkoxy, or R⁶ and R⁷ may be combined to form C₃₋₆ cycloalkyl with the carbon atom which they are attached to, wherein said methyl, ethyl, alkoxy and cycloalkyl may be substituted with 1-5 fluorine atoms. Preferably, the groups include hydrogen atom, methyl, ethyl, hydroxy group, methoxy and ethoxy. The especially preferred groups include hydrogen atom, methyl and hydroxy group.

R⁸ and R⁹ include hydrogen atom, C₁₋₆ alkyl, C₃₋₇ cycloalkyl-C₁₋₃ alkyl and C₃₋₆ cycloalkyl, wherein said alkyl, cycloalkylalkyl and cycloalkyl may be substituted with 1-5 fluorine atoms. The preferred R⁸ includes hydrogen atom, methyl, ethyl and cyclopropyl. The especially preferred R⁸ includes hydrogen atom and methyl. The preferred R⁹ includes hydrogen atom, methyl and ethyl. The especially preferred R⁹ includes hydrogen atom and methyl.

The variable n is an integer of 0 to 2. The preferred n is 0 or 2. The especially preferred n is 2.

The term "epilepsy" used herein means a chronic disease caused by hyperexcitability of brain neurons, and the disease exhibits repeated paroxysmal symptoms, impaired consciousness, abnormal sensation and behavioral aberration. The term includes diseases such as partial seizures (e.g. simple partial seizure, complex partial seizure, and secondarily generalized seizure) and generalized seizures (e.g. absence seizure, myoclonic seizure, clonic seizure, tonic-clonic seizure, atonic seizure, west syndrome, and Lennox-Gastaut syndrome). Furthermore, the term "antiepileptic drug" used herein means a medicament for preventing and/or treating the above-listed diseases, and the phrase "method for treating epilepsy" used herein means a method for preventing and/or treating the above-listed diseases.

The term "bipolar disorder" used herein means a disease exhibiting dysthymia caused by recurrent manic and depressive states. The phrase "mood-stabilizing drug for bipolar disorder" used herein means a medicament for preventing and/or treating the above-listed diseases. The phrase "method for treating bipolar disorder" used herein means a method for preventing and/or treating the above-listed diseases.

In addition, some terms used herein are optionally defined by the abbreviations below for the sake of shorthand. p-_{:} para-, t-: tert-, s-: sec-, THF: tetrahydrofuran, DMF: N,N-dimethylformamide, DME: ethylene glycol dimethyl ether, DMSO: dimethylsulfoxide, CDCl₃: chloroform-d, DMSO-d₆: dimethylsulfoxide-d₆, TFA: trifluoroacetic acid, and MeCN: acetonitrile.

The compound of formula (I) of the present invention can be synthesized by the following procedures. The compound of formula (A) can be also synthesized with a similar procedure thereto by using a starting material having substituents R^{a} - R^{d}.

### (Preparation 1)

The compound of formula (I) wherein R⁶ and R⁷ are not hydroxy group [i.e. the compound of formula (Ia) shown below] can be prepared by the following process.

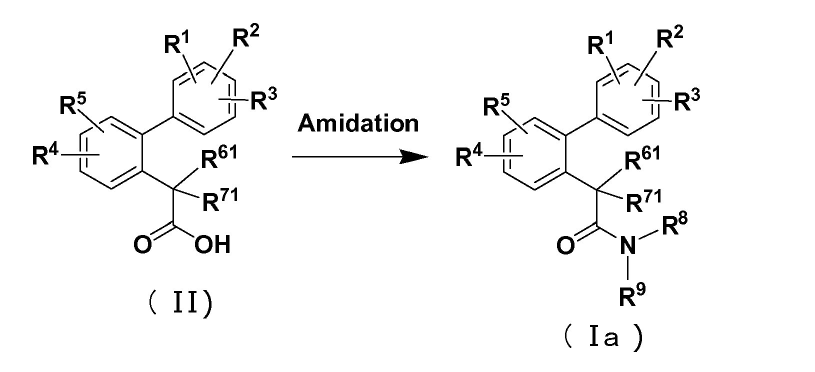

Wherein R¹ - R⁵, R⁸ and R⁹ are as defined in term 1; and R⁶¹ and R⁷¹ are independently selected from the group consisting of hydrogen atom, fluorine atom, methyl, ethyl and C₁₋₆ alkoxy, or R⁶¹ and R⁷¹ may be combined to form C₃₋₆ cycloalkyl with the carbon atom they are attached to.
Compound (Ia) can be prepared by amidating compound (II).

The amidation reaction of compound (II) can be carried out by conventional methods. For example, the reaction can be achieved by converting compound (II) to a reactive derivative such as lower alkyl ester, active ester, acid anhydride and acid halide thereof, and then reacting the reactive derivative with ammonia or an appropriate amine. The active ester used herein includes, for example, p-nitrophenyl ester, N-hydroxysuccinimide ester, and pentafluorophenyl ester. The acid anhydride used herein includes mixed acid anhydride of compound (II) with, for example, ethyl chlorocarbonate, isobutyl chlorocarbonate, isovaleric acid, and/or pivalic acid.

Alternatively, compound (Ia) can be prepared by reacting compound (II) with ammonia or an appropriate amine in the presence of a condensing agent. The condensing agent includes, for example, N,N,'-dicyclohexylcarbodiimide, 1-ethyl-3-(3-dimethylaminopropyl)carbodiimide monohydrochloride, N,N,'-carbonyldiimidazole, and benzotriazol-1-yl-oxytris(pyrrolidino)phosphonium hexafluorophosphate. These condensing agents may be used alone or in combination with a peptide-synthesis reagent such as N-hydroxysuccinimide and N-hydroxybenzotriazole.

The reaction of compound (II) with ammonia or an appropriate amine is carried out in a solvent or in a solvent-free condition. The solvent used herein includes, for example, toluene, THF, dioxane, DME, dichloromethane, chloroform, ethyl acetate, acetone, acetonitrile, DMF, and DMSO, but it should be limited based on the type of the starting compound or other factors. The above-listed solvents can be used alone or as a mixed solvent. In addition, ammonia or an appropriate amine may be used as an aqueous solution, or in the form of an acid addition salt (e.g. a hydrochloride thereof) to produce the free base during the reaction. Furthermore, usually the reaction may also be carried out in the presence of a base, and such base includes, for example, an inorganic base (e.g. potassium carbonate and sodium bicarbonate) and an organic base (e.g. triethylamine, ethyldiisopropylamine, N-methyl morpholine, pyridine and 4-dimethylaminopyridine). The reaction temperature may vary depending on the starting compound, generally, it is about -30°C to about 150 °C, preferably about -10°C to about 70 °C.

### (Preparation 2)

The compound of formula (I) wherein at least one of R⁶ and R⁷ is hydroxy group [i.e. the compound of formula (Ib) shown below] can be prepared by the following process.

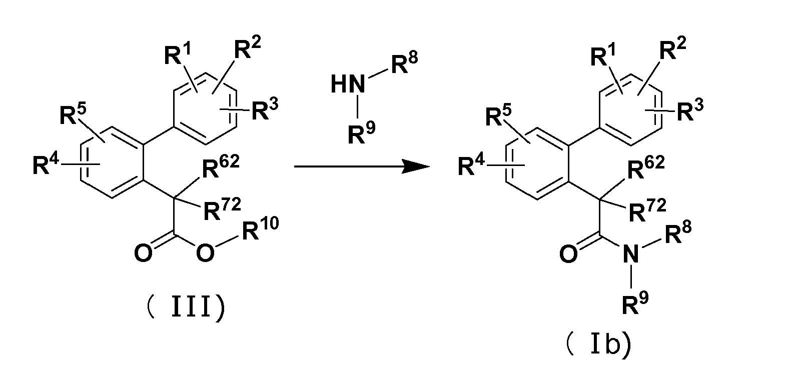

Wherein R¹ - R⁵, R⁸ and R⁹ are as defined in term 1; one of R⁶² and R⁷² is hydroxy group, and the other is hydrogen atom, methyl or ethyl; and R¹⁰ is C₁₋₆ alkyl.
Compound (Ib) can be prepared by reacting compound (III) with ammonia or an appropriate amine.

The reaction of compound (III) with ammonia or an appropriate amine is performed in a solvent or in a solvent-free condition. The solvent used herein includes, for example, toluene, THF, dioxane, DME, dichloromethane, chloroform, ethyl acetate, acetone, acetonitrile, DMF, DMSO, methanol, ethanol and ethylene glycol, but it should be limited based on the type of the starting material or other factors. The above-listed solvents can be used alone or as a mixed solvent. The reaction temperature may vary depending on the starting compound, generally, it is about -30°C to about 150°C, preferably about -10°C to about 70°C.

### (Preparation 3)

The compound of formula (I) wherein both R⁶ and R⁷ are not hydroxy group [i.e. the compound of (Ic) shown below] can be prepared by the following process.

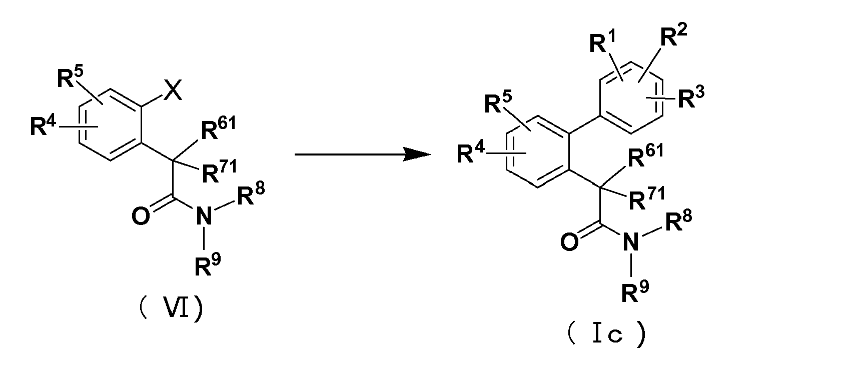

Wherein R¹ - R⁵, R⁸ and R⁹ are as defined in term 1; R⁶¹ and R⁷¹ are independently selected from the group consisting of hydrogen atom, fluorine atom, methyl, ethyl and C₁₋₆ alkoxy, or R⁶¹ and R⁷¹ may be combined to form C₃₋₆ cycloalkyl with the carbon atom they are attached to; and X is chlorine atom, bromine atom, iodine atom or triflate (i.e. CF₃SO₃-).
Compound (Ic) can be prepared by coupling compound (VI) with an appropriate substituted-phenyl boronic acid.

Compound (Ic) can be prepared by a cross-coupling reaction of compound (VI) with a boron reagent [e.g. an appropriate substituted-phenyl boronic acid, that is, PhB(OH)₂] and organometallic reagents (e.g. an appropriate substituted-phenyl zinc chloride), in the presence of a catalyst of transition metal such as palladium catalyst (e.g. tetrakis triphenylphosphine palladium) in a suitable solvent. Besides the above-shown reagents, the coupling reaction may also be performed with, for example, an inorganic base such as an alkali metal carbonate (e.g. sodium carbonate, potassium carbonate, and cesium carbonate)and potassium phosphate, and an organic base (e.g. triethylamine and diisopropylethylamine); and additionally a mineral salt (e.g. lithium chloride and cesium fluoride) may also coexist.

The appropriate boron reagent or organometallic reagent used herein may be commercially available or prepared from commercial reagents by well-known methods. The solvent used herein includes, for example, toluene, THF, dioxane, DME, ethyl acetate, acetone, acetonitrile, DMF, an alcohol (e.g. methanol, ethanol, isopropanol, and t-butanol), and water, but it should be limited based on various factors such as the type of the starting compounds and reagents. The above-listed solvents can be used alone or as a mixed solvent. The reaction temperature may vary depending on the types of the starting compounds and reagents, generally, it is 0 °C to 200°C, preferably 60°C to 150°C.

The compounds prepared by preparations 1, 2 and 3, i.e. compounds of formulae (Ia), (Ib) and (Ic), can be isolated and purified by conventional methods such as chromatography and recrystallization.

The next section shows some processes for preparing the starting compounds used in preparations 1, 2 and 3.

Compound (II) used in preparation 1 is prepared by following the scheme below.

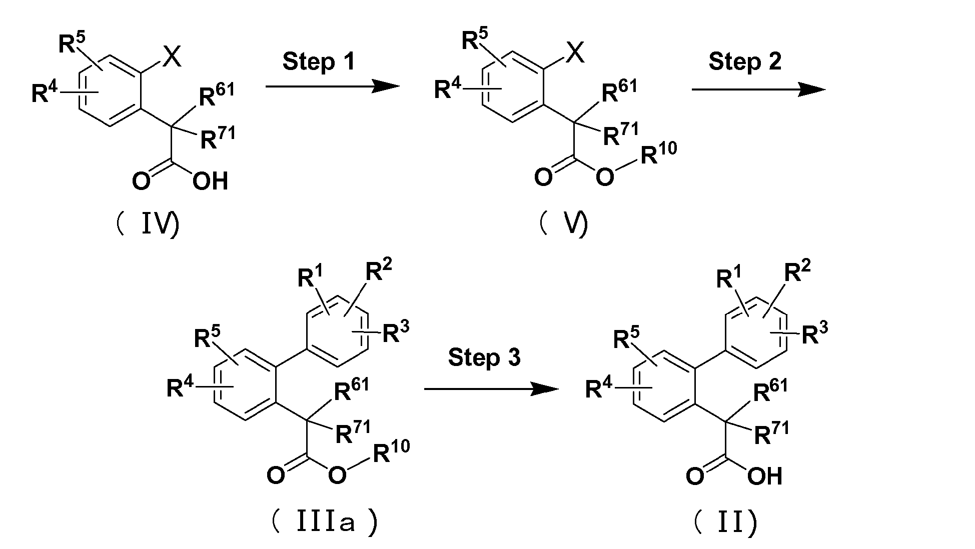

Wherein R¹ - R⁵, R⁶¹ and R⁷¹ are as defined in preparation 1; R¹⁰ is C₁₋₆ alkyl; and X is chlorine atom, bromine atom, iodine atom or triflate (i.e. CF₃SO₃-).

### (Step 1)

Compound (V) can be prepared by esterification of compound (IV).
The esterification reaction in step 1 can be carried out by conventional methods. For example, the reaction may be performed by contacting compound (IV) with an appropriate alcohol or alkyl halide in a suitable solvent under acidic or basic conditions. Alternatively, compound (V) can be synthesized by reacting compound (IV) with diazo compounds such as diazomethane and trimethylsilyldiazomethane. The solvent used herein includes, for example, THF, dioxane, DME, acetone, acetonitrile, DMF, toluene, and an alcohol (e.g. methanol, ethanol, and isopropanol), but it should be limited based on the type of the starting compound or other factors. The above-listed solvents can be used alone or as a mixed solvent. The acid used herein includes, for example, mineral acids (e.g. hydrochloric acid, sulfuric acid, and hydrofluoric acid) and organic acids (e.g. trifluoroacetic acid, methanesulfonic acid, trifluoromethanesulfonic acid, and p-toluenesulfonic acid). The base used herein includes, for example, an alkali metal hydroxide (e.g. sodium hydroxide, potassium hydroxide, and lithium hydroxide), an alkali metal alkoxide (e.g. potassium t-butoxide), and an alkali metal carbonate (e.g. sodium carbonate, potassium carbonate, and lithium carbonate). The reaction temperature may vary depending on the starting compounds and the reagents, generally, it is 0 °C to 150 °C, preferably 20 °C to 100 °C.

### (Step 2)

Compound (IIIa) can be prepared by a cross-coupling reaction of compound (V) with a boron reagent such as an appropriate substituted-phenyl boronic-acid [i.e. PhB(OH)_{2]} and organometallic reagents such as an appropriate substituted-phenyl zinc chloride, in the presence of a catalyst of transition metal such as palladium catalyst (e.g. tetrakis triphenylphosphine palladium) in a suitable solvent. Besides the above-shown reagents, the coupling reaction may also be performed with, for example, an inorganic base such as an alkali metal carbonate (e.g. sodium carbonate, potassium carbonate, and cesium carbonate)and potassium phosphate, and an organic base (e.g. triethylamine and diisopropylethylamine); and additionally a mineral salt (e.g. lithium chloride and cesium fluoride) may also coexist. In addition, compound (IIIa) having R⁶¹ and R⁷¹ of fluorine is prepared by a well-known process [see, WO 2009013963, US 2004254166, or Tetrahedron Letters, 27, 6103-6106 (1986)] or a similar process thereto.

The appropriate boron reagent or organometallic reagent used herein may be commercially available or prepared from commercial reagents by well-known methods. The solvent used herein includes, for example, toluene, THF, dioxane, DME, ethyl acetate, acetone, acetonitrile, DMF, an alcohol (e.g. methanol, ethanol, isopropanol, and t-butanol), and water, but it should be limited based on various factors such as the type of the starting compounds and reagents. The above-listed solvents can be used alone or as a mixed solvent. The reaction temperature may vary depending on the types of the starting compounds and reagents, generally, it is 0 °C to 200 °C, preferably 60 °C to 150 °C.

### (Step 3)

Compound (II) can be prepared by hydrolysis of compound (IIIa).
The hydrolysis reaction in step 3 can be carried out by conventional methods. For example, the reaction can be performed by contacting compound (IIIa) and water in a suitable solvent under acidic or basic condition. The solvent used herein includes, for example, THF, dioxane, DME, acetone, acetonitrile, DMF, DMSO, an alcohol (e.g. methanol, ethanol, and isopropanol), and water, but it should be limited based on the type of the starting compound or other factors. The above-listed solvents can be used alone or as a mixed solvent. The acid used herein includes mineral acids such as hydrochloric acid and sulfuric acid. The base used herein includes, for example, an alkali metal hydroxide (e.g. sodium hydroxide, potassium hydroxide, and lithium hydroxide), an alkali metal alkoxide (e.g. potassium t-butoxide), and an alkali metal carbonate (e.g. sodium carbonate, potassium carbonate, and lithium carbonate). The reaction temperature may vary depending on the starting compounds and the reagents, generally, it is 0 °C to 150 °C, preferably 20 °C to 100 °C.
In addition, compound (II) can be directly prepared by reacting compound (IV) in a similar manner to step 1, which means that this process does not go through steps 2 and 3.

Compound (III) used in preparation 2 can be prepared in a similar manner to the above-shown scheme.

The compound of formula (IIIa) wherein at least one of R⁶¹ and R⁷¹ is C₁₋₆ alkyl or fluorine atom [i.e. the compound of formula (IIIc) shown below] can be prepared by the following scheme. 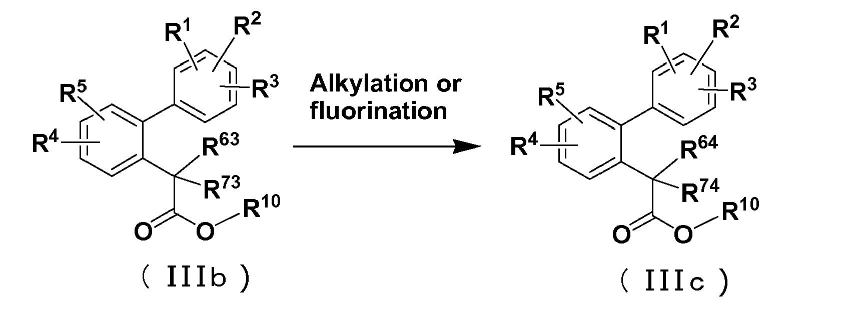 Wherein R¹ - R⁵ are as defined in term 1; at least one of R⁶³ and R⁷³ is hydrogen atom or hydroxy group, provided that when only one of R⁶³ and R⁷³ is hydrogen atom or hydroxy group, the other is the same as the definition of R⁶ and R⁷ in term 1; at least one of R⁶⁴ and R⁷⁴ is C₁₋₆ alkyl or fluorine, and the other is the same as the definition of R⁶ and R⁷ in term 1; and R¹⁰ is C₁₋₆ alkyl.
Compound (IIIc) can be prepared by alkylation or fluorination of compound (IIIb).

The above-mentioned alkylation and fluorination can be carried out by conventional methods. For example, the reaction can be performed by contacting compound (IIIb) with alkyl halide, dimethyl sulfate, (diethylamino)sulfur trifluoride, bis(2-methoxyethyl)amino sulfur trifluoride or other appropriate reagents, in a suitable solvent under neutral or basic conditions. The solvent used herein includes, for example, toluene, hexane, diethyl ether, THF, dioxane, DME, acetone, acetonitrile, DMF, an alcohol (e.g. methanol, ethanol, and isopropanol) and water, but it should be limited based on the type of the starting compound or other factors. The above-listed solvents can be used alone or as a mixed solvent. The base used herein includes, for example, an alkali metal hydroxide (e.g. sodium hydroxide, potassium hydroxide, and lithium hydroxide), an alkali metal hydride (e.g. sodium hydride and potassium hydride), an alkali metal alkoxide (e.g. sodium methoxide, sodium ethoxide, and potassium t-butoxide), an alkali metal carbonate (e.g. sodium carbonate, potassium carbonate, and lithium carbonate), a metal amide (e.g. lithium diisopropylamide, lithium hexamethyldisilazide, sodium hexamethyldisilazide, and potassium hexamethyldisilazide), and an organic base (e.g. pyridine, triethylamine, and 4-dimethylaminopyridine). The reaction temperature may vary depending on the starting compounds and the reagents, generally, it is -78 °C to 150 °C, preferably -20 °C to 70 °C.

Compound (IIIb) can be prepared in a similar manner to the process of compound (IIIa). In addition, compound (II) can also be prepared from compound (IIIc) by a process similar to step 3, that is, preparing compound (II) from compound (IIIa). Compound (IV) may be commercially available or prepared from well-known compounds by conventional methods.

Compound (VI) used in preparation 3 can be prepared from, for example, compound (IV) by the amidation reaction of preparation 1 or preparation 2.

The compound of formula (I) prepared by any one of preparations 1 to 3 can be isolated and purified by conventional methods such as chromatography, recrystallization, and reprecipitation. When the compound of formula (I) is in a racemic form, each enantiomer can be resolved and purified by conventional methods (e.g. optical resolution by chromatography with an optically-active column, preferential crystallization, and a diastereomer method) to provide an optically active substance thereof.

The novel biphenylacetamide derivatives of the present invention are useful as an antiepileptic drug, as set forth hereinafter. The compound of the present invention may be administered orally, parenterally, or intrarectally, and a daily dose thereof can be varied corresponding to the property of the compound, administration route, patient's symptom and age, or other factors. For example, when the compound is administered orally, mammals are given a dose of usually 1-200 mg/kg, preferably 5-100 mg/kg, and more preferably 10-70 mg/kg; and human beings are given a dose of usually 50-5000 mg/kg, preferably 100-4000 mg/kg, and more preferably 500-3000 mg/kg, which can be administered once per day or in several times per day with divided doses. When the compound is administered parenterally (e.g. intravenous injection), mammals are given a dose of usually 0.1-100 mg/kg, preferably 1-10 mg/kg, and more preferably 4-5 mg/kg; and human beings are given a dose of usually 50-1000 mg/kg, preferably 100-400 mg/kg, and more preferably 200-300 mg/kg.

When the compound of the present invention is used pharmaceutically as set forth above, the compound is usually administered in the form of a formulation comprising one or more pharmaceutical carriers. The pharmaceutical carriers used herein are nontoxic materials which are commonly used in the field of pharmaceutical formulation and do not react with the compound of the present invention. The pharmaceutical carrier includes, for example, citric acid, glutamic acid, glycine, lactose, inositol, glucose, mannitol, dextran, sorbitol, cyclodextrin, starch, partially-pregelatinized starch, sucrose, methyl parahydroxybenzoate, propyl parahydroxybenzoate, magnesium aluminometasilicate, synthetic aluminum silicate, microcrystalline cellulose, carboxymethylcellulose sodium, hydroxypropyl starch, carboxymethyl cellulose calcium, ion exchange resin, methylcellulose, gelatin, acacia, pullulan, hydroxypropylcellulose, low-substituted hydroxypropylcellulose, hydroxypropyl methylcellulose, polyvinylpyrrolidone, polyvinyl alcohol, alginic acid, sodium alginate, light silica, magnesium stearate, talc, tragacanth, bentonite, veegum, carboxy vinyl polymer, titanium oxide, sorbitan fatty acid ester, sodium lauryl sulfate, glycerin, glycerin fatty acid ester, purified lanolin, glycerogelatin, polysorbate, macrogol, vegetable oil, wax, propylene glycol, ethanol, benzyl alcohol, sodium chloride, sodium hydroxide, hydrochloric acid, and water.

The formulation applied in the present invention may be in the form of, for example, tablets, capsules, granules, powders, syrups, suspensions, injections, suppositories, eye drops, ointments, embrocations, adhesive skin patches, and inhalants. These formulations can be prepared by conventional methods. The liquid formulation may be in preparation when used, i.e., the solid formulation may be dissolved or suspended in water or other suitable vehicles when used. Tablets and granules may be coated by known methods. In addition, the formulations may comprise additional ingredients which are therapeutically effective.

### EXAMPLE

The present invention is illustrated in more detail by the following Reference Examples, Examples, and Tests, but it should not be construed to be limited thereto. The compounds were identified by, for example, elementary analysis, mass spectrum analysis, high performance liquid chromatography-mass spectrometry; LCMS, IR spectrum analysis, NMR spectrum (e.g. 400MHz ¹H-NMR, etc.) analysis, high performance liquid chromatography (HPLC), etc.

Some of the terms in Examples and Tables are defined by abbreviations for the sake of shorthand, which are defined below.

When NMR results are shown, "s" means singlet, "d" means doublet, "t" means triplet, "q" means quartet, "m" means multiplet, and "br" means broad.

High performance liquid chromatography-mass spectrometry; LCMS were performed under the following conditions. In addition, "MH⁺" means the observed value of mass spectrometry [MS(m/z)], "Rt" means retention time (min), and "min" means minute(s).
Detection instrument: Agilent 1100 Series for API series (Applied Biosystems)
HPLC: API150EX LC/MS system (Applied Biosystems)
Column: YMC CombiScreen ODS-A (S-5 µM, 12 nm, 4.6 x 50 mm)

### Condition A

Unless otherwise noted, the conditions in Tables of Examples are as follows:
Solvent: A = 0.05 % TFA/H₂O, B = 0.035 % TFA/MeCN
Gradient Condition: 0.0-0.5 min A 90 %, 0.5-4.2 min Linear gradient from A 90 % to 1 %, 4.2-4.4 min Linear gradient from A 1 % to 99 %
Flow rate: 3.5 mL/min
UV: 220nm

### Condition B

When * is marked in Tables of Examples, the conditions are as follows:
Solvent: A = 0.05 % TFA/H₂O, B = 0.035 % TFA/MeOH
Gradient Condition: 0.0-1.0 min A 60 %, 1.0-4.7 min Linear gradient from A 60 % to 1 %, 4.7-5.7 min Linear gradient from A 1 % to 60 %
Flow rate: 1.8 mL/min
UV: 220nm

### Reference Example 1:

### (2'-Fluorobiphenyl-2-yl)acetic acid

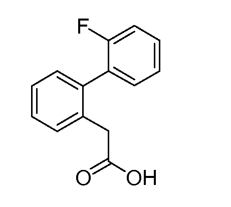

### (Step 1)

To a solution of 2-bromophenylacetic acid (12.8 g) and potassium carbonate (16.4 g) in DMF (50 ml) was added dropwise a solution of ethyl iodide (5.7 ml) in DMF (20 ml) at 0 °C. The mixture was warmed up to room temperature and stirred overnight. After the reaction was completed, the reaction solution was treated with water and extracted with ethyl acetate. The organic layer was washed with brine, dried over anhydrous magnesium sulfate, and concentrated. The residue was purified on silica gel column chromatography to give ethyl (2-bromophenyl)acetate as an oil (13.3 g).

### (Step 2)

A mixture of ethyl (2-bromophenyl)acetate (4.01 g), 2-fluoro phenylboronic acid (3.46 g), tetrakis(triphenylphosphine)palladium (0) (0.95 g), and potassium carbonate (6.82 g) in dioxane-water (10:1) (44 ml) was heated to reflux at 110 °C for 8 hours under nitrogen stream. After the reaction was completed, the reaction mixture was cooled to room temperature, treated with water, and extracted with ethyl acetate. The organic layer was washed with brine, dried over anhydrous magnesium sulfate, and concentrated. The residue was purified on silica gel column chromatography to give ethyl (2'-fluorobiphenyl-2-yl)acetate as an oil (3.87 g).

### (Step 3)

To a solution of ethyl (2'-fluorobiphenyl-2-yl)acetate (1.00 g) in THF (5.0 ml) was added a solution of lithium hydroxide monohydrate (0.19 g) in water (5.0 ml) and methanol (5.0 ml). The mixture was stirred at room temperature for 12 hours. To the reaction was added AMBERLITE^{®} IR-120 PLUS (H). The reaction mixture was adjusted to pH 4 and filtered, and the filtrate was concentrated. The precipitated crystal was collected through a filter to give (2'-fluorobiphenyl-2-yl)acetic acid as a crystal (0.89 g).
1H-NMR (CDCl₃) δ: 3.57 (s, 2H), 7.13 (t, 1H), 7.18 (t, 1H), 7.23-7.28 (m, 3H), 7.34-7.40 (m, 3H).

### Reference Examples 2-26:

The compounds shown in Table 1 were prepared in a similar manner to the preparation of Reference Example 1 using appropriate starting materials.

**Table 1**

| Ref. Ex. | Structure | Ref. Ex. | Structure | Ref. Ex. | Structure |
|---|---|---|---|---|---|
| 2 | 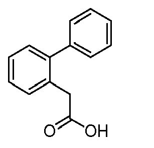 | 3 | 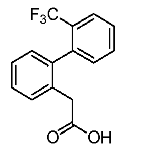 | 4 | 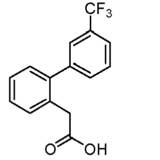 |
| 5 | 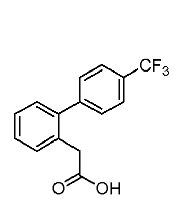 | 6 | 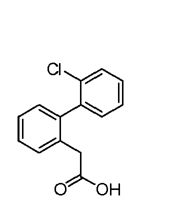 | 7 | 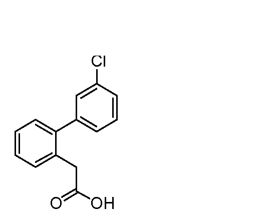 |
| 8 | 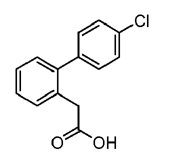 | 9 | 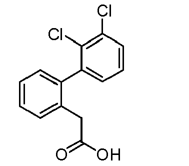 | 10 | 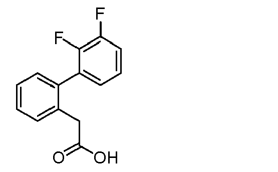 |
| 11 | 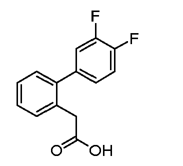 | 12 | 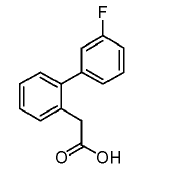 | 13 | 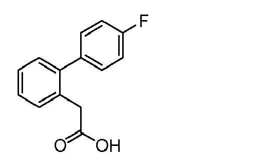 |
| 14 | 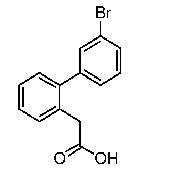 | 15 | 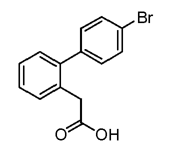 | 16 | 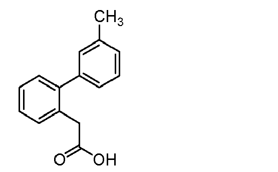 |
| 17 | 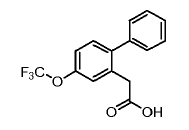 | 18 | 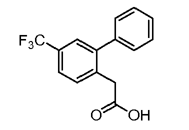 | 19 | 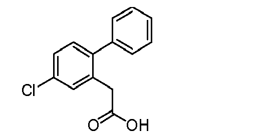 |
| 20 | 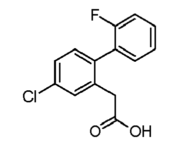 | 21 | 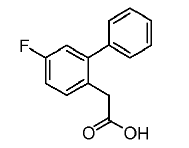 | 22 | 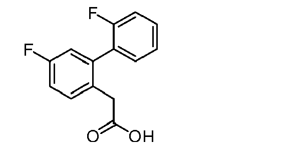 |
| 23 | 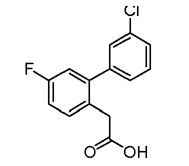 | 24 | 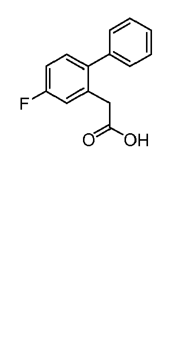 | 25 | 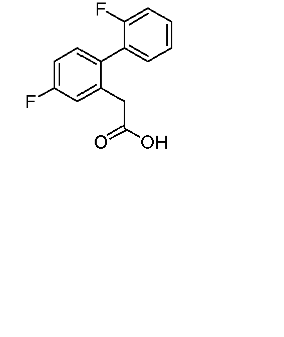 |
| 26 | 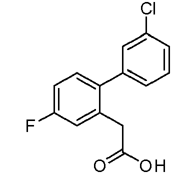 | | | | |

### Reference Example 27:

### 2-(biphenyl-2-yl)propanoic acid

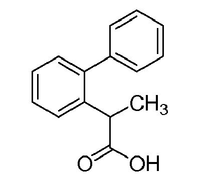

### (Step 1)

To a solution of ethyl biphenyl-2-yl acetate (2.50 g) in THF (25 ml) was added dropwise sodium hexamethyl disilazide (1.9 M in THF) (7.2 ml) at -78 °C, and the mixture was stirred at the same temperature for 0.5 hours. To the reaction solution was added dropwise methyl iodide (0.78 ml), and the mixture was stirred at -40 °C for 2.5 hours. After the reaction was completed, the mixture was treated with aqueous saturated sodium bicarbonate solution and extracted with ethyl acetate. The extract was washed with brine, dried over anhydrous magnesium sulfate, and concentrated. The residue was purified on silica gel column chromatography to give ethyl 2-(biphenyl-2-yl)propanoate (2.57 g).

### (Step 2)

To a solution of ethyl 2-(biphenyl-2-yl)propanoate (1.20 g) in THF (5.0 ml) was added a solution of lithium hydroxide monohydrate (0.24 g) in water (5.0 ml) and methanol (5.0 ml). The mixture was stirred at room temperature for 12 hours. To the reaction mixture was added AMBERLITE^{®} IR-120 PLUS(H). The reaction mixture was adjusted to pH 4 and filtered, and the filtrate was concentrated. The precipitated crystal was collected through a filter to give 2-(biphenyl-2-yl)propanoic acid as a crystal (1.03 g).
¹H-NMR (CDCl₃) δ: 1.38 (d, 3H), 3.93 (q, 1H), 7.24-7.28 (m, 2H), 7.29 (dd, 1H), 7.34-7.45 (m, 7H).

### Reference Example 28:

### Methyl biphenyl-2-yl(hydroxy)acetate

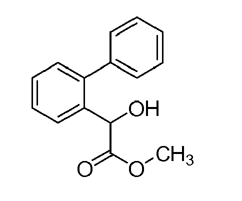

### (Step 1)

(2-Bromophenyl)(hydroxy)acetic acid (7.00 g) was dissolved in a mixture of toluene (20 ml) and methanol (20 ml). To the reaction was added dropwise trimethylsilyl diazomethane (2 mol/l in hexane) (23 ml). The mixture was stirred at room temperature for 1 hour. The solvent was evaporated under reduced pressure, and the residue was purified on silica gel column chromatography to give methyl (2-bromophenyl)(hydroxy)acetate as an oil (4.12 g).

### (Step 2)

A solution of methyl (2-bromophenyl)(hydroxy)acetate (613 mg), phenylboronic acid (457 mg), [1,1'-bis(diphenylphosphino)ferrocene]palladium (II) dichloride dichloromethane complex (1:1) (204 mg), and potassium phosphate (1.59 g) in dioxane (10 ml) was heated to reflex overnight under nitrogen stream. The reaction mixture was purified on silica gel column chromatography to give methyl biphenyl-2-yl(hydroxy)acetate as an oil (190 mg).
¹H-NMR (CDCl₃) δ: 3.37 (d, 1H), 3.71 (s, 3H), 5.25 (d, 1H), 7.30-7.34 (m, 1H), 7.39-7.45 (m, 8H).

### Reference Example 29:

### Methyl(2'-fluorobiphenyl-2-yl)(hydroxy)acetate

The following compound was prepared in a similar manner to the preparation of Reference Example 28 using an appropriate starting material.

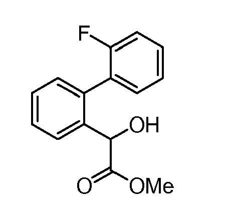

### Reference Example 30:

### Biphenyl-2-yl(methoxy)acetic acid

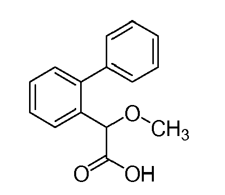

### (Step 1)

To a solution of methyl biphenyl-2-yl(hydroxy)acetate (4.79 g) and methyl iodide (2.5 ml) in DMF (50 ml) was added sodium hydride (1.11 g) under ice-cold condition. The mixture was stirred for 30 minutes, and was further stirred at room temperature for 1 hour. The reaction solution was poured into ice water, extracted with ethyl acetate, washed with brine, and dried over anhydrous magnesium sulfate. The solvent was evaporated under reduced pressure, and the residue was purified on silica gel column chromatography to give methyl biphenyl-2-yl(methoxy)acetate as a crystal (3.64 g).

### (Step 2)

To a solution of methyl biphenyl-2-yl(methoxy)acetate (1.61 g) in THF (10 ml) was added a solution of lithium hydroxide monohydrate (0.49 g) in water (10 ml) and methanol (10 ml). The mixture was stirred at room temperature overnight, aqueous 10% citric acid was added thereto and the reaction solution was adjusted to pH 3. The mixture was extracted with ethyl acetate, washed with brine, dried over anhydrous magnesium sulfate, and the solvent was evaporated to give biphenyl-2-yl(methoxy)acetic acid as an oil (1.52 g).
¹H-NMR (CDCl₃) δ: 3.19 (s, 3H), 4.94 (s, 1H), 7.31- 7.35 (m, 1H), 7.40- 7.51 (m, 8H).

### Reference Example 31:

### 1-(Biphenyl-2-yl)cyclopropane-carboxylic acid

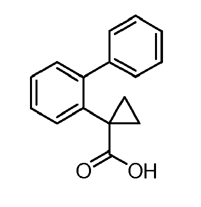

### (Step 1)

1-(2-Bromophenyl)cyclopropane-carboxylic acid (2.00 g) and phenylboronic acid (1.52 g) was dissolved in N,N-dimethylformamide (48 ml), and to the reaction was added [1,1'-bis(diphenylphosphino)ferrocene]palladium (II) dichloride-dichloromethane complex (0.34 g) and 2 N sodium carbonate aqueous solution (12 ml). After substituting with nitrogen, the mixture was stirred for 40 hours with heating at 80 °C. The solvent was evaporated under reduced pressure, 2 N sodium hydroxide aqueous solution was added to the residue, and the insoluble precipitate was removed by Celite filtration. The filtrate was washed with ethyl acetate, and treated with concentrated hydrochloric acid to adjust to pH 1. The mixture was extracted with ethyl acetate, washed with brine, and dried over anhydrous magnesium sulfate. The solvent was evaporated under reduced pressure, and the residue was purified on silica gel column chromatography to give 1-(biphenyl-2-yl)cyclopropane-carboxylic acid as a white crystal (1.58 g). ¹H-NMR (DMSO-d₆) δ: 0.77 (br, 2H), 1.20 (br, 2H), 7.19-7.21 (m, 1H), 7.30-7.44 (m, 8H).

### Reference Examples 32 and 33:

The compounds shown in Table 2 were prepared in a similar manner to the preparation of Reference Example 31 using appropriate starting materials.

**Table 2**

| Ref. Ex. | Structure | Ref. Ex. | Structure |
|---|---|---|---|
| 32 | 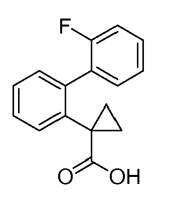 | 33 | 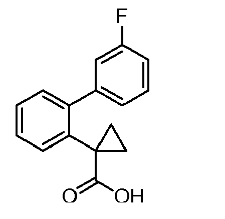 |

### Reference Example 34:

### Fluoro(3'-fluorobiphenyl-2-yl)acetic acid

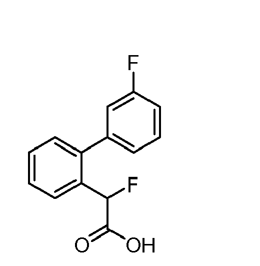

### (Step 1)

Under nitrogen atmosphere, ethyl (3'-fluorobiphenyl-2-yl)acetate (1.50 g) was dissolved in tetrahydrofuran (10 ml), lithium hexamethyldisilazide (1M in toluene) (6.4 ml) was added to the mixture at -78 °C, and the reaction was stirred for 10 minutes. To the mixture was added dropwise a solution of N-fluoro-N-(phenylsulfonyl)benzenesulfonamide (3.36 g) in tetrahydrofuran (10 ml), and the reaction was warmed up to room temperature over 1 hour and stirred overnight. The mixture was treated with aqueous saturated ammonium chloride solution, extracted with ethyl acetate, washed with brine, and dried over anhydrous magnesium sulfate. The solvent was evaporated under reduced pressure, and the residue was purified on silica gel column chromatography to give ethyl fluoro(3'-fluorobiphenyl-2-yl)acetate as a colorless oil (1.46 g).
¹H-NMR (CDCl₃) δ: 1.25 (t, 3H), 4.15-4.29 (m, 2H), 5.81 (d, 1H), 7.09-7.22 (m, 3H), 7.34-7.48 (m, 4H), 7.57-7.59 (m, 1H).

### (Step 2)

Ethyl fluoro(3'-fluorobiphenyl-2-yl)acetate (1.46 g) was dissolved in tetrahydrofuran (10 ml), and to the reaction was added a solution of lithium hydroxide monohydrate (0.67 g) in water (10 ml) and methanol (10 ml). The mixture was stirred at room temperature for 6 hours, treated with aqueous 10 % citric acid to adjust the reaction solution to pH 3, extracted with ethyl acetate, washed with brine, dried over anhydrous magnesium sulfate, and the solvent was evaporated to give fluoro(3'-fluorobiphenyl-2-yl)acetic acid as a white crystal (1.32 g). ¹H-NMR (CDCl₃) δ: 5.88 (d, 1H), 7.09-7.17 (m, 2H), 7.19-7.22 (m, 1H), 7.34-7.53 (m, 4H), 7.59-7.62 (m, 1H).

### Reference Example 35:

### Fluoro(biphenyl-2-yl)acetic acid

The following compound was prepared in a similar manner to the preparation of Reference Example 34 using an appropriate starting material.

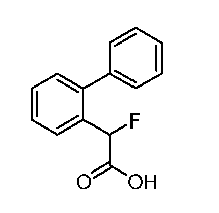

### Reference Example 36:

### Fluoro(2'-fluorobiphenyl-2-yl)acetic acid

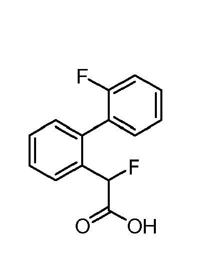

### (Step 1)

Methyl (2'-fluorobiphenyl-2-yl)hydroxyacetate (1.70 g) was dissolved in dichloromethane (20 ml), and to the reaction was added [bis(2-methoxyethyl)-amino]sulfur trifluoride (2.0 ml) under ice-cold condition. The mixture was stirred at room temperature overnight, washed with ice water, and dried over anhydrous magnesium sulfate. The solvent was evaporated under reduced pressure and the residue was purified on silica gel column chromatography to give methyl fluoro(2'-fluorobiphenyl-2-yl)acetate as a colorless oil (1.33 g).
¹H-NMR (CDCl₃) δ: 3.69 (s, 3H), 5.72 (d, 1H), 7.16-7.25 (m, 2H), 7.34-7.44 (m, 3H), 7.47-7.49 (m, 2H), 7.60 (br, 1H).

### (Step 2)

Methyl fluoro(2'-fluorobiphenyl-2-yl)acetate (1.33 g) was dissolved in tetrahydrofuran (10 ml), and to the reaction was added a solution of lithium hydroxide monohydrate (0.64 g) in water (10 ml) and methanol (10 ml). The mixture was stirred at room temperature for 6 hours, adjusted to pH 3 by adding 2 N hydrochloric acid, extracted with ethyl acetate, washed with brine, and dried over anhydrous magnesium sulfate. The solvent was evaporated to give fluoro(2'-fluorobiphenyl-2-yl)acetic acid as a colorless oil (1.33 g).
¹H-NMR (CDCl₃) δ: 5.76 (d, 1H), 7.15-7.23 (m, 2H), 7.37 (br, 3H), 7.49-7.50 (m, 2H), 7.62 (br, 1H).

### Reference Example 37:

### Fluoro(4'-fluorobiphenyl-2-yl)acetic acid

The following compound was prepared in a similar manner to the preparation of Reference Example 36 using an appropriate starting material.

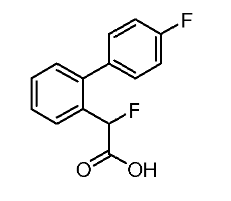

### Reference Example 38:

### Fluoro(3'-chlorobiphenyl-2-yl)acetic acid

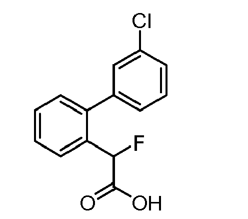

### (Step 1)

Ethyl 2-bromophenyl-hydroxyacetate (1.74 g) was dissolved in dichloromethane (20 ml), and [bis(2-methoxyethyl)-amino]sulfur trifluoride (2.1 ml) was added to the reaction under ice-cold condition. The mixture was stirred at room temperature for 1 hour, washed with ice water, and dried over anhydrous magnesium sulfate. The solvent was evaporated under reduced pressure, and the residue was purified on silica gel column chromatography to give ethyl 2-bromophenyl-fluoroacetate as a colorless oil (1.20 g).
¹H-NMR (CDCl₃) δ: 1.27 (t, 3H), 4.18-4.36 (m, 2H), 6.20 (d, 1H), 7.24-7.30 (m, 1H), 7.35-7.40 (m, 1H), 7.49-7.52 (m, 1H), 7.60-7.64 (m, 1H).

### (Step 2)

Ethyl 2-bromophenyl-fluoroacetate (1.20 g) and 3-chlorophenylboronic acid (1.08 g) was dissolved in 1,4-dioxane (20 ml). To the reaction was added tetrakis(triphenylphosphine)palladium (0.27 g), 3-chloro-phenylboronic acid (1.08 g), potassium carbonate (1.91 g), and water (2 ml). After substituting with nitrogen, the mixture was stirred overnight with heating at 80 °C, treated with water, extracted with ethyl acetate, washed with brine, and dried over anhydrous magnesium sulfate. The residue was purified on silica gel column chromatography to give fluoro(3'-chlorobiphenyl-2-yl)acetic acid as a brown oil (540 mg).
¹H-NMR (CDCl₃) δ: 5.86 (d, 1H), 7.31-7.50 (m, 7H), 7.59-7.61 (m, 1H) .

### Reference Example 39:

### Biphenyl-2-yl(difluoro)acetic acid

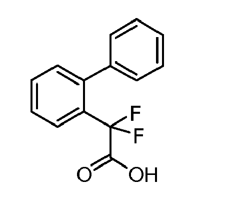

### (Step 1)

Ethyl biphenyl-2-yl(difluoro)acetate (1.59 g) was dissolved in tetrahydrofuran (10 ml). To the reaction was added a solution of lithium hydroxide monohydrate (0.72 g) in water (10 ml) and methanol (10 ml). The mixture was stirred at room temperature overnight, treated with aqueous 10 % citric acid, adjusted to pH 3, extracted with ethyl acetate, washed with brine, and dried over anhydrous magnesium sulfate. The solvent was evaporated to give carboxylic acid as a colorless oil (1.53 g).
¹H-NMR (DMSO) δ: 7.21-7.24 (m, 2H), 7.28(d, 1H), 7.37-7.39 (m, 3H), 7.54-7.62 (m, 2H), 7.71-7.73 (m, 1H), 14.5 (br, 1H) .

### Reference Example 40:

### (2'-Fluorobiphenyl-2-yl)difluoroacetic acid

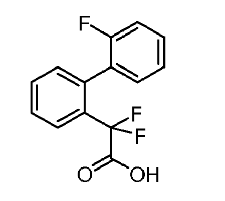

### (Step 1)

Under nitrogen atmosphere, ethyl (2'-fluorobiphenyl-2-yl)acetate (1.5 g) was dissolved in tetrahydrofuran (20 ml), and the reaction was cooled to -78 °C. To the reaction was added sodium hexamethyldisilazide (1.06 M in toluene) (12.1 ml), and the mixture was stirred at -78 °C for 20 minutes. To the mixture was added dropwise a solution of N-fluoro-N-(phenylsulfonyl)benzene-sulfonamide (4.03 g) in tetrahydrofuran (20 ml) at -78 °C, then the temperature was slowly warmed up to room temperature and the mixture was stirred overnight. The mixture was treated with aqueous saturated ammonium chloride solution, acidified, and treated with water and ethyl acetate. The organic layer was washed with brine and dried over anhydrous magnesium sulfate. The solvent was evaporated under reduced pressure, and the residue was purified on silica gel column chromatography to give ethyl (2'-fluorobiphenyl-2-yl)difluoroacetate as a colorless oil (973 mg).
¹H-NMR (CDCl₃) δ: 1.17 (t, 3H), 3.98-4.06 (m, 2H), 7.08-7.39 (m, 4H), 7.35-7.41 (m, 1H), 7.50-7.56 (m, 2H), 7.80-7.84 (m, 1H) .

### (Step 2)

Ethyl (2'-fluorobiphenyl-2-yl)difluoroacetate (973 mg) was dissolved in tetrahydrofuran (5 ml), and to the reaction was added a solution of lithium hydroxide monohydrate (416 mg) in water (5 ml) and methanol (5 ml). The mixture was stirred at room temperature for 6 hours, adjusted to pH 3 by adding aqueous 10 % citric acid, extracted with ethyl acetate, washed with brine, and dried over anhydrous magnesium sulfate. The solvent was evaporated to give (2'-fluorobiphenyl-2-yl)difluoroacetic acid as a white crystal (792 mg).
¹H-NMR (CDCl₃) δ: 7.00-7.10 (m, 2H), 7.16-7.23 (m, 1H), 7.28-7.32 (m, 2H), 7.52-7.57 (m, 2H), 7.79-7.80 (m, 1H).

### Reference Examples 41 and 42:

The compounds shown in Table 3 were prepared in a similar manner to the preparation of Reference Example 40 using appropriate starting materials.

**Table 3**

| Ref. Ex. | Structure | Ref. Ex | Structure |
|---|---|---|---|
| 41 | 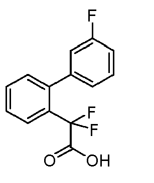 | 42 | 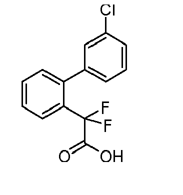 |

### Reference Examples 43-71:

The compounds shown in Table 4 were prepared in a similar manner to the preparation of Reference Example 1 using the appropriate starting materials.

**Table 4**

| Ref. Ex. | Structure | Ref. Ex. | Structure | Ref. Ex. | Structure |
|---|---|---|---|---|---|
| 43 | 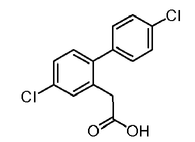 | 44 | 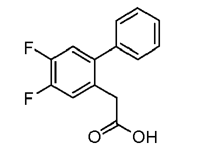 | 45 | 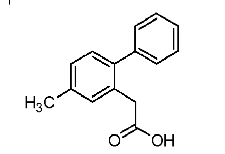 |
| 46 | 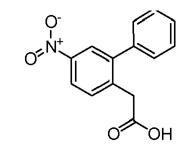 | 47 | 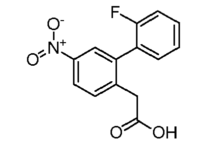 | 48 | 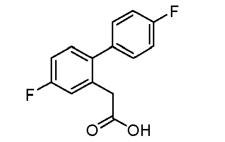 |
| 49 | 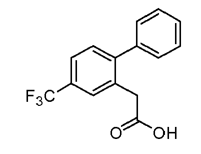 | 50 | 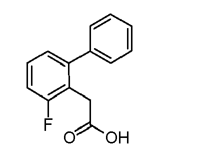 | 51 | 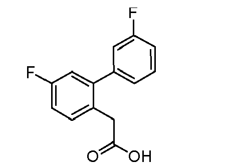 |
| 52 | 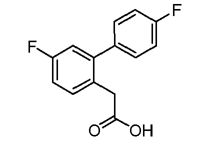 | 53 | 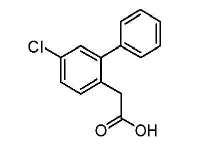 | 54 | 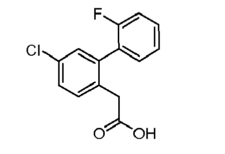 |
| 55 | 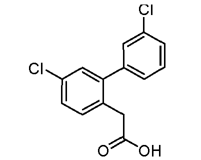 | 56 | 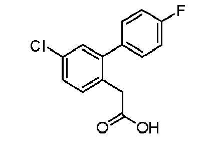 | 57 | 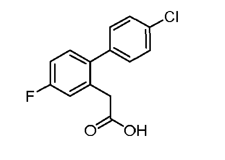 |
| 58 | 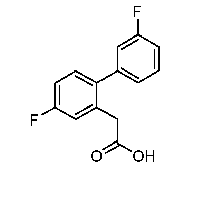 | 59 | 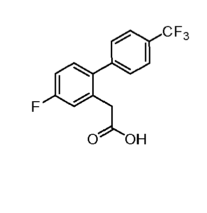 | 60 | 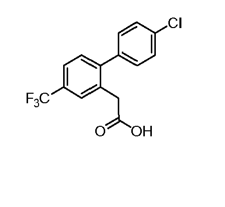 |
| 61 | 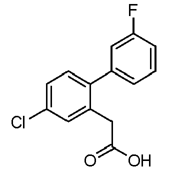 | 62 | 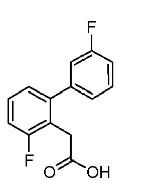 | 63 | 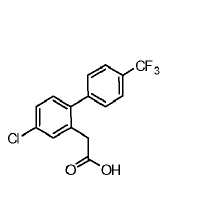 |
| 64 | 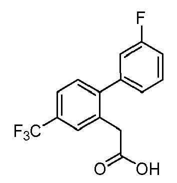 | 65 | 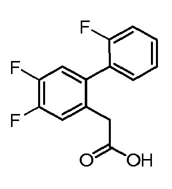 | 66 | 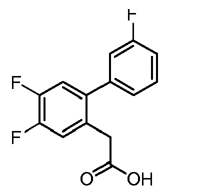 |
| 67 | 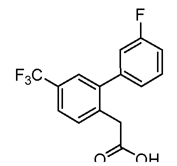 | 68 | 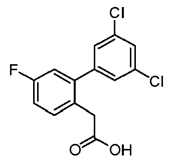 | 69 | 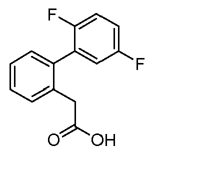 |
| 70 | 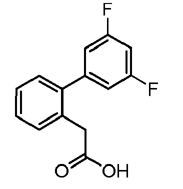 | 71 | 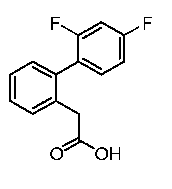 | | |

### Reference Examples 72-73:

The compounds shown in Table 5 were prepared in a similar manner to the preparation of Reference Example 27 using appropriate starting materials.

**Table 5**

| Ref. Ex. | Structure | Ref. Ex. | Structure |
|---|---|---|---|
| 72 | 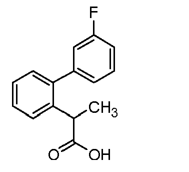 | 73 | 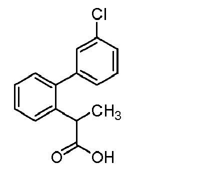 |

### Reference Examples 74-86:

The compounds shown in Table 6 and Table 7 were prepared in a similar manner to the preparation of Reference Example 28 using appropriate starting materials.

**Table 6**

| Ref. Ex. | Structure | Ref. Ex. | Structure | Ref. Ex. | Structure |
|---|---|---|---|---|---|
| 74 | 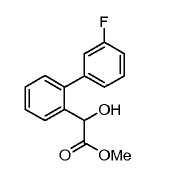 | 75 | 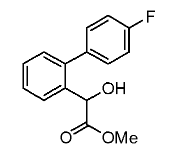 | 76 | 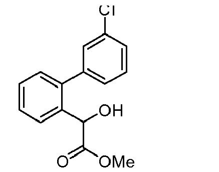 |
| 77 | 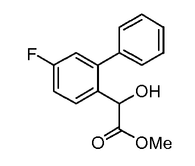 | 78 | 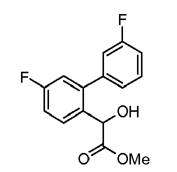 | 79 | 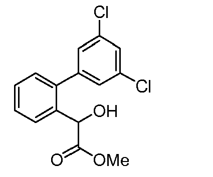 |
| 80 | 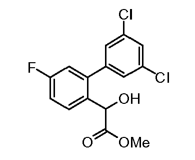 | 81 | 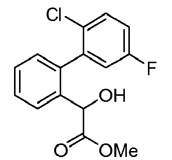 | 82 | 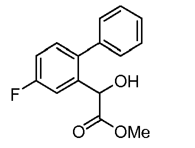 |
| 83 | 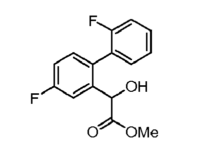 | 84 | 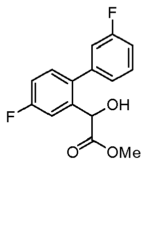 | 85 | 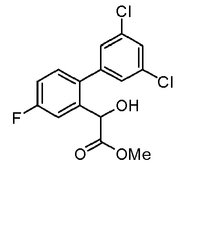 |
| 86 | 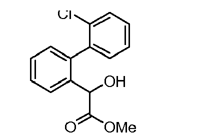 | | | | |

**Table 7**

| Ref. Ex. | Structure | Ref. Ex. | Structure | Ref . Ex. | Structure |
|---|---|---|---|---|---|
| 87 | 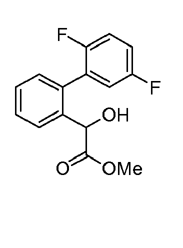 | 88 | 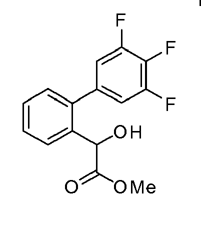 | 89 | 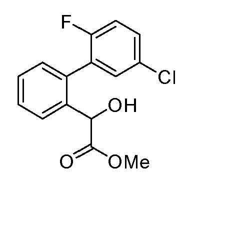 |
| 90 | 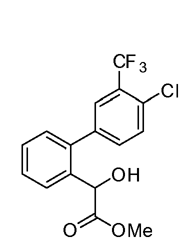 | 91 | 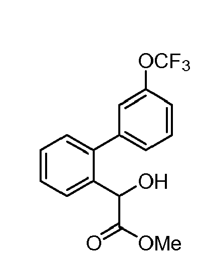 | 92 | 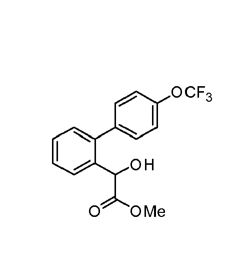 |
| 93 | 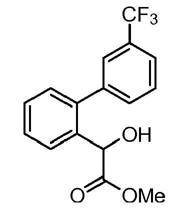 | | | | |

### Reference Examples 94-97:

The compounds shown in Table 8 were prepared in a similar manner to the preparation of Reference Example 30 using appropriate starting materials.

**Table 8**

| Ref. Ex. | Structure | Ref. Ex. | Structure | Ref. Ex. | Structure |
|---|---|---|---|---|---|
| 94 | 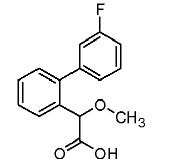 | 95 | 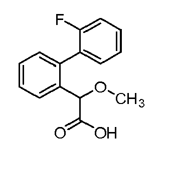 | 96 | 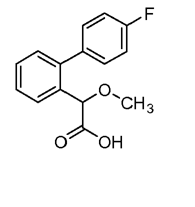 |
| 97 | 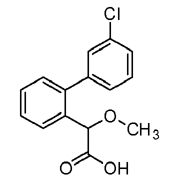 | | | | |

### Example 1:

### 2-(2'-Fluorobiphenyl-2-yl)acetamide

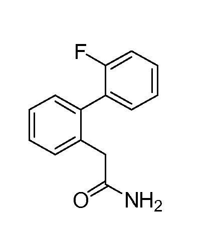

To a suspension of (2'-fluorobiphenyl-2-yl)acetic acid prepared in Reference Example 1 (0.96 g) in dichloromethane (8.0 ml) was added DMF (0.05 ml) and oxalyl chloride(0.43 ml). The mixture was stirred at room temperature for 1 hour and concentrated to give a pale yellow crystal. The crystal was dissolved in THF (3.0 ml), aqueous 28% ammonia (2.0 ml) was added to the solution, and the mixture was stirred at room temperature overnight. The resultant solid was collected on a filter and dried to give 2-(2'-fluorobiphenyl-2-yl)acetamide as a crystal (0.93 g).
¹H-NMR (CDCl₃) δ: 3.50 (s, 2H), 5.24 (br, 2H), 7.13-7.44 (m, 8H)
MS (m/z) 230 (MH⁺), Rt= 2.88 min.

### Examples 2-48:

The compounds shown in Table 9 were prepared in a similar manner to the preparation of Example 1 using appropriate starting materials.

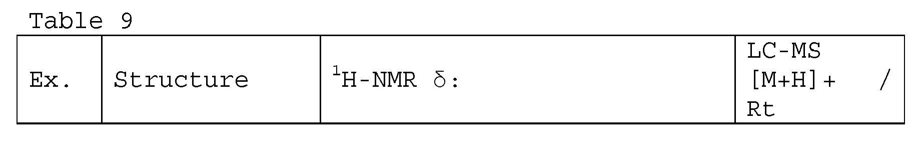

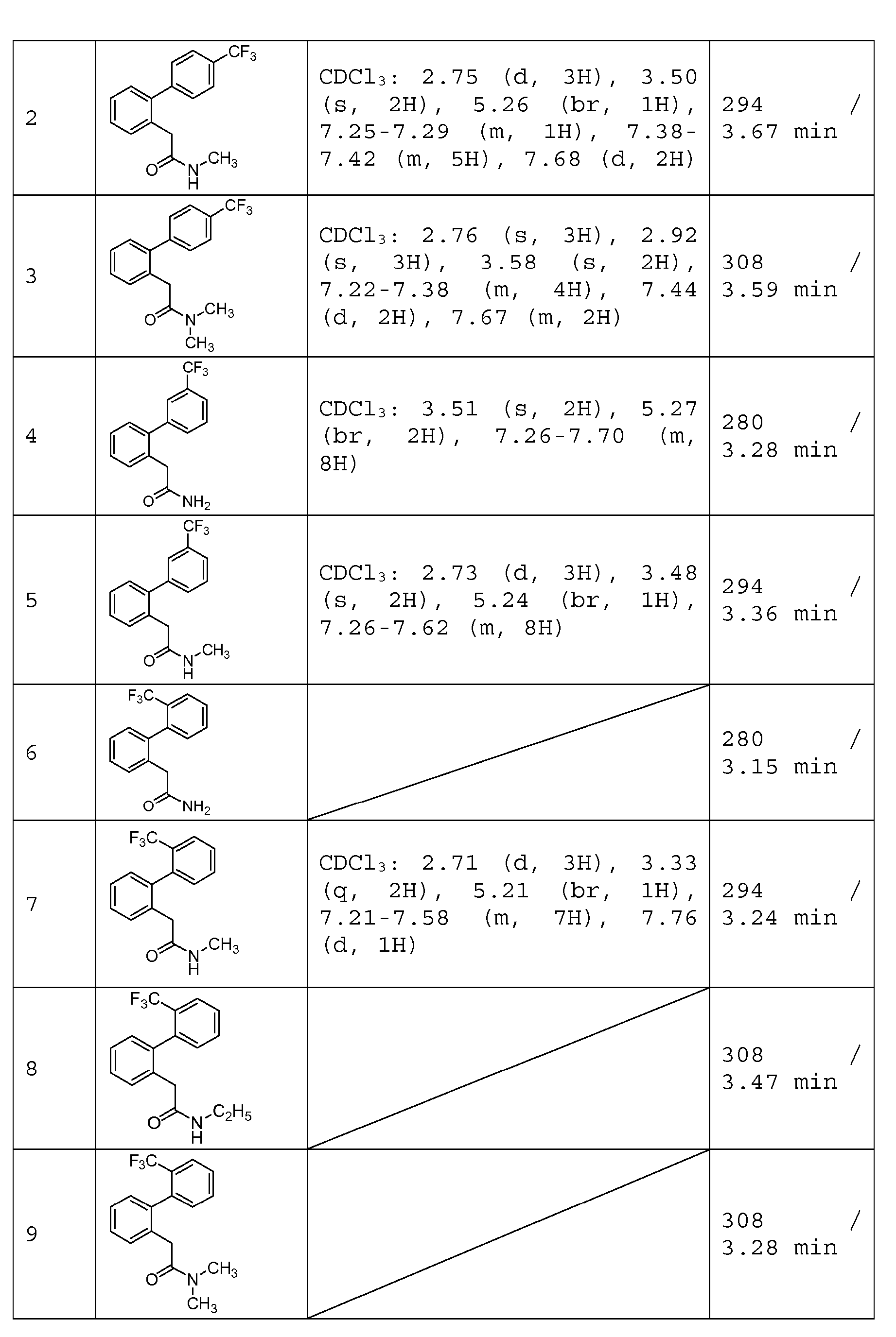

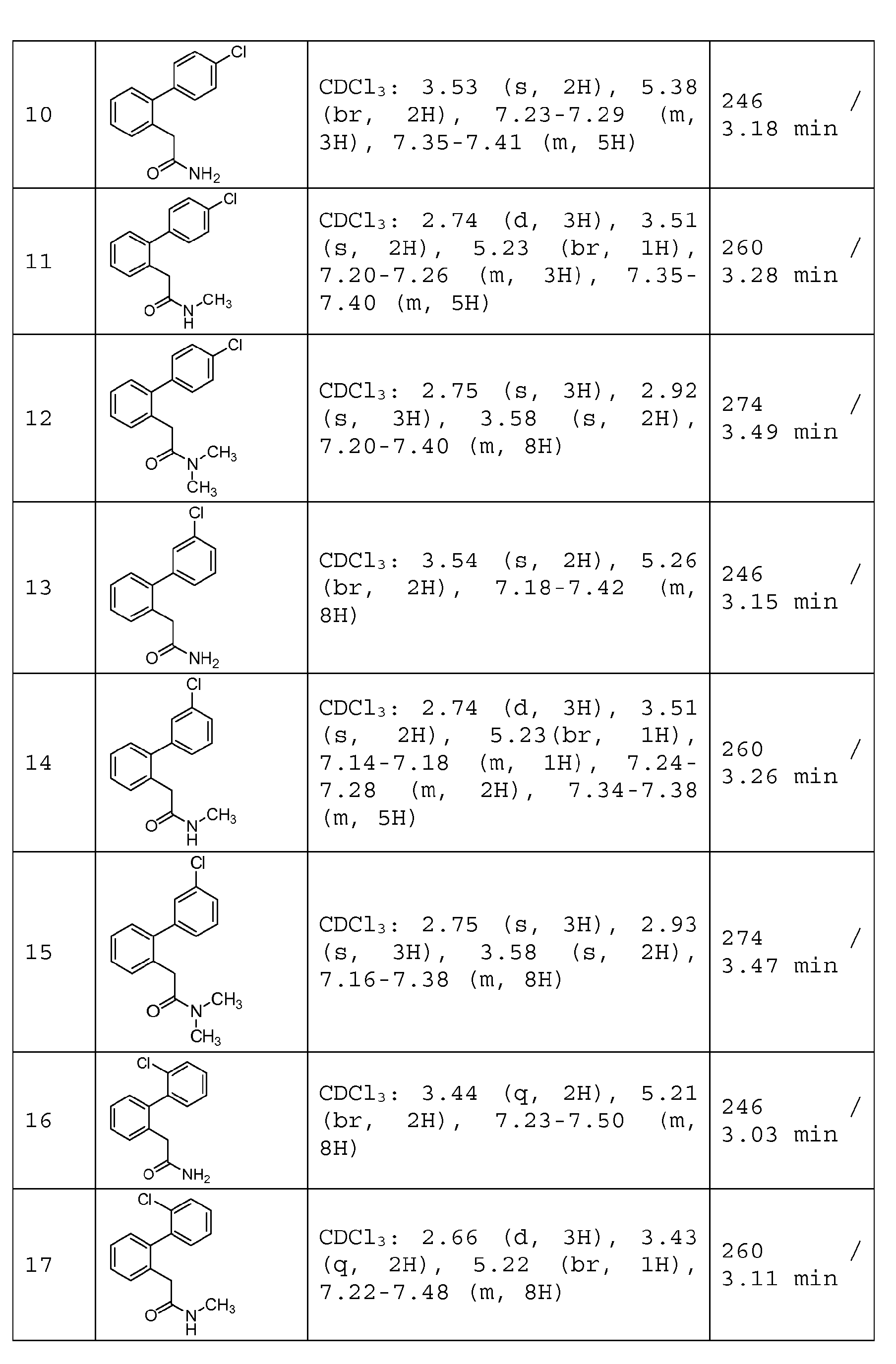

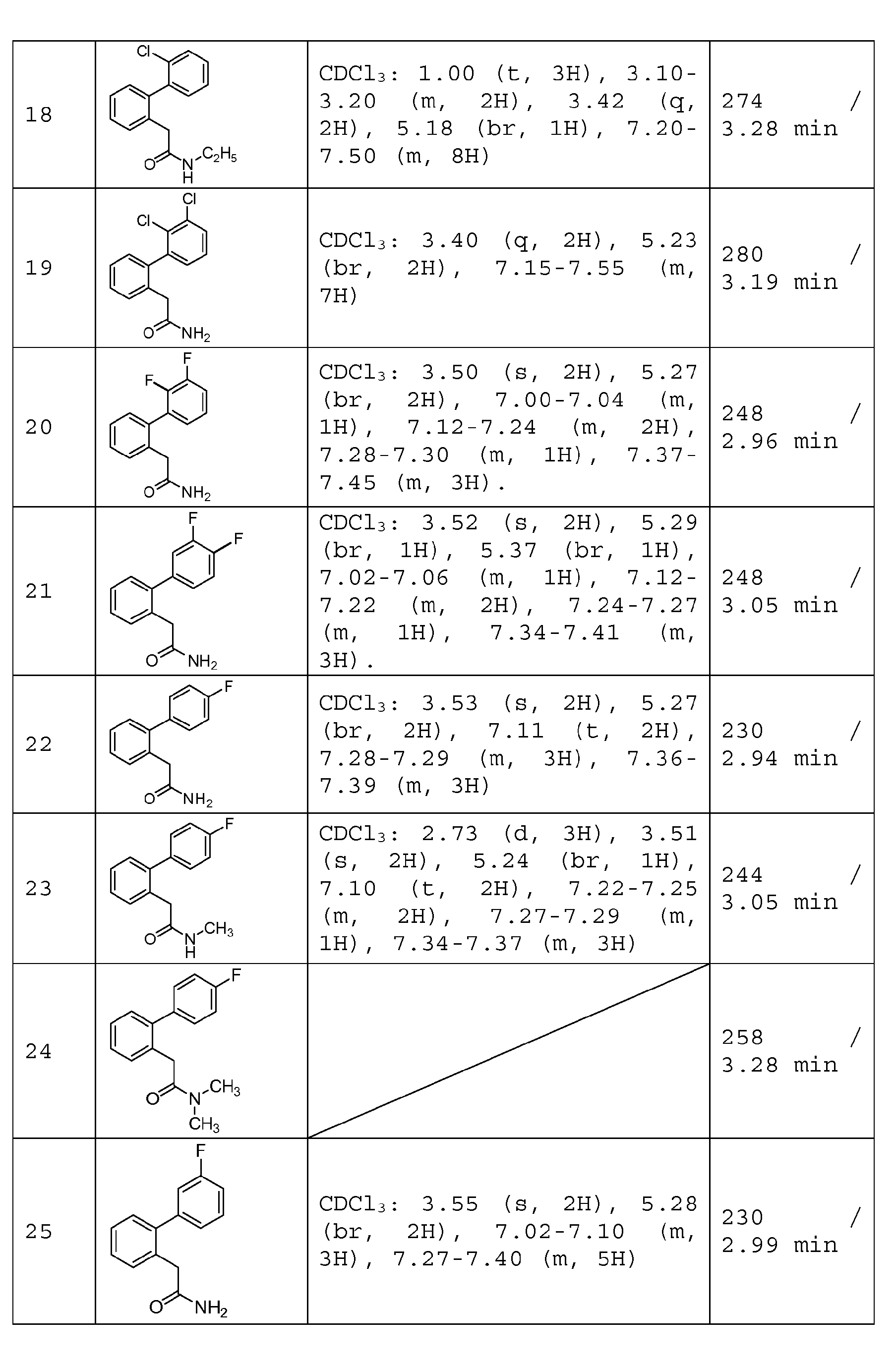

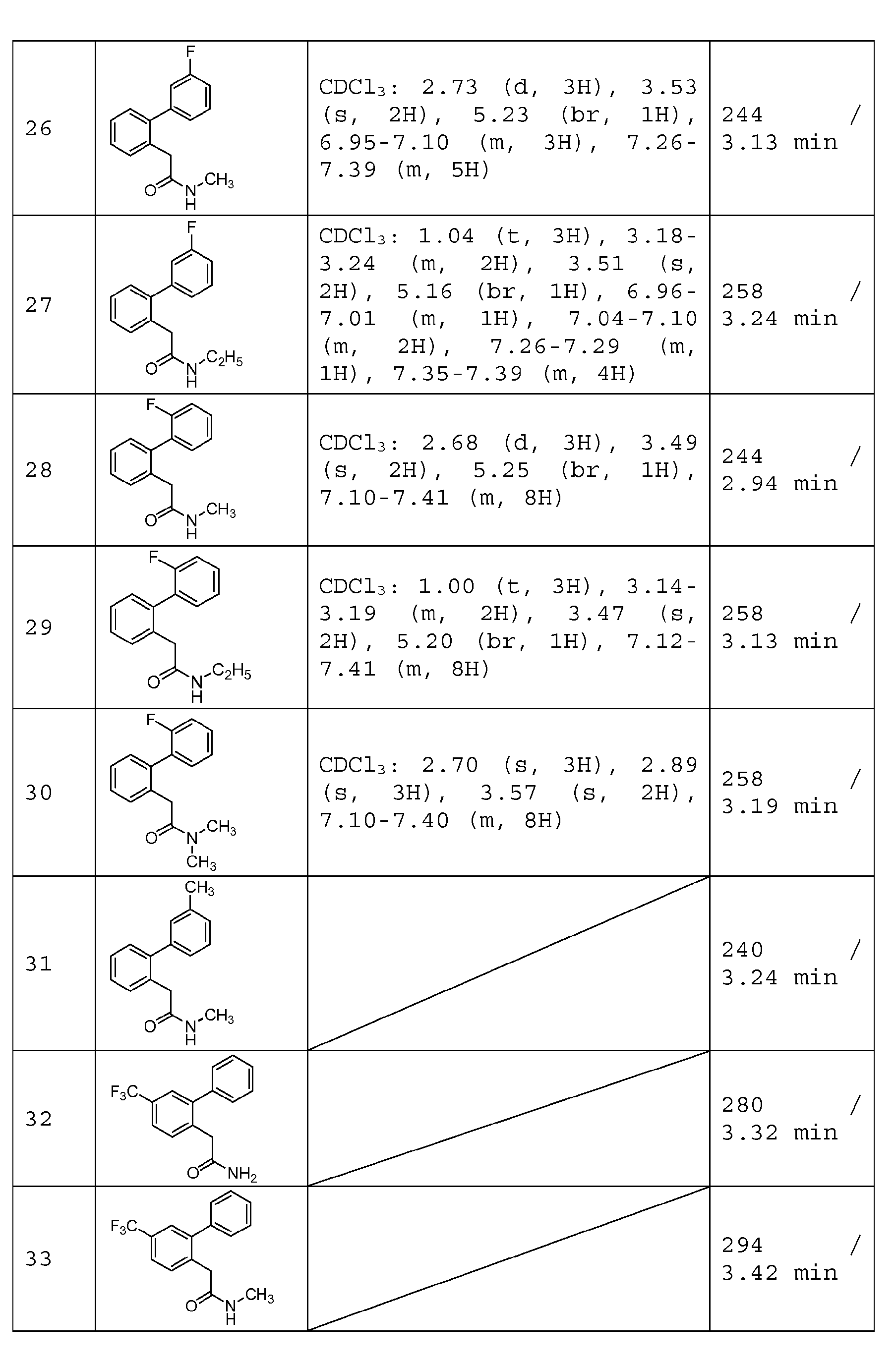

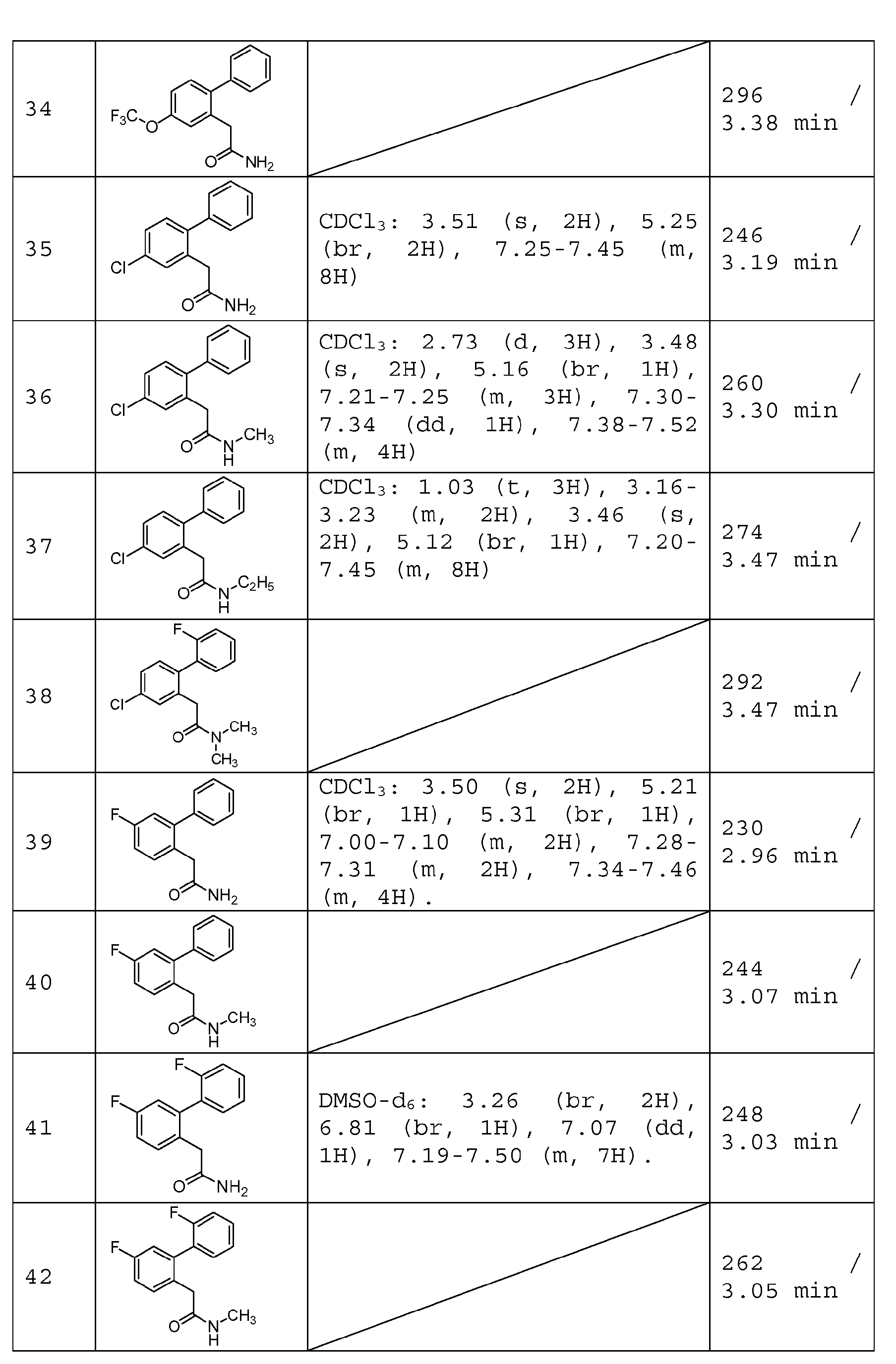

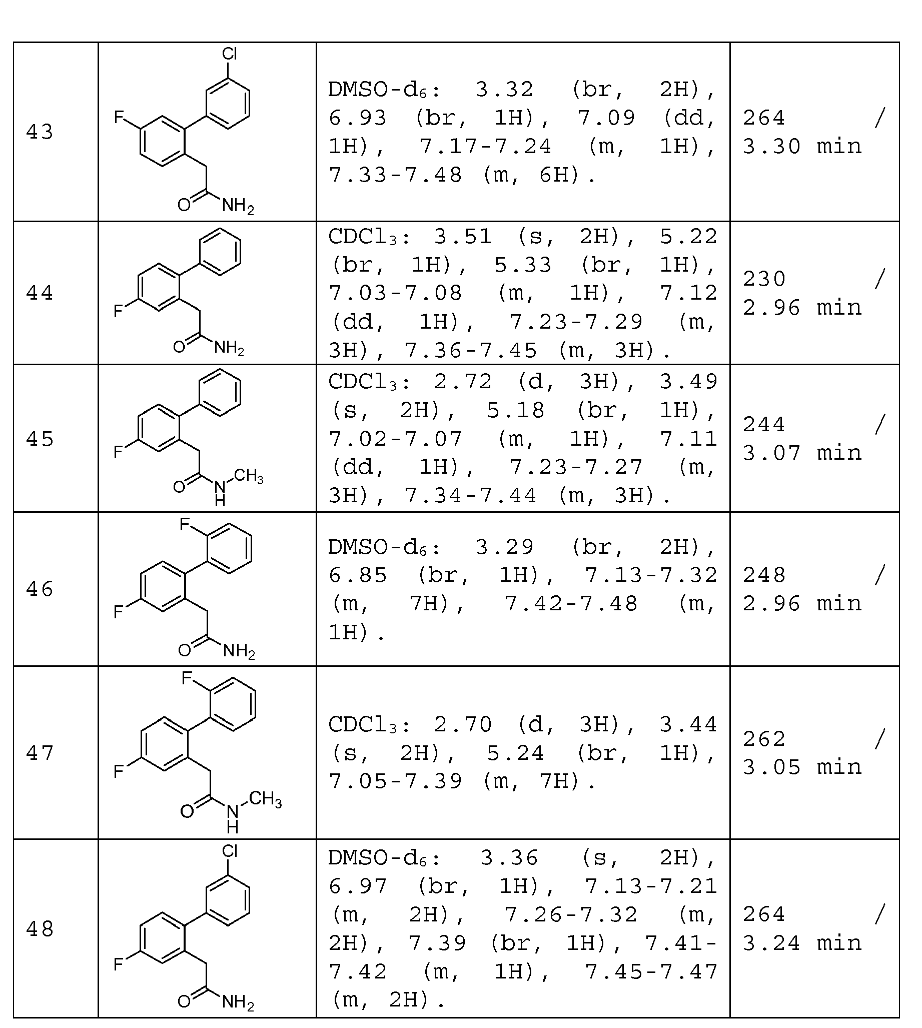

### Example 49:

### 2-(4'-Bromobiphenyl-2-yl)acetamide

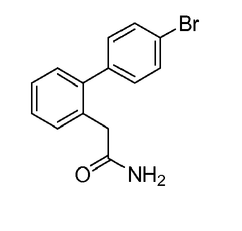

2-(4'-Bromobiphenyl-2-yl)acetic acid prepared in Reference Example 15 (391 mg), ammonium hydrogen carbonate (148 mg), and di-t-butyl dicarbonate (351 mg) were dissolved in a mixture of pyridine (0.5 ml) and dioxane (10 ml). The mixture was stirred at room temperature overnight, treated with water, and concentrated. The resultant crystal was collected through a filter to give 2-(4'-bromobiphenyl-2-yl)acetamide as a crystal (356 mg).
¹H-NMR (CDCl₃) δ: 3.52 (s, 2H), 5.33 (br, 2H), 7.17-7.21 (m, 2H), 7.26-7.39 (m, 4H), 7.53-7.56 (m, 2H).
MS (m/z) 290 (MH⁺), Rt = 3.26 min.

### Examples 50-52:

The compounds shown in Table 10 were prepared in a similar manner to the preparation of Example 49 using appropriate starting materials.

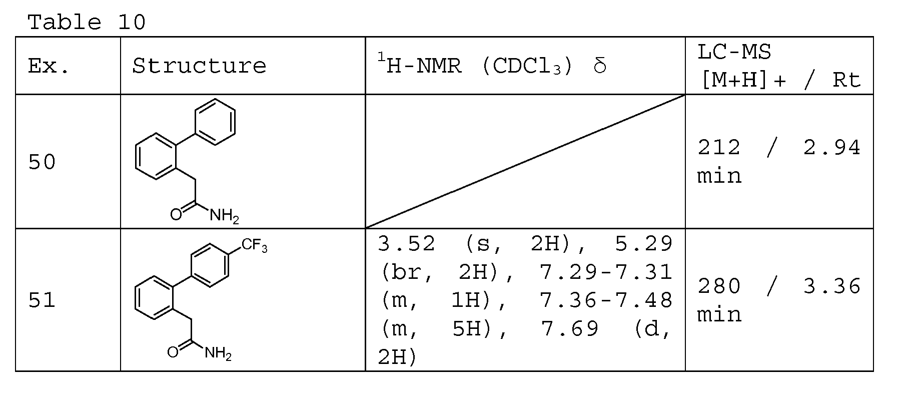

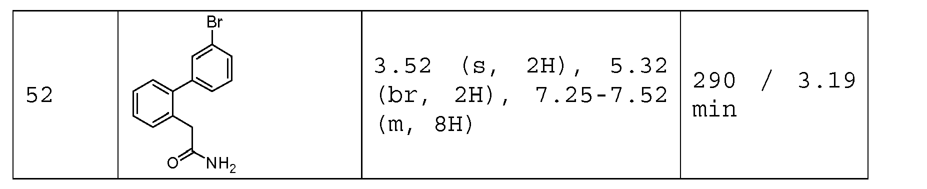

### Example 53:

### 2-(4'-Bromobiphenyl-2-yl)-N-methylacetamide

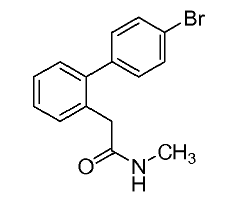

A solution of 2-(4'-bromobiphenyl-2-yl)acetic acid prepared in Reference Example 15 (391 mg), 1-ethyl-3-(3-dimethylaminopropylcarbodiimide) hydrochloride (515 mg), 1-hydroxybenzotriazole monohydrate (363 mg), methylamine hydrochloride (272 mg), N,N-diisopropylethylamine (0.70 ml) in DMF (10 ml) was stirred at room temperature overnight. The reaction solution was treated with aqueous 10 % citric acid, extracted with ethyl acetate, and sequentially washed with saturated sodium bicarbonate aqeous solution and brine. The organic layer was dried over anhydrous magnesium sulfate. The reaction mixture was concentrated under reduced pressure, and the residue was treated with hexane and collected through a filter to give 2-(4'-bromobiphenyl-2-yl)-N-methylacetamide as a crystal (353 mg).
¹H-NMR (CDCl₃) δ: 2.73 (d, 3H), 3.50 (s, 2H), 5.25 (br, 1H) , 7.13-7.16 (m, 2H), 7.25-7.36 (m, 4H), 7.52-7.55 (m, 2H). MS (m/z) 304 (MH⁺), Rt = 3.32 min.

### Examples 54-55:

The compounds shown in Table 11 were prepared in a similar manner to the preparation of Example 53 using appropriate starting materials.

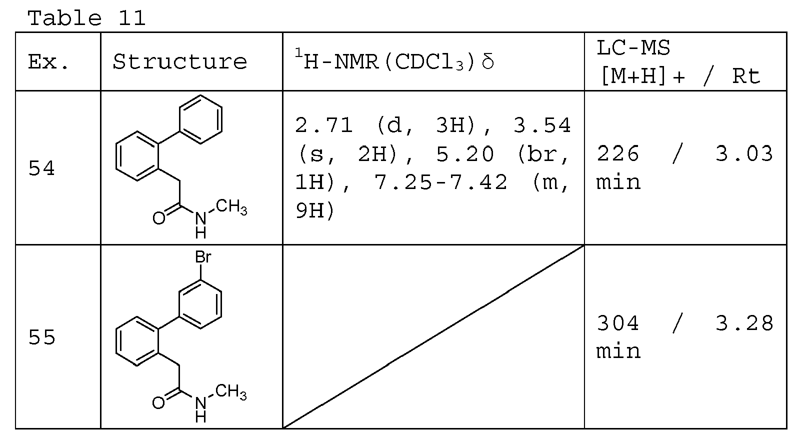

### Example 56:

### 2-(Biphenyl-2-yl)propanamide

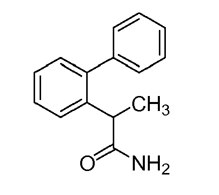

2-(Biphenyl-2-yl)propanoic acid prepared in Reference Example 27 (1.03 g) was suspended in dichloromethane (8.0 ml). To the reaction was added DMF (0.05 ml) and oxalyl chloride (0.51 ml). The mixture was stirred at room temperature for 1 hour and concentrated to give a pale yellow crystal. The resultant cryastal was dissolved in THF (2.0 ml), aqueous 28% ammonia (1.5 ml) was added to the reaction, and the mixture was stirred at room temperature overnight. The resultant white solid was collected on a filter and dried to give 2-(biphenyl-2-yl)propanamide as a crystal (0.31 g).
¹H-NMR (CDCl₃) δ: 1.47 (d, 3H), 3.74 (q, 1H) , 5.17 (br, 2H), 7.25-7.53 (m, 9H)
MS (m/z) 226 (MH⁺), Rt = 3.09 min.

### Examples 57-58:

The compounds shown in Table 12 were prepared in a similar manner to the preparation of Example 56 using appropriate starting materials.

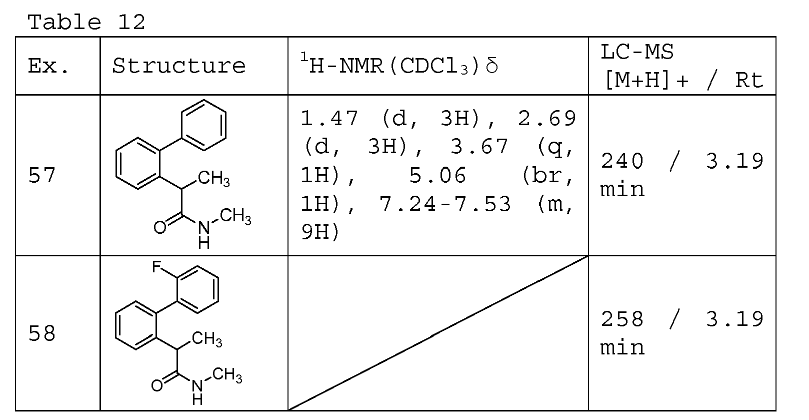

### Example 59:

### 2-(Biphenyl-2-yl)-2-hydroxyacetamide

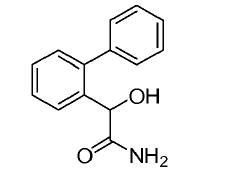

Methyl biphenyl-2-yl(hydroxy)acetate prepared in Reference Example 28 (190 mg) was dissolved in a solution of 7 mol/l ammonia in methanol (10 ml). The reaction was stirred at room temperature for 60 hours. Water (20 ml) was added to the solution, and methanol was evaporated under reduced pressure. The resultant crystal was collected through a filter, washed with water, and dried to give 2-(biphenyl-2-yl)-2-hydroxyacetamide as a crystal (86 mg).
¹H-NMR (DMSO-d₆) δ: 4.92 (d, 1H), 6.02 (d, 1H), 7.24-7.26 (m, 1H), 7.33-7.50 (m, 8H), 7.57-7.60 (m, 2H).
MS (m/z) 228 (MH⁺), Rt = 2.52 min.

### Example 60:

### 2-(2'-Fluorobiphenyl-2-yl)-2-hydroxyacetamide

The following compound was prepared in a similar manner to the preparation of Example 59 using the compound prepared in Reference Example 29 as a starting material. 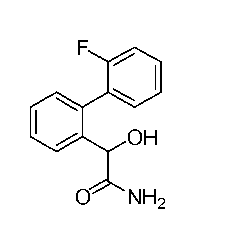 ¹H-NMR (DMSO-d₆) δ: 4.74 (br, 1H), 6.04 (d, 1H), 7.22-7.53 (m, 10H).
MS (m/z) 246 (MH⁺), Rt = 2.56 min.

### Example 61:

### 2-(Biphenyl-2-yl)-2-methoxyacetamide

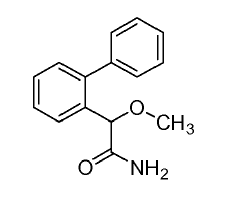

Biphenyl-2-yl(methoxy)acetic acid prepared in Reference Example 30 (506 mg) was dissolved in dichloromethane (10 ml). To the reaction was added DMF (0.05 ml) and a solution of 2 M oxalyl chloride in dichloromethane (2.1 ml). The mixture was stirred at room temperature for 1 hour and concentrated to give a brown crystal. The resultant crystal was dissolved in THF (10 ml) and to the reaction was added aqueous 28% ammonia (2 ml). The mixture was stirred at room temperature for 2 days. The reaction solution was treated with aqueous 10 % citric acid, extracted with ethyl acetate, washed with brine, and dried over anhydrous magnesium sulfate. The solvent was evaporated to give 2-(biphenyl-2-yl)-2-methoxyacetamide as a crystal (475 mg).
¹H-NMR (CDCl₃) δ: 3.06 (s, 3H), 4.83 (s, 1H) , 5.81 (br, 1H) , 6.76 (br, 1H), 7.26-7.51 (m, 9H).
MS (m/z) 242 (MH⁺), Rt = 3.05 min.

### Examples 62-63:

The compounds shown in Table 13 were prepared in a similar manner to the preparation of Example 61 using appropriate starting materials.

**Table 13**

| Ex. | Structure | ¹H-NMR (CDCl₃) δ | LC-MS [M+H] + / Rt |
|---|---|---|---|
| 62 | 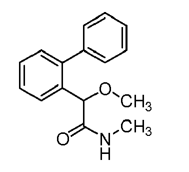 | 2.85 (d, 3H), 3.04 (s, 3H), 4.81 (s, 1H), 6.83 (br, 1H), 7.28- 7.32 (m, 1H), 7.34- 7.44 (m, 6H), 7.53 (br, 1H) , 7.55 (br, 1H) . | 256 / 3.13 min |
| 63 | 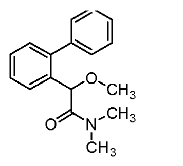 | 2.43 (s, 3H), 2.90 (s, 3H), 3.37 (s, 3H), 4.80 (s, 1H), 7.30- 7.56 (m, 9H). | 270 / 3.15 min |

### Example 64:

### 2-(2',3',5'-Trichlorobiphenyl-2-yl)acetamide

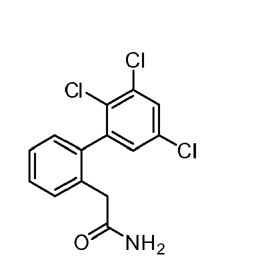

2-Bromoacetamide (3.84 g), 2,3,5-trichloro-phenylboronic acid (6.04 g), tetrakis(triphenylphosphine)palladium (4.12 g), 2 N sodium carbonate aqueous solution (27 ml), barium hydroxide octahydrate (1.12 g), and ethanol (24 ml) was added to toluene (84 ml). After substituting with nitrogen, the mixture was heated to reflex for 20 hours, treated with water, extracted with ethyl acetate, washed with brine, and dried over anhydrous magnesium sulfate. The solvent was evaporated under reduced pressure, and the residue was purified on silica gel column chromatography to give 2-(2',3',5'-trichlorobiphenyl-2-yl)acetamide as a white crystal (640 mg) .
¹H-NMR (CDCl₃) δ: 3.36 (d, 1H) , 3.47 (d, 1H) , 5.25 (br, 1H) , 5.33 (br, 1H), 7.17-7.19 (m, 2H), 7.36-7.46 (m, 3H), 7.53 (d, 1H) .
MS (m/z) 314 (MH⁺) , Rt = 4.41 min*.

### Examples 65-108:

The compounds shown in Table 14 were prepared in a similar manner to the preparation of Example 64 using appropriate starting materials.

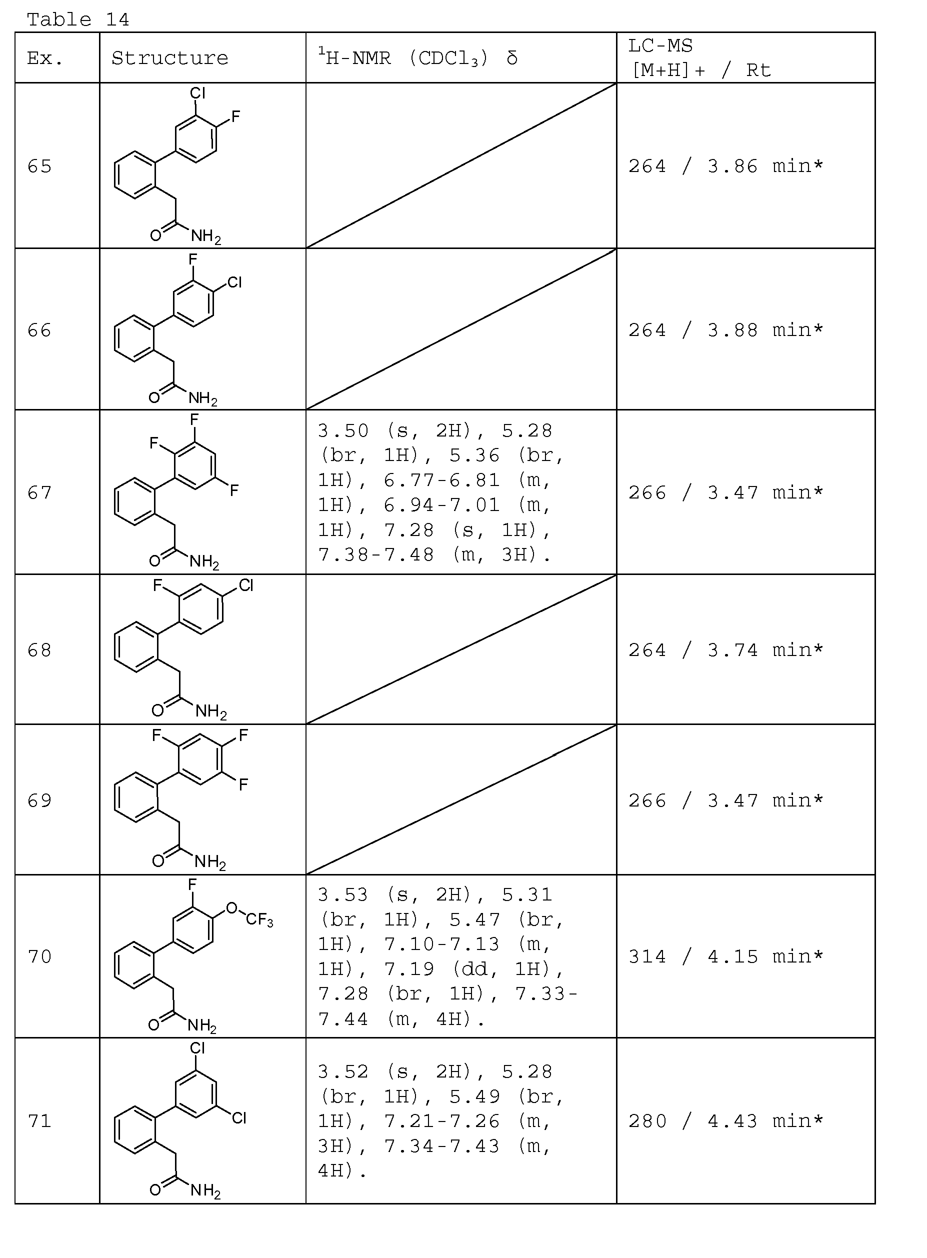

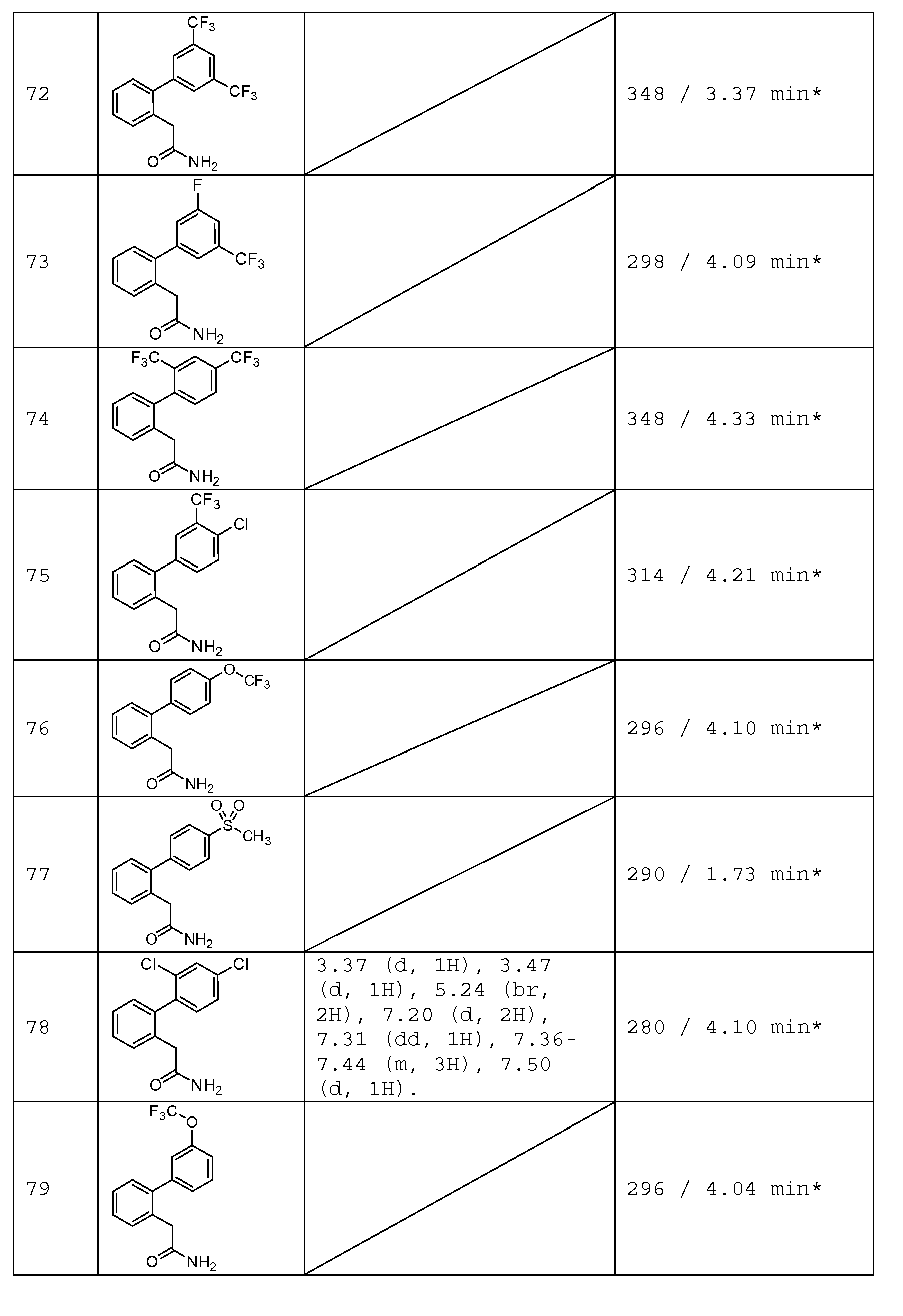

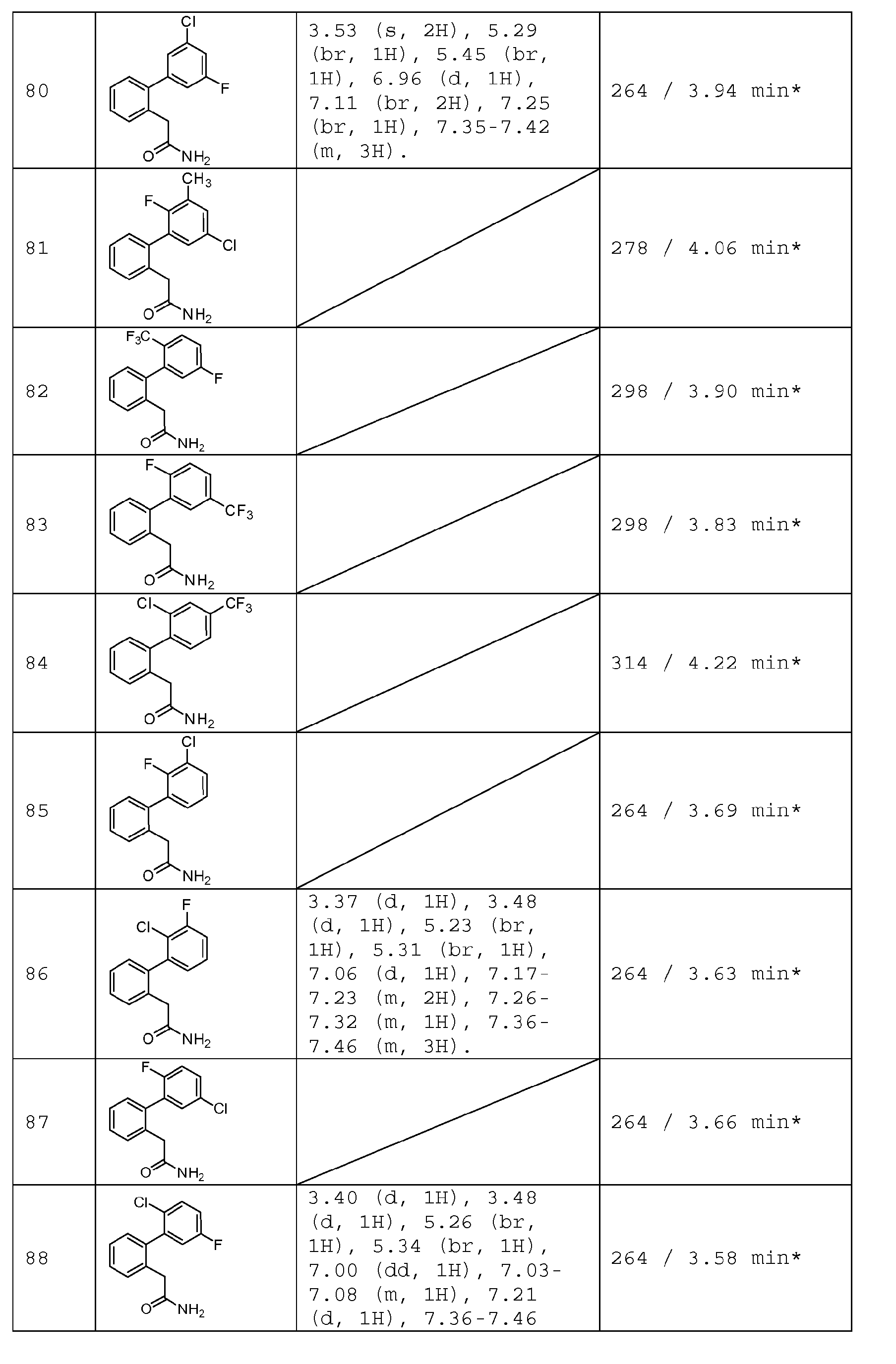

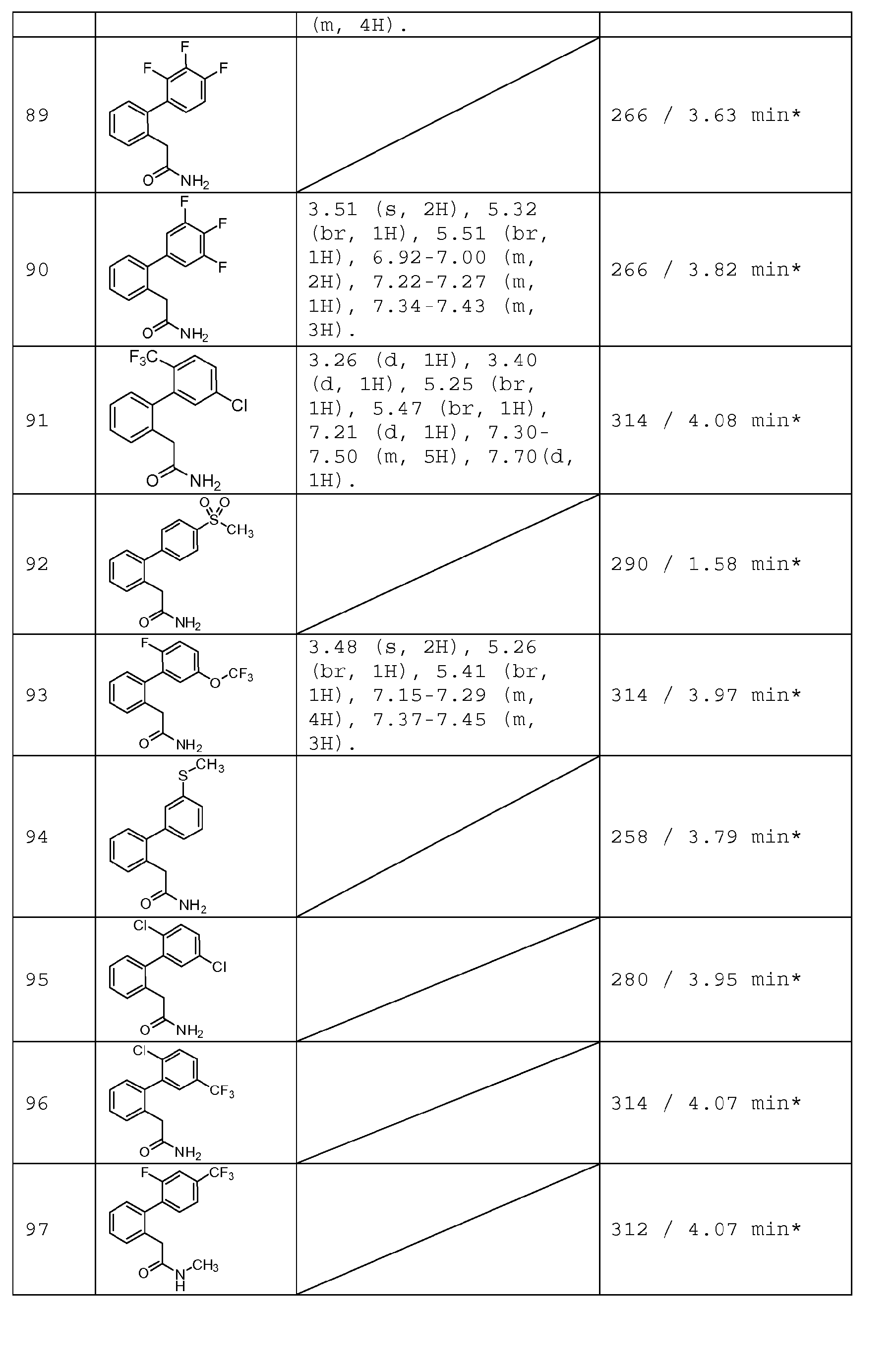

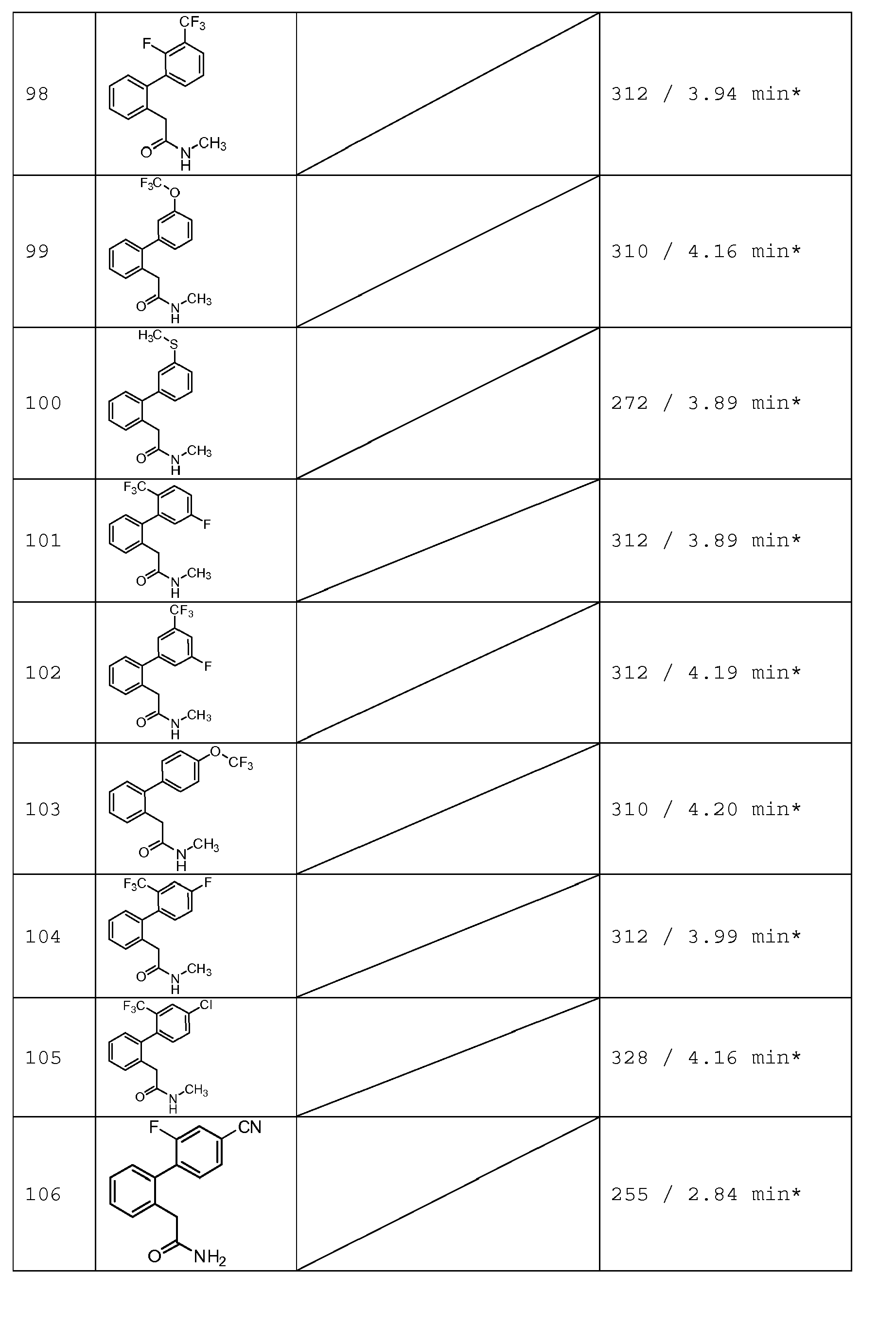

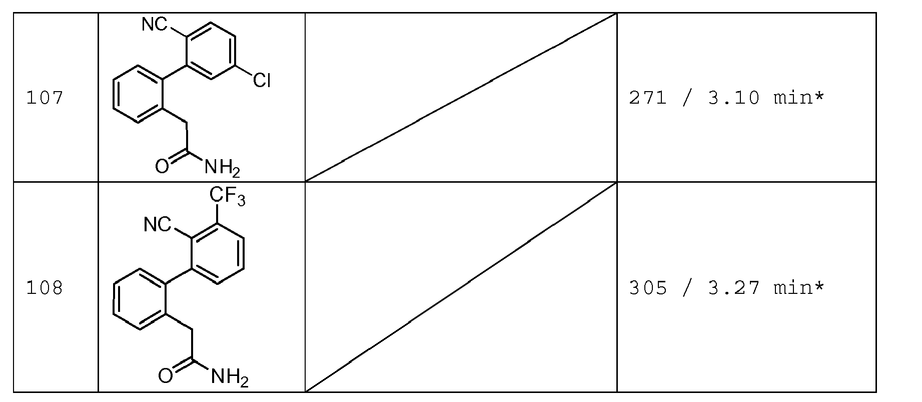

### Examples 109-173:

The compounds shown in Table 15 were prepared in a similar manner to the preparation of Example 1 using appropriate starting materials.

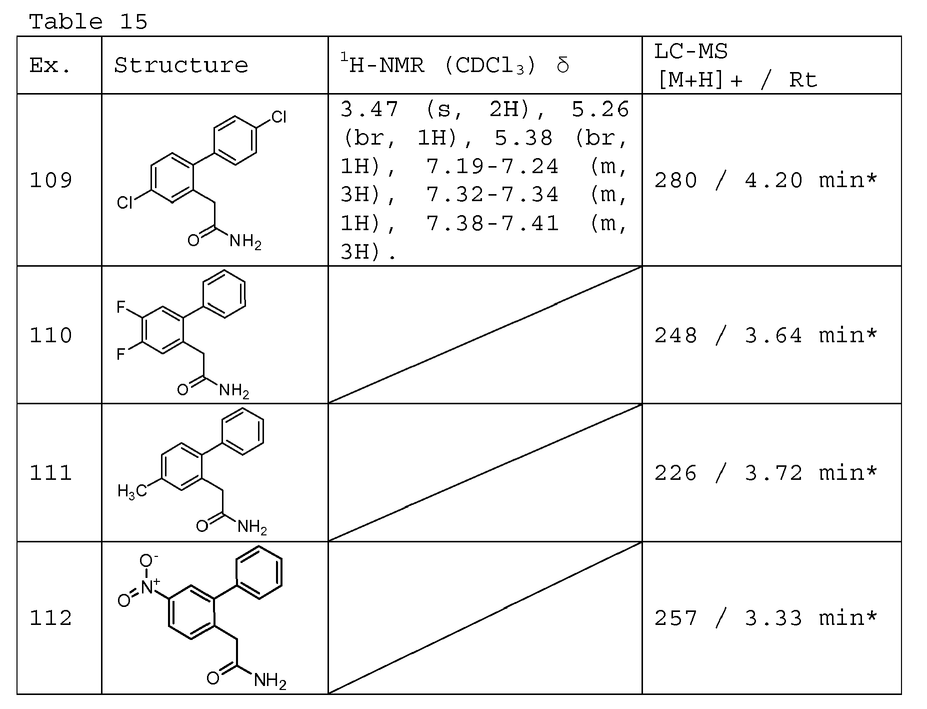

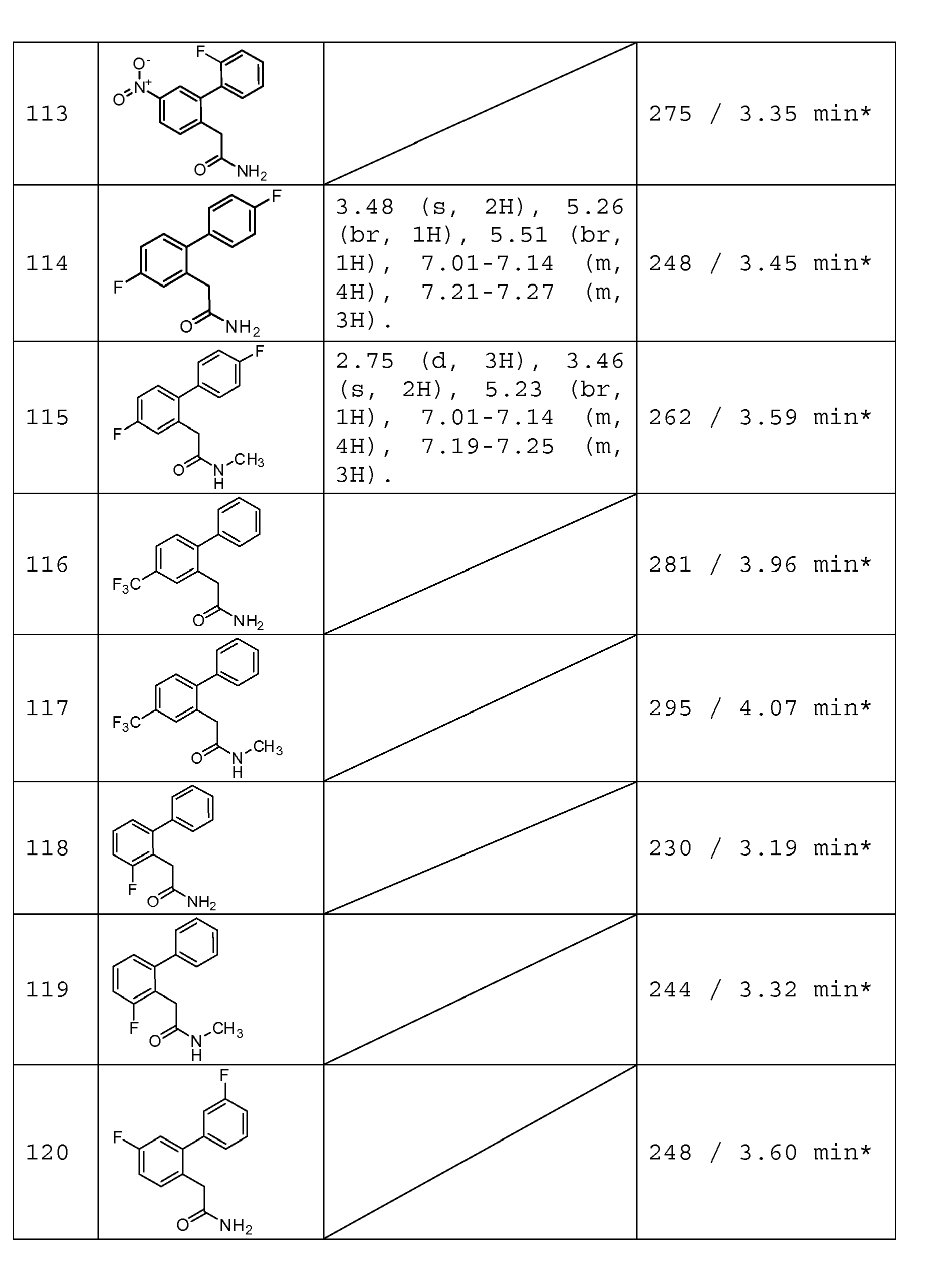

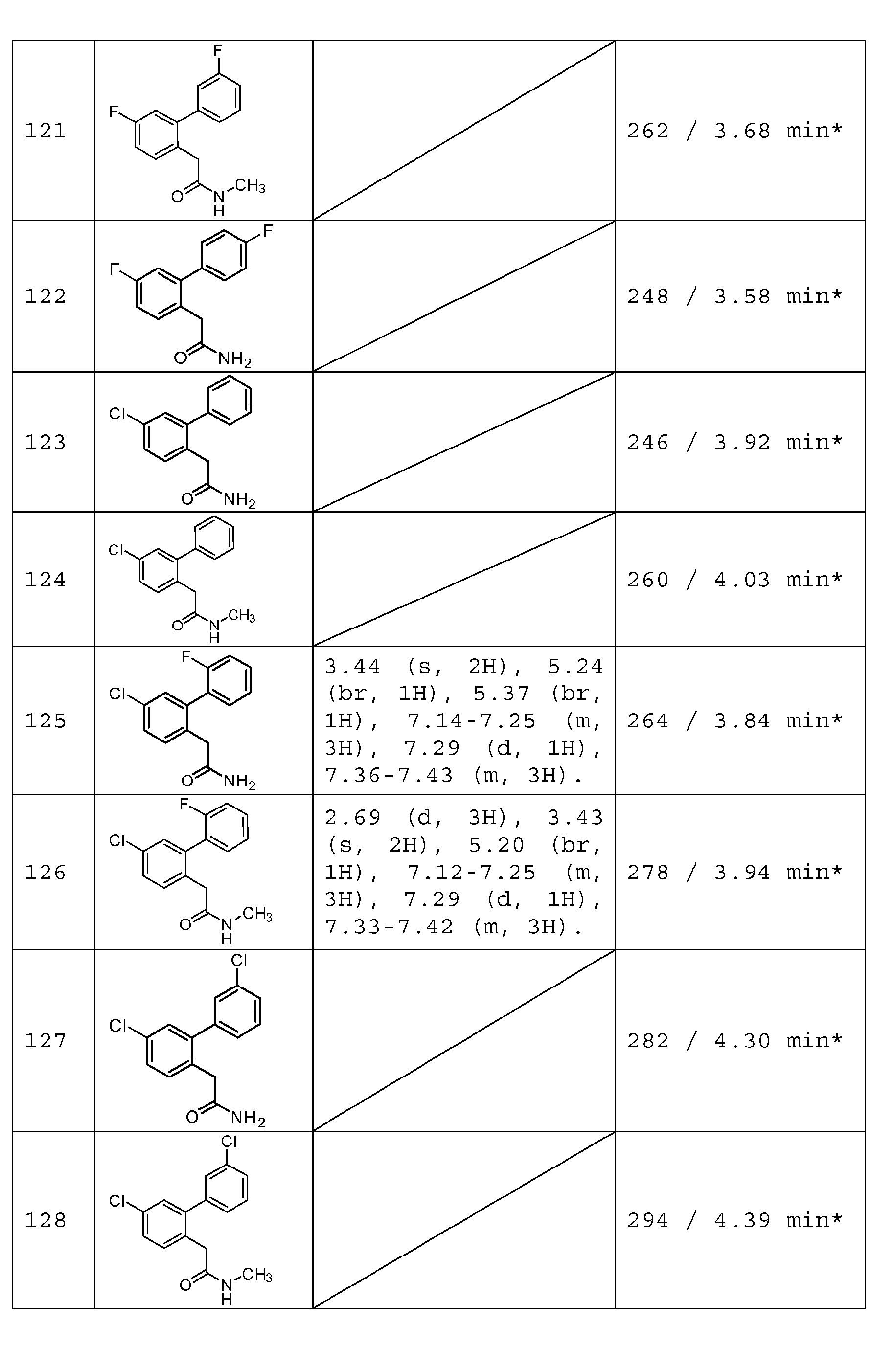

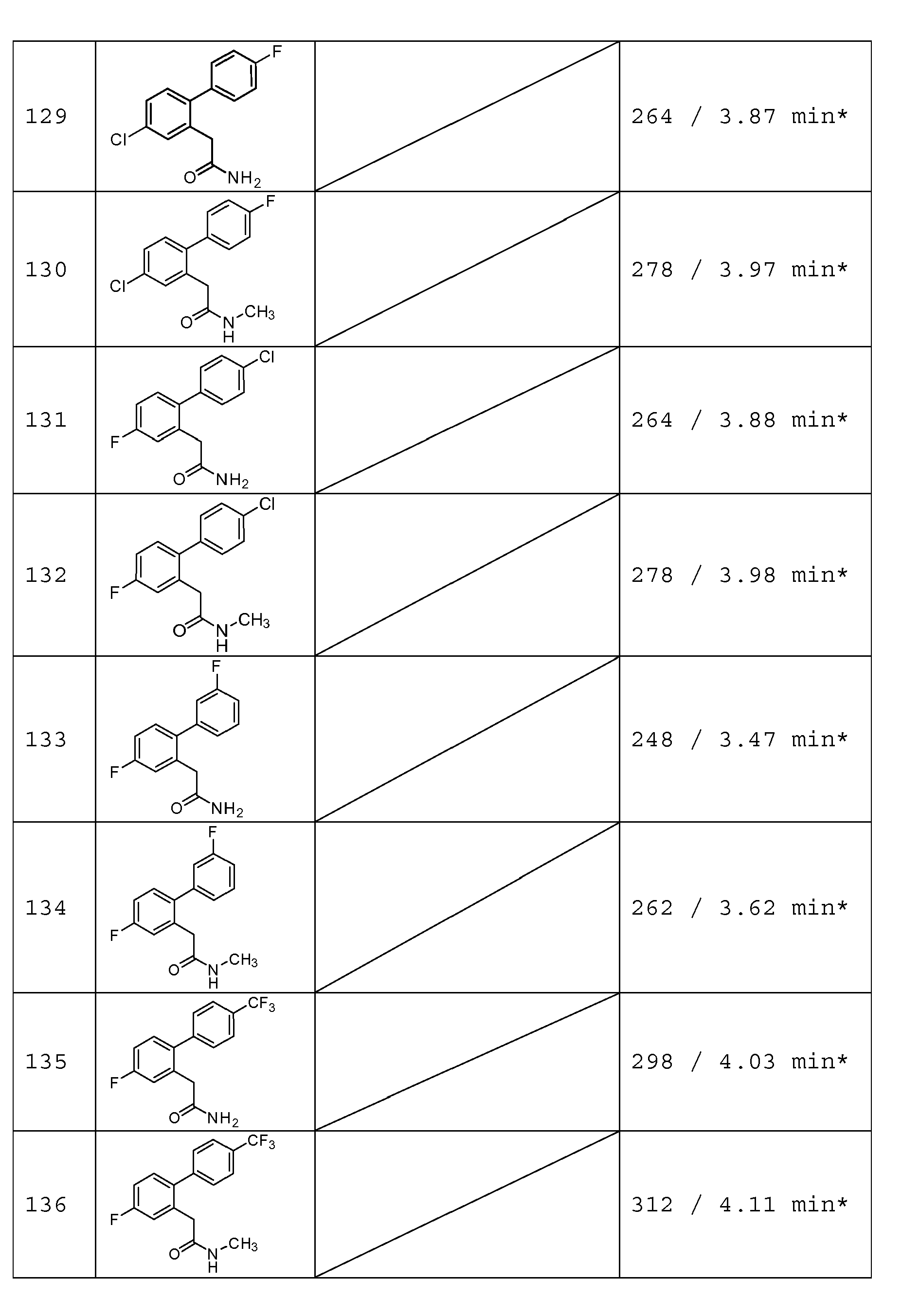

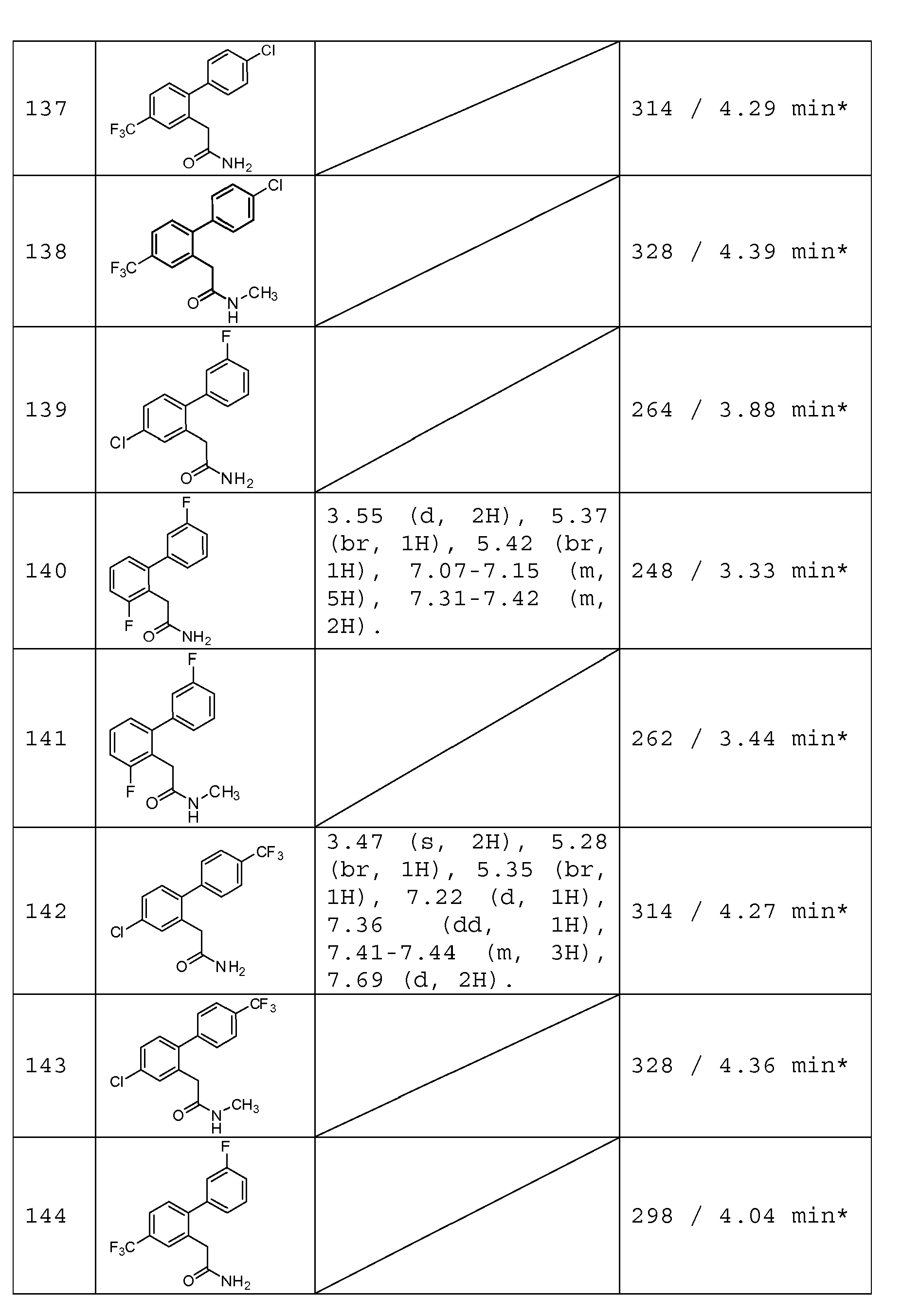

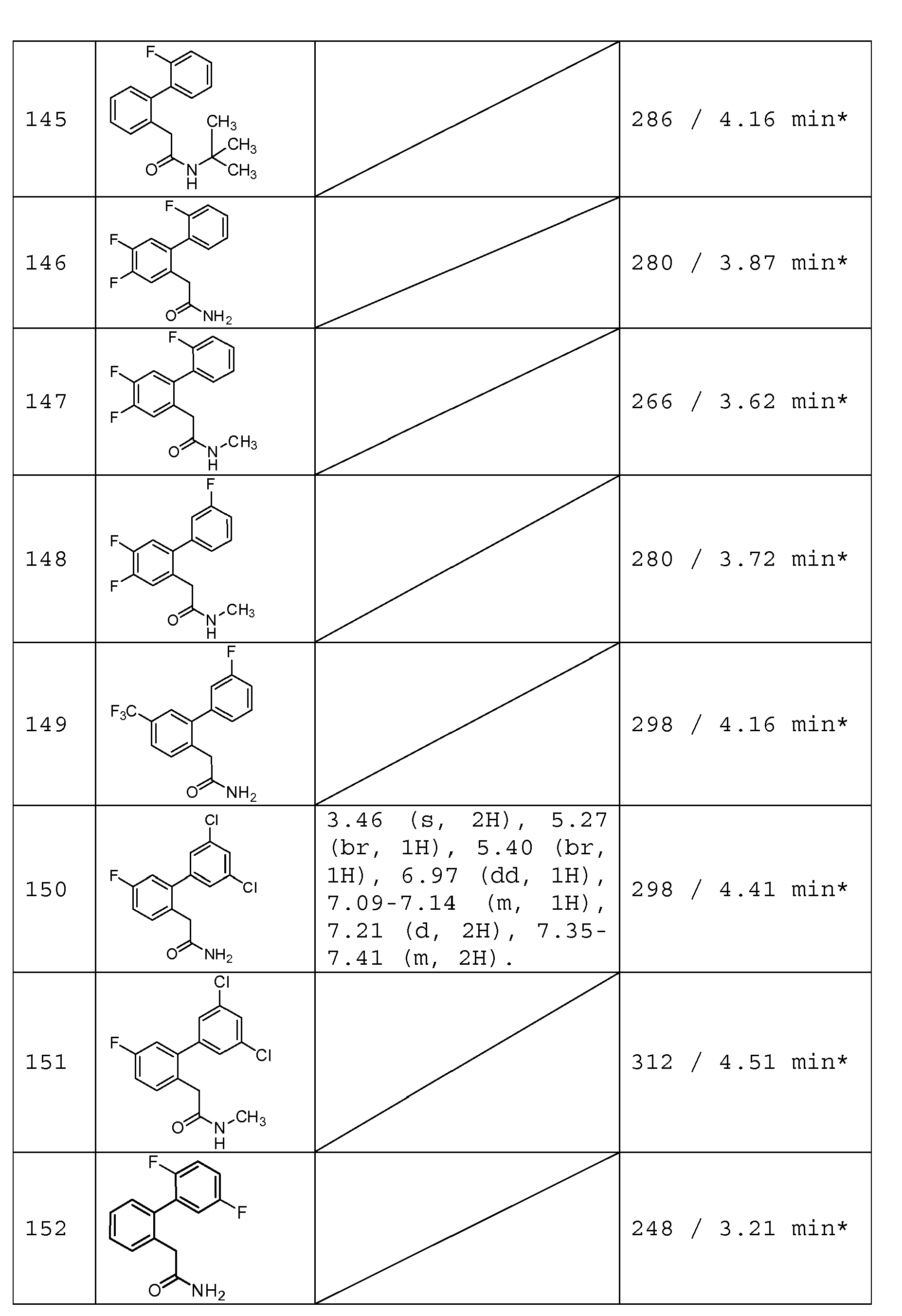

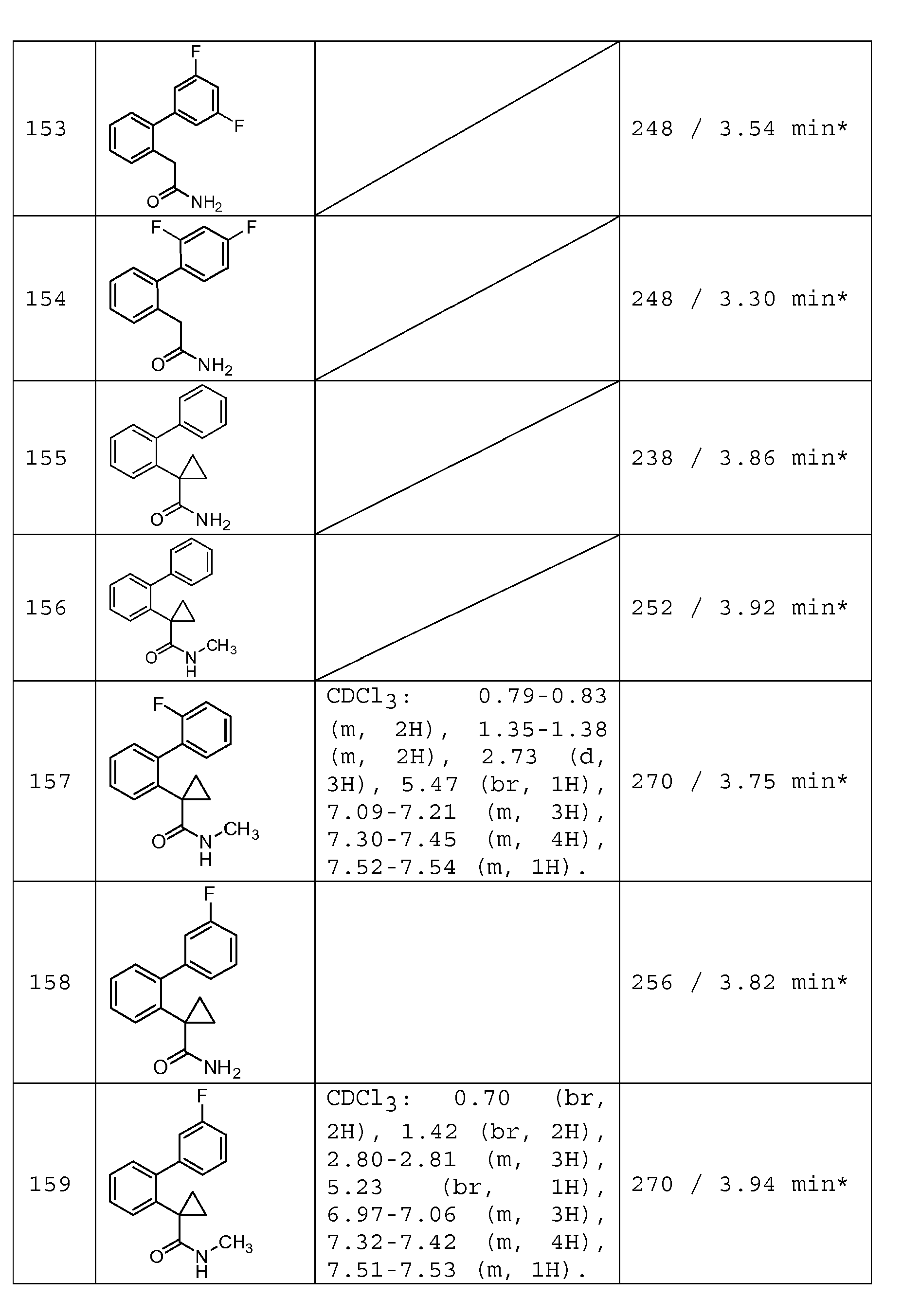

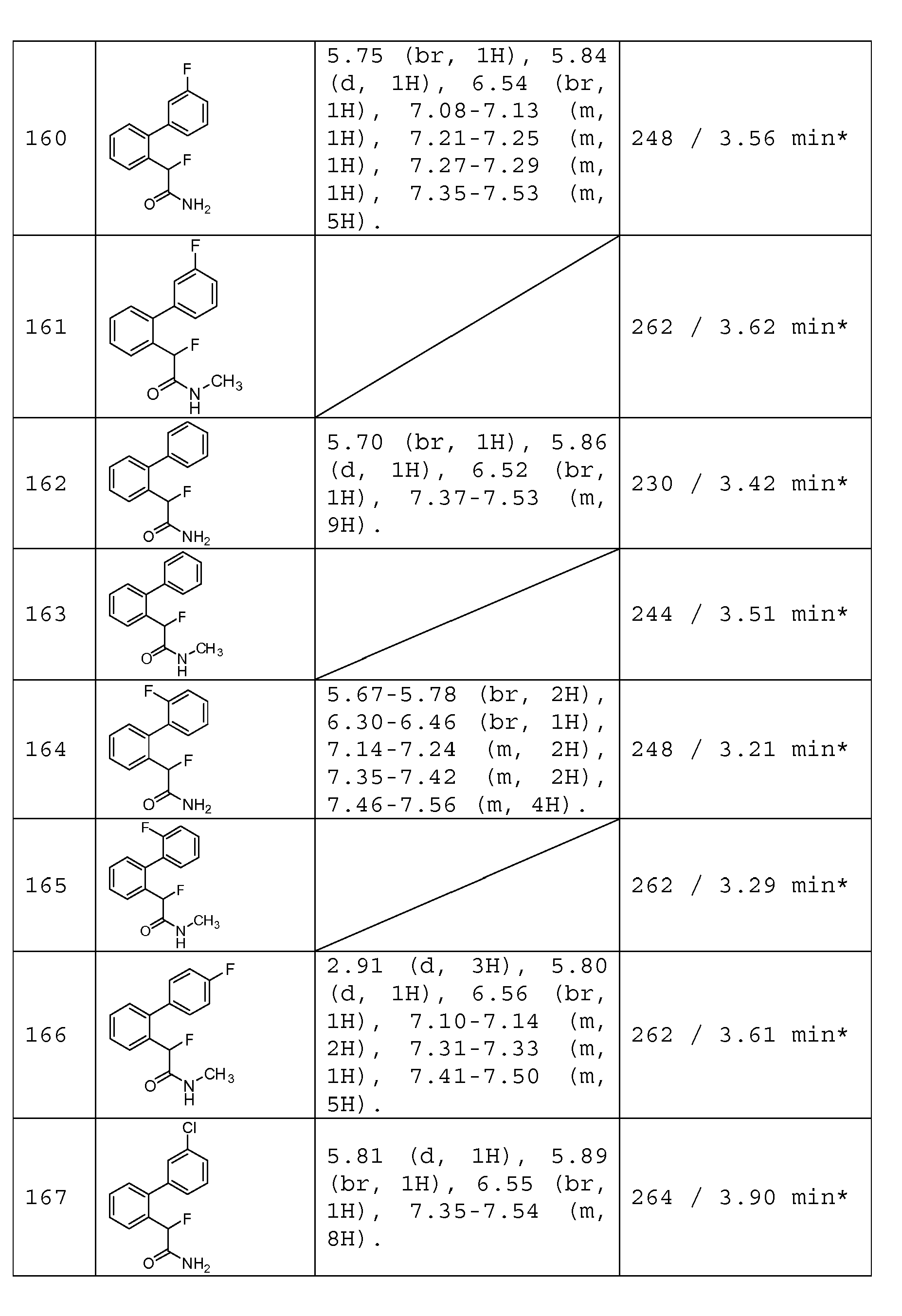

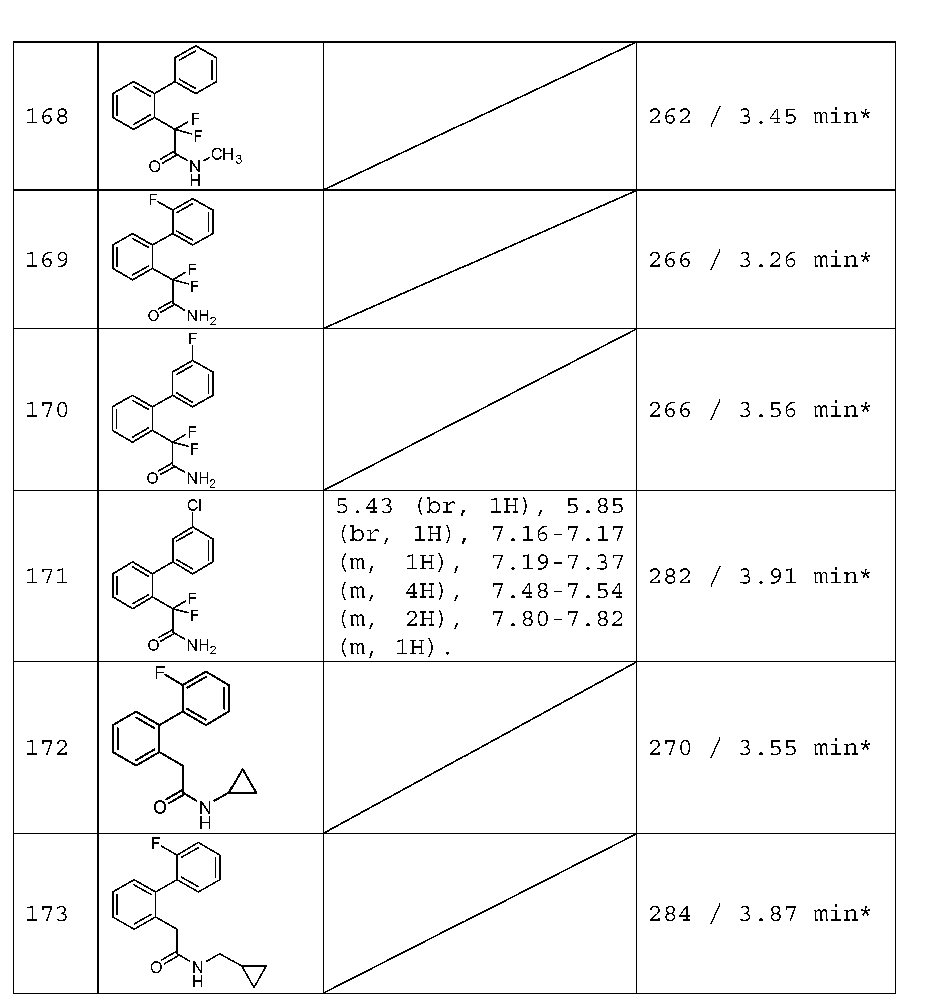

### Example 174:

The compound shown in Table 16 was prepared in a similar manner to the preparation of Example 49 using an appropriate starting material.

**Table 16**

| Ex. | Structure | ¹H-NMR (CDCl₃) δ | LC-MS [M+H] +/ Rt |
|---|---|---|---|
| 174 | 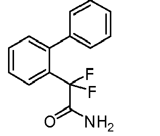 | 5.40 (br, 1H) , 5.68 (br, 1H), 7.27-7.29 (m, 3H), 7.35-7.36 (m, 3H), 7.46- 7.54 (m, 2H), 7.80-7.83 (m, 1H). | 248 / 3.42 min* |

### Examples 175-177:

The compounds shown in Table 17 were prepared in a similar manner to the preparation of Example 56 using appropriate starting materials.

**Table 17**

| Ex. | Structure | LC-MS [M+H]+ / Rt |
|---|---|---|
| 175 | 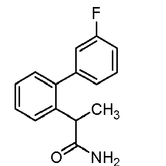 | 244 / 3.73 min* |
| 176 | 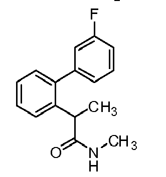 | 258 / 3.85 min* |
| 177 | 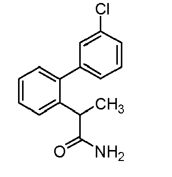 | 260 / 4.09 min* |

### Examples 178-198:

The compounds shown in Table 18 and Table 19 were prepared in a similar manner to the preparation of Example 59 using appropriate starting materials.

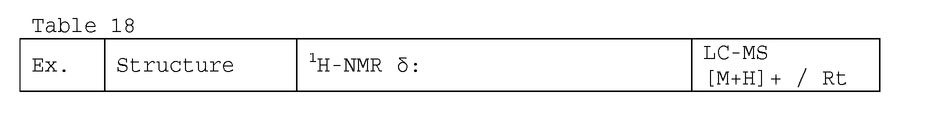

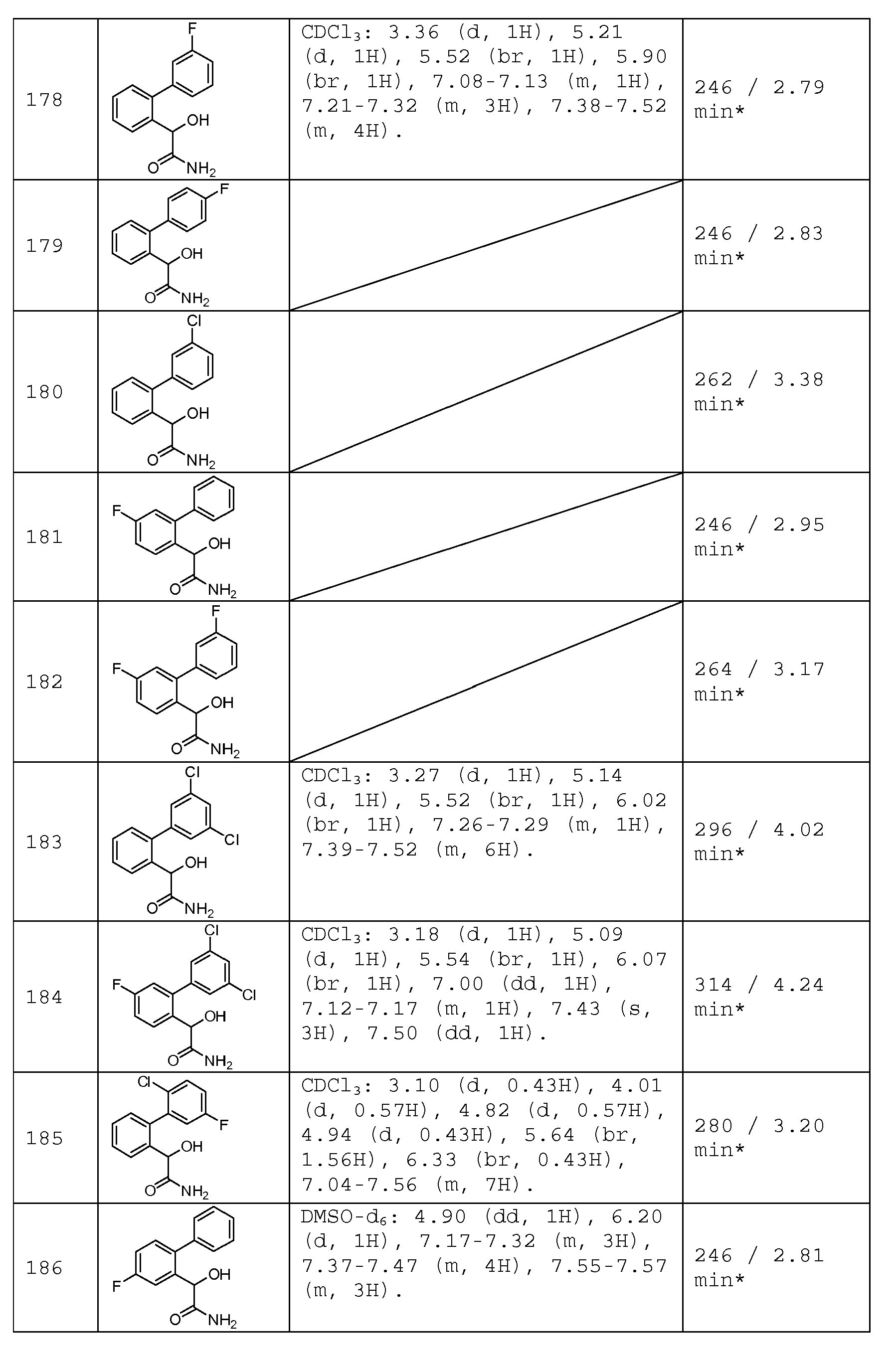

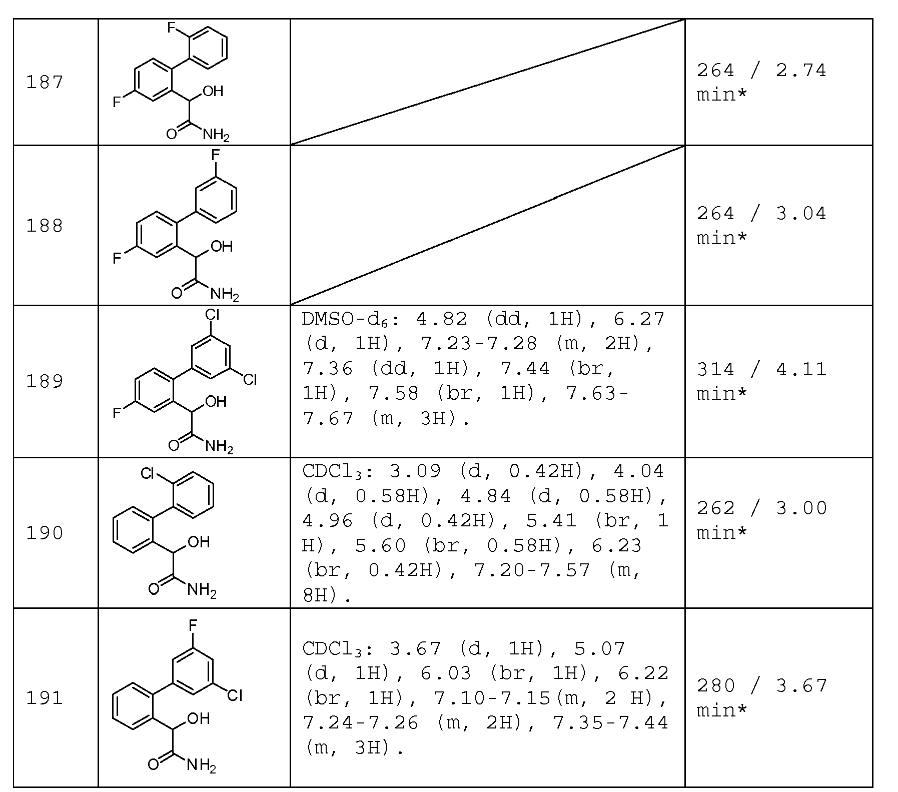

**Table 19**

| Ex. | Structure | ¹H-NMR (CDCl₃) δ | LC-MS [M+H]⁺ / Rt |
|---|---|---|---|
| 192 | 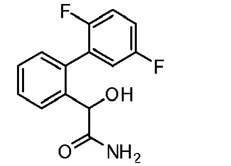 | DMSO-d₆: 4.75 (br, 1H), 6.10 (br, 1H), 7.24-7.39 (m, 6H), 7.42-7.46 (m, 2H), 7.53 (br, 2H). | 264 / 2.71 min* |
| 193 | 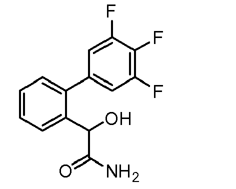 | CDCl₃: 3.22 (d, 1H), 5.14 (d, 1H), 5.64 (br, 1H), 6.16 (br, 1H), 7.20 (dd, 2H), 7.25-7.28 (m, 1H), 7.39-7.52 (m, 3H). | 282 / 3.48 min* |
| 194 | 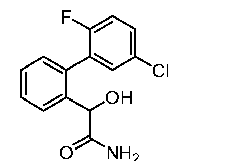 | DMSO-d₆: 4.72 (br, 1H), 6.10 (br, 1H), 7.25 (d, 1H), 7.32-7.79 (m, 8H). | 280 / 3.24 min* |
| 195 | 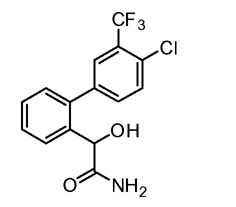 | CDCl₃: 3.30 (br, 1H), 5.07 (d, 1H), 5.74 (br, 1H), 6.18 (br, 1H), 7.27-7.30 (m, 1H), 7.40-7.51 (m, 3H), 7.56 (d, 1H), 7.65-7.73 (m, 1H) , 7.84 (d, 1H) . | 330 / 3.99 min* |
| 196 | 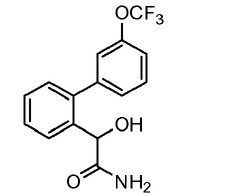 | CDCl₃: 3.32 (d, 1H), 5.17 (d, 1H), 5.56 (br, 1H), 5.97 (br, 1H), 7.24-7.29 (m, 1H), 7.30-7.32 (m, 1H), 7.38-7.52 (m, 6H). | 312 / 3.74 min* |
| 197 | 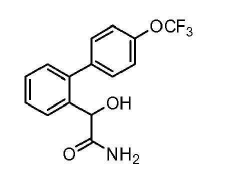 | CDCl₃: 3.30 (d, 1H), 5.17 (d, 1H), 5.59 (br, 1H), 6.00 (br, 1H), 7.28-7.31 (m, 3H), 7.38-7.46 (m, 2H), 7.48-7.56 (m, 3H). | 312 / 3.78 min* |
| 198 | 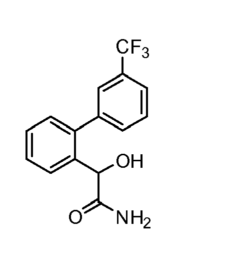 | CDCl₃: 3.32 (d, 1H), 5.12 (d, 1H), 5.64 (br, 1H), 6.03 (br, 1H), 7.30-7.33 (m, 1H), 7.40-7.47 (m, 2H), 7.49-7.52 (m, 1H), 7.55-7.59 (m, 1H), 7.65-7.67 (m, 1H), 7.73-7.75 (m, 2H). | 296 / 3.57 min* |

### Examples 199-206:

The compounds shown in Table 20 were prepared in a similar manner to the preparation of Example 61 using appropriate starting materials.

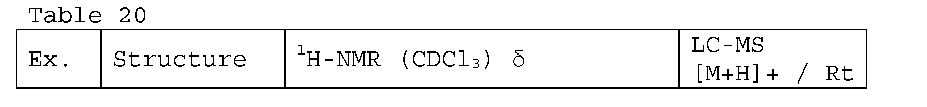

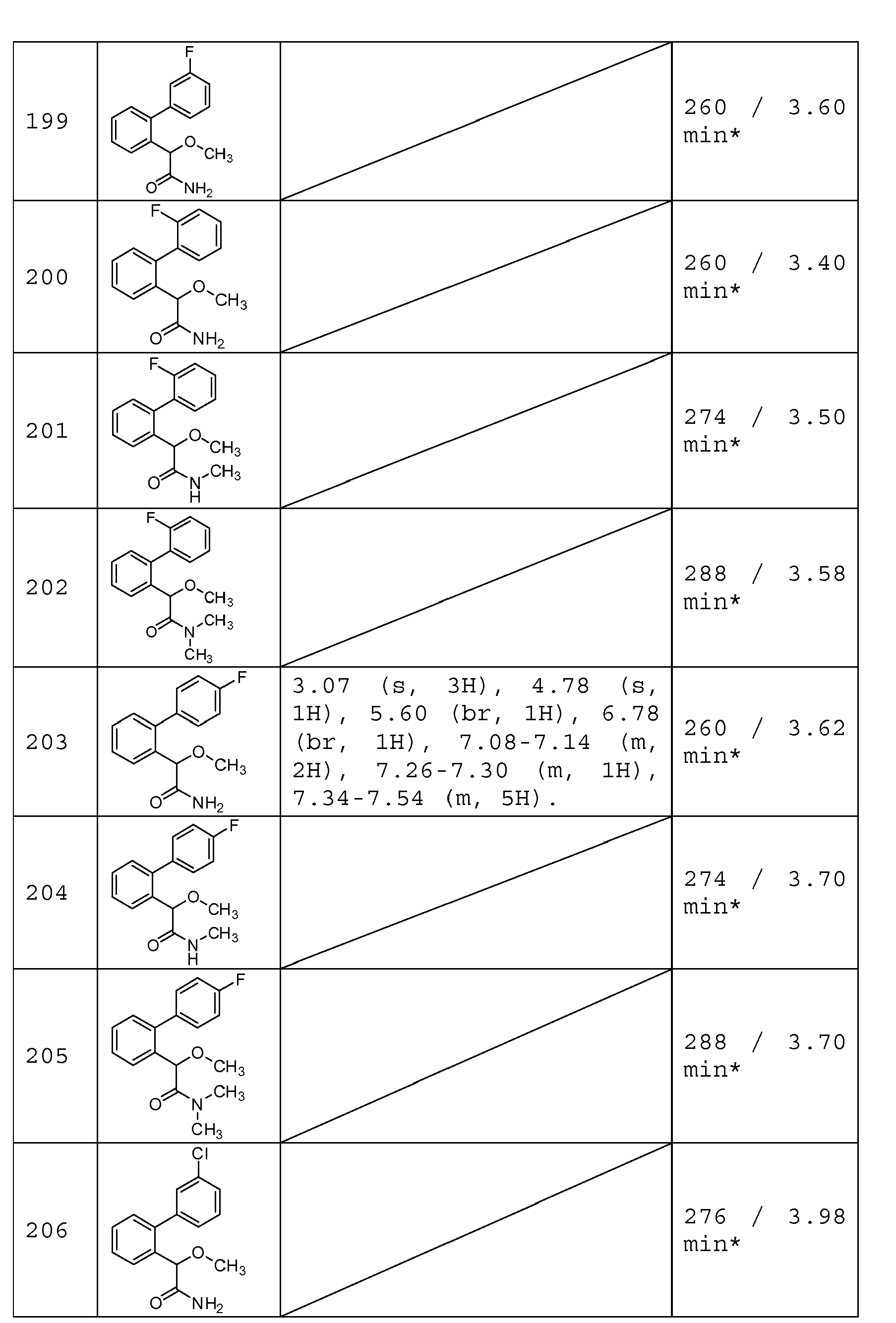

### Examples 207 and 208:

### 2-(Biphenyl-2-yl)-2R-hydroxyacetamide and 2-(biphenyl-2-yl)-2S-hydroxyacetamide

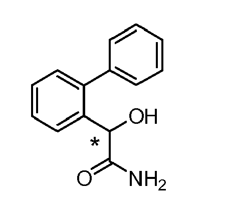

The fraction of the compound prepared in Example 59 was resolved on Daicel CHIRALPAK^{®} IA (mobile phase: HexEtOH-MeOH) to afford first peak (enantiomer A) and second peak (enantiomer B).
Example 207 (enantiomer A): retention time 4.13 min, Chiral HPLC (Chiralpak^{®} IA, 0.46 cmI.D. x 25 cmL, mobile phase: Hex/EtOH/MeOH = 60/20/20, flow rate: 1.0 ml/min, temperature: 40 °C, wavelength: 260 nm), [α] D20.4 = +127.0° (c 0.521, CH₃OH)
Example 208 (enantiomer B): retention time 6.99 min, Chiral HPLC (Chiralpak^{®} IA, 0.46 cmI.D. x 25 cmL, mobile phase: Hex/EtOH/MeOH = 60/20/20, flow rate: 1.0 ml/min, temperature: 40 °C, wavelength: 260 nm), [α] D20.4 = - 125.8° (c 0.985, CH₃OH)

### Examples 209-214:

The compounds shown in Table 21 were prepared in a similar manner to the preparation of Examples 207 and 208 using appropriate starting materials.

**Table 21**

| Ex. | Structure | Resolution Condition | Note |
|---|---|---|---|
| 209 | 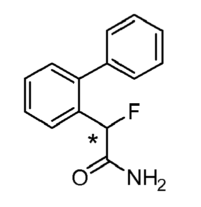 | Retention time 3.66 min Chiral HPLC (Chiralpak^{®} AS-H, 0.46cmI.D. x 25 cmL, mobile phase:100% MeOH, flow rate: 1.0 ml/min, temperature: 40 °C, wavelength: 270nm ) | 2-(Biphenyl-2-yl)-2-fluoro-acetamide (enantiomer A), optically active substance |
| 210 | 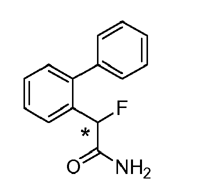 | Retention time 4.47 min Chiral HPLC (Chiralpak^{®} AS-H, 0.46cmI.D. x 25 cmL, mobile phase:100% MeOH, flow rate: 1.0 ml/min, temperature: 40 °C, wavelength: 270nm ) | 2-(Biphenyl-2-yl)-2-fluoro-acetamide (enantiomer B), optically active substance |
| 211 | 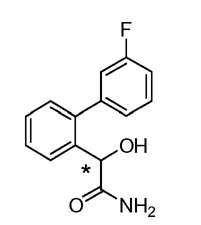 | Retention time 4.01 min Chiral HPLC (Chiralpak^{®} IA, 0.46cmI.D. x 25 cmL, mobile phase: Hex/EtOH/MeOH = 60/20/20, flow rate: 1.0 ml/min, temperature: 40 °C, wavelength: 260nm) | 2- (3' -Fluorobiphenyl-2-yl)-2-hydroxy-acetamide (enantiomer A), optically active substance |
| 212 | 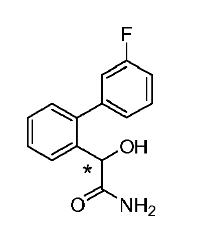 | Retention time 5.68 min Chiral HPLC (Chiralpak^{®} IA, 0.46cmI.D. x 25 cmL, mobile phase: Hex/EtOH/MeOH = 60/20/20, flow rate: 1.0 ml/min, temperature: 40 °C, wavelength: 260nm ) | 2- (3' -Fluorobiphenyl-2-yl)-2-hydroxyacetamide (enantiomer B), optically active substance |
| 213 | 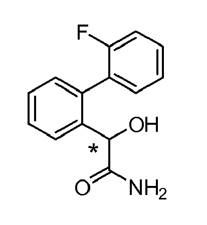 | Retention time 6.14 min Chiral HPLC (Chiralpak^{®} IA, 0.46cmI.D. x 25 cmL, mobile phase: Hex/EtOH/MeOH = 80/15/5, flow rate: 1.0 ml/min, temperature: 40 °C, wavelength: 265nm ) | 2- (2' -Fluorobiphenyl-2-yl)-2-hydroxy-acetamide (enantiomer A), optically active substance |
| 214 | 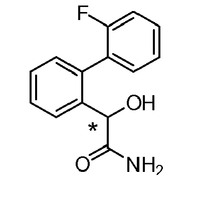 | Retention time 9.17 min Chiral HPLC (Chiralpak^{®} IA, 0.46cmI.D. x 25 cmL, mobile phase: Hex/EtOH/MeOH = 80/15/5, flow rate: 1.0 ml/min, temperature: 40 °C, wavelength: 265nm) | 2- (2' -Fluorobiphenyl-2-yl)-2-hydroxy-acetamide (enantiomer B), optically active substance |

### Examples 215 and 216:

### 2-(3',5'-Dichlorobiphenyl-2-yl)-2R-hydroxyacetamide and 2-(3',5'-dichlorobiphenyl-2-yl)-2S-hydroxyacetamide

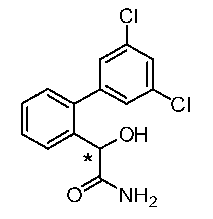

### (Step 1)

Example 183 (2.62 g) was dissolved in toluene (34 ml), and to the reaction was added pyridinium p-toluenesulfonic acid (22 mg) and (*S*)-allyl-2-oxabicyclo[3,3,0]oct-8-ene (2.66 g). The mixture was stirred at room temperature for 1 hour, and the reaction was quenched by adding saturated sodium bicarbonate aqueous solution. The reaction mixture was extracted with ethyl acetate, washed with brine, and then with anhydrous magnesium sulfate. The solvent was evaporated under reduced pressure. The diastereomers were resolved by silica gel column chromatography to give the first-eluted compound (1.74 g) and the second-eluted compound (1.66 g) each as a white crystal.
The first-eluted compound: ¹H-NMR (CDCl₃) δ: 1.48-1.68 (m, 6H), 1.75-1.81 (m, 1H), 1.90-1.94 (m, 1H), 2.12-2.17 (m, 1H), 2.29-2.34 (m, 1H), 3.04(dd, 1H),3.50-3.56 (m, 1H), 5.07-5.14 (m, 2H), 5.20(s,1H),5.43 (br, 1H), 5.78-5.88 (m, 1H), 6.63 (br, 1H), 7.18(dd, 1H),7.29-7.40 (m, 3H), 7.48(d, 1H), 7.53 (d, 2H) .
The second-eluted compound: ¹H-NMR (CDCl₃) δ: 0.94-1.03 (m, 1H), 1.37-1.52 (m, 5H), 1.70-1.77 (m, 1H), 1.93-1.98 (m, 1H), 2.09-2.14 (m, 1H), 2.21-2.26 (m, 1H), 3.71-3.77 (m, 1H), 3.82-3.87 (m, 1H), 5.06-5.14 (m, 2H), 5.30 (s,1H), 5.47 (br, 1H), 5.75-5.85 (m, 1H), 6.74 (br, 1H), 7.20-7.22 (m, 1H), 7.32-7.42 (m, 3H), 7.48-7.51 (m, 1H), 7.54 (br, 2H).

### (Step 2)

The ether compound (1.74 g) which is the first-eluted compound was dissolved in methanol (20 ml), and to the reaction was added p-toluenesulfonic acid monohydrate (74 mg). The mixture was stirred at room temperature for 8 hours, and the reaction was quenched by adding saturated sodium bicarbonate aqueous solution. The mixture was extracted with chloroform and dried over anhydrous magnesium sulfate. The solvent was evaporated under reduced pressure and the residue was purified on silica gel column chromatography to give the alcohol compound as a white amorphous product (896 mg).
Example 215 (enantiomer A): retention time 13.3 min, Chiral HPLC (Daicel Chiralpak^{®} AD-H, 0.46 cm I.D. x 25 cmL, mobile phase: Hex/EtOH = 90/10, flow rate: 1.0 ml/min, temperature: 30 °C, wavelength: 254 nm).

The ether compound (1.74 g) which is the second-eluted compound was dissolved in methanol (20 ml), and to the reaction was added p-toluenesulfonic acid monohydrate (71 mg). The mixture was stirred at room temperature for 8 hours, and the reaction was quenched by adding saturated sodium bicarbonate aqueous solution. The mixture was extracted with chloroform and dried over anhydrous magnesium sulfate. The solvent was evaporated under reduced pressure and the residue was purified on silica gel column chromatography to give the alcohol compound as a white amorphous product (783 mg).
Example 216 (enantiomer B): retention time 14.2 min, Chiral HPLC (Daicel Chiralpak^{®} AD-H, 0.46 cm I.D. x 25 cmL, mobile phase: Hex/EtOH = 90/10, flow rate: 1.0 ml/min, temperature: 30 °C, wavelength: 254 nm).

### Examples 217-220:

The compounds shown in Table 22 were prepared in a similar manner to the preparation of Examples 215 and 216 using appropriate starting materials.

**Table 22**

| Ex. | Structure | Resolution Condition | Note |
|---|---|---|---|
| 217 | 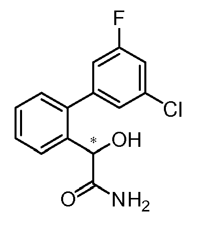 | Retention time 12.7 min Chiral HPLC (Daicel Chiralpak^{®} AD-H, 0.46 cm I.D. x 25 cmL, mobile phase: Hex/EtOH = 90/10, flow rate: 1.0 ml/min, temperature: 30 °C, wavelength: 254 nm). | 2-(3'-Chloro-5'-fluorobiphenyl-2-yl)-2-hydroxy-acetamide (enantiomer A), optically active substance |
| 218 | 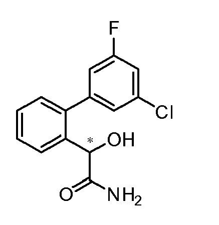 | Retention time 13.3 min Chiral HPLC (Daicel Chiralpak^{®} AD-H, 0.46 cm I.D. x 25 cmL, mobile phase: Hex/EtOH = 90/10, flow rate: 1.0 ml/min, temperature: 30 °C, wavelength: 254 nm). | 2-(3'-Chloro-5'-fluorobiphenyl-2-yl)-2-hydroxy-acetamide (enantiomer B), optically active substance |
| 219 | 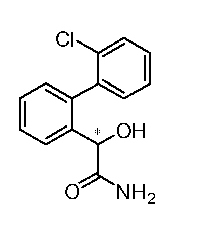 | Retention time 9.0 min Chiral HPLC (Daicel Chiralpak^{®} AD-H, 0.46 cm I.D. x 25cmL, mobile phase: Hex/EtOH = 80/20, flow rate: 1.0 ml/min, temperature: 30 °C, wavelength: 254 nm). | 2-(2'-Chlorobiphenyl-2-yl)-2-hydroxy-acetamide (enantiomer A), optically active substance |
| 220 | 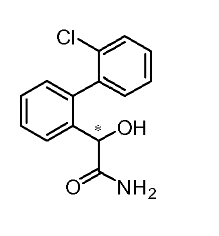 | Retention time 10.2 min Chiral HPLC (Daicel Chiralpak^{®} AD-H, 0.46 cm I.D. x 25 cmL, mobile phase: Hex/EtOH = 80/20, flow rate: 1.0 ml/min, temperature: 30 °C, wavelength: 254 nm). | 2-(2'-chlorobiphenyl-2-yl)-2-hydroxy-acetamide (enantiomer B), optically active substance |

### (Tests)

Hereinafter, pharmaceutical test results of some representative compounds of the present invention and explanations thereof are shown, but the present invention should not be construed to be limited thereto.

In order to evaluate the antiepileptic activity of each drug, maximum electroshock seizure (MES) model, subcutaneously pentetrazol-injected model (scPTZ), and amygdala kindling model in rats were used because they show a good clinical prediction. If a compound exhibits an anticonvulsant activity in any one of theses models, a medicament of such compound is expected to exhibit an antiepileptic activity in clinical use. And, if a compound equally exhibits an anticonvulsant activity in both MES and scPTZ models, a medicament of such compound is expected to be an antiepileptic agent which could exibit a broad antiepileptic spectrum against partial seizures and generalized seizures in clinical use, like valproic acid. Furthermore, 6 Hz psychomotor seizure model shows a characteristic resistant to existing antiepileptic drugs. Thus, if a compound exhibits an anticonvulsant activity in this model, a medicament of such compound is expected to be efficacious for patients refractory to existing antiepileptic drugs. Consequently, if a compound equally shows an activity in MES, scPTZ, and 6 Hz models, such compound can have a clinical utility which is hardly found among existing antiepileptic drugs.
Meanwhile, there are no reports about animal models which provide a good clinical prediction to evaluate the improving efficacy for bipolar disorder. However, bipolar disorder comprises "manic state" and "depressive state", and some animals are reported to be useful as a model to evaluate the improvement of each pathological condition individually. In general, an index of manic state is excessive hyperactivity induced by administering methamphetamine and chlordiazepoxide in combination, and that of depressive state is immobility induced by forced swimming. If a compound shows an improved activity in both models, a medicament of such compound is expected to stabilize the disruptive mood-change between manic and depressive state.

### Test 1: Test with maximum electroshock seizure (MES) model

Test 1 evaluates the anticonvulsant action of medicaments. Animal model used herein represents the clinical phenotypes of generalized tonic seizure and secondarily generalized tonic-clonic seizure. Test compounds (50 or 100 mg/kg) were orally administered to Slc: ddY male mice (3 mice in each group, body weight 20-30 g). After an hour of administration, their corneas were electrically stimulated (60 Hz, 25 mA, 0.2 second) to induce tonic convulsion (tonic extensor in their hind limbs). Then, the mice were observed to investigate whether extension of their hind limbs was inhibited or not. To the control mice, 0.5 % tragacanth solution or 0.5 % methylcellulose solution was administered. The results are shown in Table 23.

### Test 2: Test with subcutaneously pentetrazol-injected model (scPTZ)

Similar to Test 1, Test 2 evaluates the anticonvulsant action of medicaments. However, unlike Test 1, animal model used herein represents the clinical phenotypes of generalized absence seizure and myoclonic seizure. Test compounds (50 or 100 mg/kg) were orally administered to Slc: ddY male mice (3 mice in each group, body weight 20-30 g). After an hour, pentetrazol (85 mg/kg) was subcutaneously administered. Then, the mice were observed for 30 minutes to investigate whether clonic seizure was developed or not. To the control mice, 0.5 % tragacanth solution or 0.5 % methylcellulose solution was administered. The results are shown in Table 23.

**Table 23**

| Ex. | MES (positive number/ tested number) | | scPTZ (positive number / tested number) | |
|---|---|---|---|---|
| | 100 mg/kg | 50 mg/kg | 100 mg/kg | 50 mg/kg |
| 1 | 3/3 | 3/3 | 2/3 | 2/3 |
| 2 | 3/3 | 3/3 | 0/3 | - |
| 3 | 3/3 | 3/3 | 1/3 | - |
| 4 | 2/3 | 0/3 | 2/3 | 0/3 |
| 5 | 3/3 | 0/3 | 2/3 | 1/3 |
| 6 | 3/3 | 0/3 | 1/3 | - |
| 7 | 3/3 | 2/3 | 2/3 | 0/3 |
| 8 | 3/3 | 0/3 | 2/3 | 0/3 |
| 9 | 1/3 | - | 2/3 | 0/3 |
| 10 | 3/3 | 1/3 | 1/3 | - |
| 11 | 3/3 | 0/3 | 1/3 | - |
| 12 | 3/3 | 0/3 | 1/3 | - |
| 13 | 3/3 | 2/3 | 2/3 | 3/3 |
| 14 | 3/3 | 0/3 | 0/3 | - |
| 15 | 2/3 | 0/3 | 0/3 | - |
| 16 | 3/3 | 3/3 | 3/3 | 2/3 |
| 17 | 0/3 | - | 1/3 | - |
| 18 | 3/3 | 0/3 | 1/3 | - |
| 19 | 2/3 | 1/3 | 0/3 | - |
| 20 | 3/3 | 2/3 | 2/3 | 2/3 |
| 21 | 3/3 | 1/3 | 3/3 | 0/3 |
| 22 | 3/3 | 2/3 | 1/3 | - |
| 23 | 3/3 | 3/3 | 1/3 | - |
| 24 | 3/3 | 1/3 | 0/3 | - |
| 25 | 3/3 | 3/3 | 3/3 | 3/3 |
| 26 | 3/3 | 3/3 | 0/3 | - |
| 27 | 3/3 | 0/3 | 1/3 | - |
| 28 | 3/3 | 0/3 | 2/3 | 0/3 |
| 29 | 2/3 | 0/3 | 1/3 | - |
| 30 | 3/3 | 0/3 | 2/3 | 1/3 |
| 31 | 0/3 | - | 2/3 | 1/3 |
| 32 | 3/3 | 1/3 | 0/3 | - |
| 33 | 2/3 | 1/3 | 1/3 | - |
| 34 | 0/3 | - | 2/3 | 1/3 |
| 35 | 2/3 | 0/3 | 0/3 | - |
| 36 | 3/3 | 0/3 | 1/3 | - |
| 37 | 0/3 | - | 1/3 | - |
| 38 | 0/3 | - | 3/3 | 0/3 |
| 39 | 3/3 | 3/3 | 2/3 | 1/3 |
| 40 | 3/3 | 1/3 | 0/3 | - |
| 41 | 3/3 | 3/3 | 2/3 | 2/3 |
| 42 | 1/3 | - | 2/3 | 1/3 |
| 43 | 2/3 | 2/3 | 2/3 | 1/3 |
| 44 | 3/3 | 3/3 | 0/3 | - |
| 45 | 3/3 | 3/3 | 0/3 | - |
| 46 | 3/3 | 2/3 | 3/3 | 0/3 |
| 47 | 3/3 | 3/3 | 0/3 | - |
| 48 | 3/3 | 1/3 | 3/3 | 1/3 |
| 49 | 2/3 | 0/3 | 2/3 | 0/3 |
| 50 | 3/3 | 2/3 | 1/3 | - |
| 51 | 3/3 | 0/3 | 1/3 | - |
| 52 | 3/3 | 2/3 | 2/3 | 1/3 |
| 53 | 3/3 | 2/3 | 0/3 | - |
| 54 | 3/3 | 3/3 | 1/3 | - |
| 55 | 3/3 | 0/3 | 0/3 | - |
| 56 | 3/3 | 0/3 | 2/3 | 3/3 |
| 57 | 2/3 | 2/3 | 2/3 | 2/3 |
| 58 | 2/3 | 0/3 | 2/3 | 0/3 |
| 59 | 3/3 | 3/3 | 2/3 | 0/3 |
| 60 | 3/3 | 3/3 | 2/3 | 1/3 |
| 61 | 2/3 | 0/3 | 3/3 | 0/3 |
| 62 | 3/3 | 2/3 | 1/3 | - |
| 63 | 2/3 | 0/3 | 2/3 | 1/3 |
| 64 | 3/3 | 3/3 | 3/3 | 0/3 |
| 65 | 2/3 | - | 1/3 | - |
| 66 | 2/3 | - | 1/3 | - |
| 67 | 2/3 | 2/3 | 2/3 | 2/3 |
| 68 | 3/3 | 0/3 | 2/3 | 0/3 |
| 69 | 1/3 | - | 2/3 | - |
| 70 | 3/3 | 1/3 | 2/3 | 2/3 |
| 71 | 3/3 | 2/3 | 2/3 | 2/3 |
| 72 | 2/3 | - | 0/3 | - |
| 73 | 3/3 | 1/3 | 3/3 | 0/3 |
| 74 | 2/3 | - | 1/3 | - |
| 75 | 3/3 | 0/3 | 2/3 | 1/3 |
| 76 | 3/3 | 0/3 | 2/3 | 1/3 |
| 77 | 0/3 | - | 2/3 | - |
| 78 | 3/3 | 3/3 | 2/3 | 0/3 |
| 79 | 3/3 | 1/3 | 3/3 | 1/3 |
| 80 | 3/3 | 3/3 | 3/3 | 2/3 |
| 81 | 1/3 | - | 0/3 | - |
| 82 | 1/3 | - | 3/3 | - |
| 83 | 2/3 | 0/3 | 2/3 | 1/3 |
| 84 | 2/3 | 0/3 | 2/3 | 1/3 |
| 85 | 3/3 | - | 1/3 | - |
| 86 | 2/3 | 3/3 | 2/3 | 1/3 |
| 87 | 3/3 | 1/3 | 2/3 | 0/3 |
| 88 | 3/3 | 2/3 | 3/3 | 2/3 |
| 89 | 3/3 | 0/3 | 2/3 | 1/3 |
| 90 | 2/3 | 2/3 | 2/3 | 2/3 |
| 91 | 3/3 | 3/3 | 2/3 | 2/3 |
| 92 | 0/3 | - | 1/3 | - |
| 93 | 3/3 | 3/3 | 3/3 | 1/3 |
| 94 | - | - | 1/3 | - |
| 95 | 2/3 | 1/3 | 3/3 | 0/3 |
| 96 | 1/3 | - | 2/3 | - |
| 97 | 2/3 | 1/3 | 1/3 | - |
| 98 | 2/3 | 0/3 | 0/3 | - |
| 99 | 3/3 | 2/3 | 1/3 | - |
| 100 | 0/3 | - | 3/3 | - |
| 101 | 3/3 | - | 0/3 | - |
| 102 | 0/3 | - | 2/3 | - |
| 103 | 3/3 | - | 1/3 | - |
| 104 | 3/3 | - | 0/3 | - |
| 105 | 0/3 | - | 2/3 | - |
| 106 | 0/3 | - | 2/3 | - |
| 107 | 0/3 | - | 3/3 | - |
| 108 | 0/3 | - | 2/3 | - |
| 109 | 3/3 | 3/3 | 3/3 | 2/3 |
| 110 | 2/3 | 0/3 | 1/3 | - |
| 111 | 0/3 | - | 2/3 | 1/3 |
| 112 | 1/3 | - | 2/3 | 1/3 |
| 113 | 3/3 | 2/3 | 1/3 | - |
| 114 | 3/3 | 3/3 | 1/3 | - |
| 115 | 3/3 | 3/3 | 0/3 | - |
| 116 | 0/3 | - | 2/3 | 1/3 |
| 117 | 0/3 | - | 2/3 | 1/3 |
| 118 | 3/3 | 3/3 | 1/3 | - |
| 119 | 3/3 | 3/3 | 0/3 | - |
| 120 | 3/3 | 0/3 | 1/3 | - |
| 121 | 3/3 | 0/3 | 1/3 | - |
| 122 | 0/3 | - | 2/3 | 0/3 |
| 123 | 3/3 | - | 1/3 | - |
| 124 | 3/3 | 0/3 | 3/3 | 0/3 |
| 125 | 3/3 | 2/3 | 2/3 | 1/3 |
| 126 | 2/3 | 0/3 | 3/3 | 2/3 |
| 127 | 2/3 | - | 1/3 | - |
| 128 | 2/3 | - | 0/3 | - |
| 129 | 3/3 | - | 1/3 | - |
| 130 | 3/3 | - | 0/3 | - |
| 131 | 3/3 | - | 0/3 | - |
| 132 | 3/3 | - | 0/3 | - |
| 133 | 3/3 | - | 1/3 | - |
| 134 | 3/3 | - | 0/3 | - |
| 135 | 3/3 | - | 0/3 | - |
| 136 | 3/3 | - | 0/3 | - |
| 137 | 0/3 | - | 2/3 | - |
| 138 | 0/3 | - | 2/3 | - |
| 139 | 2/3 | - | 1/3 | - |
| 140 | 3/3 | 3/3 | 2/3 | 2/3 |
| 141 | 2/3 | - | 0/3 | - |
| 142 | 2/3 | 1/3 | 2/3 | 3/3 |
| 143 | 0/3 | - | 3/3 | - |
| 144 | 1/3 | - | 2/3 | - |
| 145 | 0/3 | - | 1/3 | - |
| 146 | 2/3 | - | 0/3 | - |
| 147 | 3/3 | - | 1/3 | - |
| 148 | 0/3 | - | 1/3 | - |
| 149 | 2/3 | 1/3 | 2/3 | 0/3 |
| 150 | 3/3 | 3/3 | 3/3 | 1/3 |
| 151 | 2/3 | - | 0/3 | - |
| 152 | 3/3 | 3/3 | 3/3 | 2/3 |
| 153 | 2/3 | 0/3 | 3/3 | 0/3 |
| 154 | 3/3 | 1/3 | 1/3 | - |
| 155 | 0/3 | - | 2/3 | 0/3 |
| 156 | 3/3 | 0/3 | 1/3 | - |
| 157 | 3/3 | 1/3 | 2/3 | 2/3 |
| 158 | 0/3 | - | 2/3 | - |
| 159 | 2/3 | 1/3 | 2/3 | 1/3 |
| 160 | 2/3 | 1/3 | 3/3 | 2/3 |
| 161 | 2/3 | - | 0/3 | - |
| 162 | 3/3 | 3/3 | 3/3 | 3/3 |
| 163 | 3/3 | 1/3 | 0/3 | - |
| 164 | 3/3 | 3/3 | 1/3 | - |
| 165 | 3/3 | 2/3 | 1/3 | - |
| 166 | 3/3 | 2/3 | 2/3 | 1/3 |
| 167 | 3/3 | 1/3 | 3/3 | 2/3 |
| 168 | 3/3 | 1/3 | 1/3 | - |
| 169 | 3/3 | - | 1/3 | - |
| 170 | 3/3 | 0/3 | 2/3 | 1/3 |
| 171 | 3/3 | 3/3 | 3/3 | 1/3 |
| 172 | 3/3 | - | 0/3 | - |
| 173 | 1/3 | - | 0/3 | - |
| 174 | 3/3 | 3/3 | 2/3 | 1/3 |
| 175 | 2/3 | 0/3 | 3/3 | 0/3 |
| 176 | 1/3 | - | 0/3 | - |
| 177 | 0/3 | - | 1/3 | - |
| 178 | 3/3 | 2/3 | 3/3 | 3/3 |
| 179 | 3/3 | 1/3 | 1/3 | - |
| 180 | 3/3 | 1/3 | 1/3 | - |
| 181 | 3/3 | 1/3 | 2/3 | 0/3 |
| 182 | 3/3 | 1/3 | 3/3 | 0/3 |
| 183 | 3/3 | 3/3 | 2/3 | 2/3 |
| 184 | 3/3 | 2/3 | 2/3 | 1/3 |
| 185 | 3/3 | 2/3 | 3/3 | 2/3 |
| 186 | 3/3 | 3/3 | 2/3 | 2/3 |
| 187 | 3/3 | - | 0/3 | - |
| 188 | 3/3 | 1/3 | 2/3 | 0/3 |
| 189 | 2/3 | 3/3 | 2/3 | 1/3 |
| 190 | 3/3 | 2/3 | 2/3 | 2/3 |
| 191 | 3/3 | 3/3 | 3/3 | 1/3 |
| 192 | 3/3 | - | 1/3 | - |
| 193 | 3/3 | 3/3 | 2/3 | 3/3 |
| 194 | 3/3 | 1/3 | 2/3 | 1/3 |
| 195 | 3/3 | - | 1/3 | - |
| 199 | 3/3 | 1/3 | 1/3 | - |
| 200 | 3/3 | 0/3 | 2/3 | 1/3 |
| 201 | 2/3 | 1/3 | 2/3 | 1/3 |
| 202 | 2/3 | 1/3 | 3/3 | 0/3 |
| 203 | 2/3 | 1/3 | 3/3 | 2/3 |
| 204 | 3/3 | 0/3 | 0/3 | - |
| 205 | 3/3 | 0/3 | 2/3 | 1/3 |
| 206 | 2/3 | 1/3 | 2/3 | 0/3 |
| 207 | 3/3 | 2/3 | 2/3 | 2/3 |
| 208 | 3/3 | 2/3 | 2/3 | 1/3 |
| 209 | 3/3 | 3/3 | 2/3 | 2/3 |
| 210 | 3/3 | 2/3 | 3/3 | 3/3 |
| 211 | 3/3 | 3/3 | 2/3 | 2/3 |
| 212 | 3/3 | 3/3 | 2/3 | 2/3 |
| 213 | 3/3 | 0/3 | 1/3 | - |
| 214 | 3/3 | 2/3 | 1/3 | - |
| 215 | 2/3 | 2/3 | 2/3 | 0/3 |
| 216 | 2/3 | 1/3 | 1/3 | 1/3 |
| 217 | - | 2/3 | 3/3 | 1/3 |
| 218 | 3/3 | 1/3 | 2/3 | 1/3 |
| 219 | 3/3 | 3/3 | 3/3 | 0/3 |
| 220 | 3/3 | 3/3 | 3/3 | 1/3 |

As shown in Table 23, the compounds of the present invention exhibit an anticonvulsant action through oral administration in maximum electroshock seizure (MES) model tests and/or subcutaneously pentetrazol-injected model (scPTZ) tests. In particular, an anticonvulsant action was observed in both tests for the compounds of Examples 1, 13, 16, 20, 25, 41, 57, 67, 71, 80, 88, 90, 91, 109, 140, 152, 162, 178, 183, 185, 186, 190, 207, 209, 210, 211 and 212.

### Test 3: Test with amygdala-kindling model in rats

Test 3 evaluates the anticonvulsant action of medicaments in rats, besides mice in Tests 1 and 2. Animal model (kindling model) used herein is a very similar model to clinical findings of focal onset seizures (i.e. simple partial seizure, complex partial seizure, and secondarily generalized tonic-clonic seizure), which is known to provide excellent clinical prediction. A chronic electrode was implanted in the cortex (frontal and posterior regions) and the amygdala of Slc: Wistar male rats (body weight 250-300 g). One week after the above operation, electrical stimulation (50 Hz, 400 pA, 1 second) into the amygdala was conducted once a day for about two weeks. The stimulation condition was determined according to the method of Loscher et al. [Epilepsy Res., 40: 63-77 (2000)]. The stimulation was continued until the seizure stage 5 in the seizure severity stages according to the method of Racine et al. [Motor seizure. Electroenceph. Clin. Neurophysiol., 32:281-294(1972)] was observed 10 consecutive times. Kindling model was thus completed. Then, test compounds (100 mg/kg) were orally administered to this model. After an hour of administration, the rats were evaluated based on three parameters, i.e., a threshold stimulus current to induce seizure (i.e. seizure threshold), the duration of afterdischrage observed on an electroencephalogram, and seizure severity (stage scores 1-5). The results of the seizure threshold and the afterdischarge duration were shown as average percentage of change from the pretreatment value obtained before the administration of each test compound. To the control rats, 0.5 % tragacanth solution was administered. The results are shown in Table 24. Minus means decreased or shortened.

**Table 24**

| Ex. (100mg/kg) (n) | Seizure threshold (uA) Δ % Change | Afterdischarge duration (sec) Δ % change | Seizure severity score |
|---|---|---|---|
| Control (6) | 0.0 | -5.5 | 5.0 |
| 1 (2) | 69.3 | -88 | 2.0 |
| 25(2) | 69.4 | -100 | 0.0 |

As shown in Table 24, the compounds of Examples 1 and 25 exhibit potent efficacy against the amygdala-kindling model by oral administration at a dose of 100 mg/kg.

### Test 4: Test of 6 Hz psychomotor seizure model

Similar to Tests 1 and 2, Test 4 evaluates the anticonvulsant action of medicaments. However, unlike Tests 1 and 2, the animal model used herein represents clinical phenotypes of seizures resistant to existing antiepileptic drugs. Each test compound (100 mg/kg) was orally administered to Slc: ddY male mouse (5 mice in each group, body weight 20-30 g). After 30 minutes or an hour of administration, their corneas were electrically stimulated (6 Hz, 32 mA, 3 seconds) to induce symptoms of myoclonus in their forelimbs, Straub tail, and immobility. Then, the mice were observed to investigate whether all these symptoms were inhibited or not. To the control mice, 0.5 % tragacanth solution, 0.5 % methylcellulose solution, or olive oil was administered. The results are shown in Table 25.

**Table 25**

| Ex. | 6 Hz (Positive number/ tested number/dose) | ED₅₀ |
|---|---|---|
| 1 | 10/10/100 mg | 56.2 |
| 13 | 2/10/100 mg | - |
| 16 | 8/10/50 mg | 38.1 |
| 25 | 10/10/100 mg | 61.9 |
| 39 | 1/5/100 mg | - |
| 41 | 4/5/100 mg | - |
| 46 | 5/10/100 mg | 120 |
| 50 | 3/5/100 mg | - |
| 52 | 1/5/100 mg | - |
| 59 | 7/10/100 mg | 91.5 |
| 60 | 1/5/100 mg | - |
| 64 | 3/5/100 mg | - |
| 71 | 7/10/100 mg | 82.8 |
| 78 | 3/5/100 mg | - |
| 80 | 4/5/100 mg | - |
| 86 | 2/5/100 mg | - |
| 88 | 3/5/100 mg | - |
| 91 | 5/5/100 mg | - |
| 93 | 2/5/100 mg | - |
| 178 | 5/10/100 mg | 117 |
| 183 | 4/5/100 mg | - |
| 185 | 5/5/100 mg | - |
| 189 | 3/5/100 mg | - |
| 190 | 5/5/100 mg | - |
| 207 | 7/10/100 mg | 86.6 |
| 208 | 7/10/100 mg | 90.6 |
| 209 | 6/10/100 mg | 109 |
| 210 | 4/5/100 mg | - |
| 211 | 5/10/100 mg | 100 |
| 212 | 1/5/100 mg | - |
| 215 | 7/10/100 mg | 64.8 |

As shown in Table 25, the compounds of Examples 1, 25, 91, 185, 190 and 207 exhibit potent efficacy in 6 Hz psychomotor seizure model by oral administration at a dose of 100 mg/kg.

### Test 5: Rotarod performance test

Test 5 evaluates whether motor disturbance is caused by medicaments. On the day before the test, Slc: ddY male mice (body weight 20-30 g) were trained so that they could walk on a rotarod apparatus for 3 minutes without falling, wherein a cylinder of 4 cm diameter is rotated with a speed of 13 rotations per minute. Test compounds were orally administered to a group consisting of 10 mice. After an hour of administration, the mice were put on the rotarod apparatus and their walking performance were observed for 100 seconds. When the mice fell down within 100 seconds because of disturbed motor coordination, such mice were counted as positive. To the control mice, 0.5 % methylcellulose solution was administered. The results are shown in Table 26.

**Table 26**

| Ex. | The rate of mice which fell within 100 seconds (%) | | | |
|---|---|---|---|---|
| | 100 mg/kg | 200 mg/kg | 300 mg/kg | 400 mg/kg |
| 25 | 0 | 10 | 60 | 90 |
| 46 | 20 | 20 | 60 | 90 |
| 71 | 0 | 20 | 30 | 70 |

### Test 6: Evaluation of antidepressant effect

Test 6 is a typical test to evaluate the antidepressive activity of medicaments (Porsolt et al., Nature, 266: 730-732, 1977, Porsolt et al., Eur. J. Pharmacol., 47: 379- 391, 1978). Crl: CD (SD) male rats (12 rats in each group, body weight 200-250 g) were forced to swim for 15 minutes in a water pool with 200 mm diameter. In the pool, water was filled up to a depth of 205 mm from the bottom of the pool, and thus the rats could not touch the bottom of the pool with their hind limbs. Fifteen minutes after the completion of the forced swimming, test compounds were orally administered to the rats. On the next day, the test compounds were administered to the rats at the same dose as the previous day, and the rats were put in the pool for 5 minutes at the same clock time as the previous day. The duration of immobility (i.e. period which the rats did not swim) was observed for each rat. The antidepressant effect was evaluated by calculating how long the duration of immobility was shortened against the control. To the control, 0.5 % methylcellulose solution was administered. Note that in the same conditions as above, the duration of immobility was dose-dependently shortened by Lamotrigine which is known as a medicament efficacious for treating bipolar disorder. The results are shown in Table 27.

**Table 27**

| Ex. | The rate of the shortened duration of immobility (% control) | |
|---|---|---|
| | 10 mg/kg | 30 mg/kg |
| 1 | 23.4 | 24.9 |
| 16 | 19.9 | 21.9 |

### Test 7: Evaluation of antimanic effect

Test 7 evaluates the antimanic effect of medicaments. The evaluation system is a test system whose utility has been already confirmed with a known antimanic agent which is clinically used as a mood-stabilizing drug, not as a drug for treating schizophrenia (Arban et al., Behav. Brain Res., 158:123-132, 2005, Foreman et al., Pharmacol. Biochem. Behav., 89: 523-534, 2008). Test compounds were orally administered to Slc: C57BL/6J male mice (8 mice in each group, body weight 20-26 g). After 30 minutes of administration, methamphetamine (4 mg/kg) and chlordiazepoxide (10 mg/kg) were administered intraperitoneally. Then, the mice were allowed to move freely in an acrylic cage (W 360 mm x D 230 mm x H 310 mm) for 90 minutes. The movement distances of the mice were measured with Ethovision software (Ver. 3.1). Among the 90 minutes, the result of the latter 60 minutes with the exception of the first 30 minute's result for adaptation to the new environment was used as data of the movement distance. The antimanic effect was evaluated by calculating how long movement distance was shortened against the control. To the control mice, 0.5 % methylcellulose solution was administered. Note that in the same conditions as above, the movement distance was dose-dependently shortened by Lamotrigine which is known as a medicament clinically efficacious for treating bipolar disorder. The results are shown in Table 28.

**Table 28**

| Ex. | The rate of the shortened movement-distance (% control) | | |
|---|---|---|---|
| | 30 mg/kg | 60 mg/kg | 100 mg/kg |
| 1 | 24.1 | 43.8 | 73.8 |
| 16 | 45.7 | 69.1 | 84.9 |

### Formulation Example 1: Formulation of tablet

By a conventional method, the compound of Example 1 (250 g), corn starch (54 g), carboxymethylcellulose calcium (40 g), microcrystalline cellulose (50 g) and magnesium stearate (6 g) are mixed, granulated, and compressed into a tablet of 400 mg to provide 1000 tablets thereof.

### Formulation Example 2: Formulation of powder

By a conventional method, the compound of Example 1 (500 g), lactose (470 g), hydroxypropylcellulose (25 g) and light anhydrous silicic acid (5 g) are mixed to prepare powders.

### INDUSTRIAL APPLICABILITY

As described above, the biphenylacetamide derivatives of the present invention exhibit a strong anticonvulsant action in all evaluation models, i.e., maximum electro shock seizure (MES) model which is a representative model to evaluate an antiepileptic drug, subcutaneously pentetrazol-injected model (scPTZ), 6 Hz psychomotor seizure model, and amygdala-kindling model in rats. The compounds of the present invention are therefore useful as an antiepileptic drug to prevent and/or treat diseases such as partial seizures (e.g. simple partial seizure, complex partial seizure, and secondarily generalized tonic-clonic seizure) and generalized seizures (e.g. absence seizure, myoclonic seizure, clonic seizure, tonic seizure, tonic-clonic seizure, atonic seizure, west syndrome, and Lennox-Gastaut syndrome). Furthermore, the compounds of the present invention are expected to prevent and/or treat intractable epilepsy which has been hardly cured by conventional medicaments yet. Moreover, the compounds of the present invention exhibit the efficacy in model systems to evaluate both antidepressant effect and antimanic effect, and thereby the compounds of the present invention are also useful as an antidepressive agent, an antimanic agent, and/or a medicament for treating and/or preventing bipolar disorder.

## Claims

1. A compound of the following formula (A): 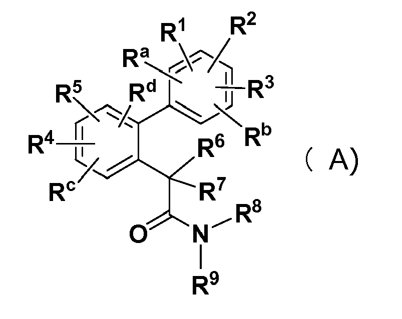 or a hydrate or a solvate thereof wherein
R¹ R² R³, R^{a} and R^{b} are each bound to any one different carbon atom having a replaceable hydrogen atom in the benzene ring to which they are attached, and are independently selected from the group consisting of hydrogen atom, fluorine atom, chlorine atom, bromine atom, C₁₋₆ alkyl, C₁₋₆ alkoxy substituted with 1-3 fluorine atoms, C₁₋₆ alkyl-S(O)ₙ- and cyano, wherein said alkyl and alkylS(O)ₙ- may be substituted with 1-5 fluorine atoms, provided that when any two of R¹, R², R³, R^{a} and R^{b} are each 2'-methyl and 3'-methyl, at least one of the other substituents is other than hydrogen atom,
R⁴, R⁵, R^{c} and R^{d} are each bound to any one different carbon atom having a replaceable hydrogen atom in the benzene ring to which they are attached, and are independently selected from the group consisting of hydrogen atom, fluorine atom, chlorine atom, C₁₋₆ alkyl, C₁₋₆ alkoxy substituted with 1-3 fluorine atoms, C₁₋₆ alkylS(O)ₙ-, cyano and nitro, wherein said alkyl and alkyl - S(O)ₙ- may be substituted with 1-5 fluorine atoms,
R⁶ and R⁷ are independently selected from the group consisting of hydrogen atom, fluorine atom, methyl, ethyl, hydroxy group and C₁₋₆ alkoxy, provided that when one of R⁶ and R⁷ is hydroxy group, the other is not fluorine atom, hydroxy group or C₁₋₆ alkoxy, or R⁶ and R⁷ may be combined to form C₃₋₆ cycloalkyl with the carbon atom which they are attached to, wherein said methyl, ethyl, alkoxy and cycloalkyl may be substituted with 1-5 fluorine atoms,
R⁸ and R⁹ are independently selected from the group consisting of hydrogen atom, C₁₋₆ alkyl, C₃₋₇ cycloalkyl-C₁₋₃ alkyl and C₃₋₆ cycloalkyl, wherein said alkyl, cycloalkyl-alkyl and cycloalkyl may be substituted with 1-5 fluorine atoms,
n is an integer of 0 to 2,
provided that when R¹ R², R³, R⁴, R⁵, R^{a}, R^{b}, R^{c} and R^{d} are all hydrogen atoms, and R⁶ and R⁷ are both hydrogen atoms, at least one of R⁸ and R⁹ is hydrogen atom, and
when R¹, R², R³, R⁴, R⁵, R^{a}, R^{b}, R^{c} and R^{d} are all hydrogen atoms, and at least one of R⁶ and R⁷ is alkoxy, R⁸ and R⁹ are independently selected from the group consisting of hydrogen atom, C₁₋₃ alkyl, C₃₋₇ cycloalkyl-C₁₋₃ alkyl and C₃₋₆ cycloalkyl.

2. The compound of claim 1 or a hydrate or a solvate thereof wherein R^{a}, R^{b}, R^{c} and R^{d} are all hydrogen atoms.

3. The compound of claim 2 or a hydrate or a solvate thereof wherein R⁸ and R⁹ are independently selected from the group consisting of hydrogen atom, C₁₋₃ alkyl, C₃₋₇ cycloalkyl-C₁₋₃ alkyl and C₃₋₆ cycloalkyl, wherein said alkyl, cycloalkyl-alkyl and cycloalkyl may be substituted with 1-5 fluorine atoms, provided that when R¹, R², R³, R⁴ and R⁵ are all hydrogen atoms, at least one of R⁸ and R⁹ is hydrogen atom.

4. The compound of claim 2 or claim 3, or a hydrate or a solvate thereof wherein R¹, R² and R³ are independently selected from the group consisting of hydrogen atom, fluorine atom, chlorine atom, bromine atom, trifluoromethyl, trifluoromethoxy, C₁₋₃ alkyl-S(O)ₙ- and cyano, wherein said alkyl-S(O)ₙ- may be substituted with 1-5 fluorine atoms.

5. The compound of claim 2 or claim 3, or a hydrate or a solvate thereof wherein R¹, R² and R³ are independently selected from the group consisting of hydrogen atom, fluorine atom, chlorine atom, bromine atom, trifluoromethyl and trifluoromethoxy.

6. The compound of any one of claims 2 to 5 or a hydrate or a solvate thereof wherein R⁹ is hydrogen atom.

7. The compound of any one of claims 2 to 6 or a hydrate or a solvate thereof wherein R⁴ and R⁵ are independently selected from the group consisting of hydrogen atom, fluorine atom, chlorine atom, C₁₋₃ alkyl, trifluoromethyl and trifluoromethoxy.

8. The compound of any one of claims 2 to 7 or a hydrate or a solvate thereof wherein R⁶ and R⁷ are independently selected from the group consisting of hydrogen atom, methyl, ethyl, hydroxy group and C₁₋₃ alkoxy, provided that when one of R⁶ and R⁷ is hydroxy group, the other is not hydroxy group or C₁₋₃ alkoxy.

9. The compound of claim 2 or a hydrate or a solvate thereof wherein
R¹, R² and R³ are independently selected from the group consisting of hydrogen atom, fluorine atom, chlorine atom, bromine atom, trifluoromethyl and trifluoromethoxy,
R⁴ and R⁵ are independently selected from the group consisting of hydrogen atom, fluorine atom, chlorine atom, trifluoromethyl and trifluoromethoxy,
R⁶ and R⁷ are independently selected from the group consisting of hydrogen atom, methyl, hydroxy group and methoxy provided that when one of R⁶ and R⁷ is hydroxy group, the other is not hydroxy group or methoxy,
R⁸ is hydrogen atom, methyl or ethyl, and
R⁹ is hydrogen atom.

10. The compound of claim 2 or claim 9, or a hydrate or a solvate thereof wherein R¹, R², R³, R⁴ and R⁵ are not all hydrogen atom at the same time.

11. The compound of claim 2 or a hydrate or solvate thereof wherein the compound is selected from:
N,N-dimethyl-2-[4'-(trifluoromethyl)biphenyl-2-yl]-acetamide,
2-[3'-(trifluoromethyl)biphenyl-2-yl]acetamide,
N-methyl-2-[3'-(trifluoromethyl)biphenyl-2-yl]-acetamide,
N-methyl-2-[2'-(trifluoromethyl)biphenyl-2-yl]-acetamide,
N-ethyl-2-[2'-(trifluoromethyl)biphenyl-2-yl]acetamide,
2-(3',4'-difluorobiphenyl-2-yl)acetamide,
2-(4'-fluorobiphenyl-2-yl)acetamide,
2-(4'-fluorobiphenyl-2-yl)-N-methylacetamide,
2-(3'-fluorobiphenyl-2-yl)-N-methylacetamide,
2-(2'-fluorobiphenyl-2-yl)-N-methylacetamide,
2-(2'-fluorobiphenyl-2-yl)-N,N-dimethylacetamide,
N-methyl-2-(3'-methylbiphenyl-2-yl)-acetamide,
2-(5-fluorobiphenyl-2-yl)acetamide,
2-(4-fluorobiphenyl-2-yl)-N-methylacetamide,
2-(2',4-difluorobiphenyl-2-yl)acetamide,
2-(2',4-difluorobiphenyl-2-yl)-N-methylacetamide,
2-(3'-chloro-4-fluorobiphenyl-2-yl)acetamide,
2-(4'-bromobiphenyl-2-yl)acetamide,
2-(biphenyl-2-yl)acetamide,
2-(3'-bromobiphenyl-2-yl)acetamide,
2-(4'-bromobiphenyl-2-yl)-N-methylacetamide,
2-(biphenyl-2-yl)-N-methylacetamide,
2-(biphenyl-2-yl)propanamide,
2-(2'-fluorobiphenyl-2-yl)-N-methyl-propanamide,
2-(biphenyl-2-yl)-2-hydroxyacetamide,
2-(2'-fluorobiphenyl-2-yl)-2-hydroxyacetamide,
2-(biphenyl-2-yl)-2-methoxyacetamide,
2-(biphenyl-2-yl)-2-methoxy-N-methylacetamide,
2-(biphenyl-2-yl)-2-methoxy-N,N-dimethylacetamide,
2-(2',3',5'-trichlorobiphenyl-2-yl)acetamide,
2-(4'-chloro-2'-fluorobiphenyl-2-yl)acetamide,
2-[3'-fluoro-4'-(trifluoromethoxy)biphenyl-2-yl]-acetamide,
2-[3'-fluoro-5'-(trifluoromethyl)biphenyl-2-yl]-acetamide,
2-[4'-chloro-3'-(trifluoromethyl)biphenyl-2-yl]-acetamide,
2-[4'-(trifluoromethoxy)biphenyl-2-yl]acetamide,
2-(2',4'-dichlorobiphenyl-2-yl)acetamide,
2-[3'-(trifluoromethoxy)biphenyl-2-yl]acetamide,
2-[2'-fluoro-5'-(trifluoromethyl)biphenyl-2-yl]-acetamide,
2-[2'-chloro-4'-(trifluoromethyl)biphenyl-2-yl]-acetamide,
2-(2'-chloro-3'-fluorobiphenyl-2-yl)acetamide,
2-(5'-chloro-2'-fluorobiphenyl-2-yl)acetamide,
2-(2',3',4'-trifluorobiphenyl-2-yl)acetamide,
2-[2'-fluoro-5'-(trifluoromethoxy)biphenyl-2-yl]-acetamide,
2-(2',5'-dichlorobiphenyl-2-yl)acetamide,
N-methyl-2-[3'-(trifluoromethoxy)biphenyl-2-yl]-acetamide,
2-(2'-fluoro-5-nitrobiphenyl-2-yl)acetamide,
2-(3-fluorobiphenyl-2-yl)acetamide,
2-(3-fluorobiphenyl-2-yl)-N-methylacetamide,
2-(5-chlorobiphenyl-2-yl)-N-methylacetamide,
2-(5-chloro-2'-fluorobiphenyl-2-yl)acetamide,
2-(5-chloro-2'-fluorobiphenyl-2-yl)-N-methylacetamide,
2-[4-chloro-4'-(trifluoromethyl)biphenyl-2-yl]-acetamide,
2-[3'-fluoro-5-(trifluoromethyl)biphenyl-2-yl]-acetamide,
2-(3',5'-dichloro-5-fluorobiphenyl-2-yl)acetamide,
2-(3',5'-difluorobiphenyl-2-yl)acetamide,
1-(2'-fluorobiphenyl-2-yl)-N-methyl-cyclopropanecarboxamide,
1-(3'-fluorobiphenyl-2-yl)-N-methyl-cyclopropanecarboxamide,
2-fluoro-2-(3'-fluorobiphenyl-2-yl)acetamide,
2-fluoro-2-(2'-fluorobiphenyl-2-yl)-N-methylacetamide,
2-fluoro-2-(4'-fluorobiphenyl-2-yl)-N-methylacetamide,
2-(3'-chlorobiphenyl-2-yl)-2-fluoroacetamide,
2,2-difluoro-2-(3'-fluorobiphenyl-2-yl)acetamide,
2-(3'-chlorobiphenyl-2-yl)-2,2-difluoroacetamide,
2-(biphenyl-2-yl)-2,2-difluoroacetamide,
2-(3'-fluorobiphenyl-2-yl)propanamide,
2-(5-fluorobiphenyl-2-yl)-2-hydroxyacetamide,
2-(3',5-difluorobiphenyl-2-yl)-2-hydroxyacetamide,
2-(3',5'-dichloro-5-fluorobiphenyl-2-yl)-2-hydroxy-acetamide,
2-(3',4-difluorobiphenyl-2-yl)-2-hydroxyacetamide,
2-(3',5'-dichloro-4-fluorobiphenyl-2-yl)-2-hydroxy-acetamide,
2-(5'-chloro-2'-fluorobiphenyl-2-yl)-2-hydroxy-acetamide,
2-(2'-fluorobiphenyl-2-yl)-2-methoxyacetamide,
2-(2'-fluorobiphenyl-2-yl)-2-methoxy-N-methylacetamide,
2-(2'-fluorobiphenyl-2-yl)-2-methoxy-N,N-dimethylacetamide,
2-(4'-fluorobiphenyl-2-yl)-2-methoxyacetamide,
2-(4'-fluorobiphenyl-2-yl)-2-methoxy-N,N-dimethylacetamide, or
2-(3'-chlorobiphenyl-2-yl)-2-methoxyacetamide.

12. The compound of claim 2 or a hydrate or solvate thereof wherein the compound is selected from:
2-(2'-fluorobiphenyl-2-yl)acetamide,
N-methyl-2-[4'-(trifluoromethyl)biphenyl-2-yl]-acetamide,
2-(3'-chlorobiphenyl-2-yl)acetamide,
2-(2'-chlorobiphenyl-2-yl)acetamide,
2-(2',3'-difluorobiphenyl-2-yl)acetamide,
2-(3'-fluorobiphenyl-2-yl)acetamide,
2-(2',5-difluorobiphenyl-2-yl)acetamide,
2-(4-fluorobiphenyl-2-yl)acetamide,
2-(biphenyl-2-yl)-N-methylpropanamide,
2-(biphenyl-2-yl)-2-hydroxyacetamide,
2-(2'-fluorobiphenyl-2-yl)-2-hydroxyacetamide,
2-(2',3',5'-trifluorobiphenyl-2-yl)acetamide,
2-(3',5'-dichlorobiphenyl-2-yl)acetamide,
2-(3'-chloro-5'-fluorobiphenyl-2-yl)acetamide,
2-(2'-chloro-5'-fluorobiphenyl-2-yl)acetamide,
2-(3',4',5'-trifluorobiphenyl-2-yl)acetamide,
2-[5'-chloro-2'-(trifluoromethyl)biphenyl-2-yl]-acetamide,
2-(4,4'-dichlorobiphenyl-2-yl)acetamide,
2-(4,4'-difluorobiphenyl-2-yl)acetamide,
2-(4,4'-difluorobiphenyl-2-yl)-N-methylacetamide,
2-(3,3'-difluorobiphenyl-2-yl)acetamide,
2-(2',5'-difluorobiphenyl-2-yl)acetamide,
2-(biphenyl-2-yl)-2-fluoroacetamide,
2-fluoro-2-(2'-fluorobiphenyl-2-yl)acetamide,
2-(3'-fluorobiphenyl-2-yl)-2-hydroxyacetamide,
2-(3',5'-dichlorobiphenyl-2-yl)-2-hydroxyacetamide,
2-(2'-chloro-5'-fluorobiphenyl-2-yl)-2-hydroxy-acetamide,
2-(4-fluorobiphenyl-2-yl)-2-hydroxyacetamide,
2-(2'-chlorobiphenyl-2-yl)-2-hydroxyacetamide,
2-(3'-chloro-5'-fluorobiphenyl-2-yl)-2-hydroxy-acetamide, or
2-hydroxy-2-(3',4',5'-trifluorobiphenyl-2-yl)acetamide.

13. A pharmaceutical composition comprising the compound of any one of claims 1-12 or a hydrate or a solvate thereof, and a pharmaceutically acceptable carrier.

14. An antiepileptic drug comprising the compound of any one of claims 1-12 or a hydrate or a solvate thereof as an active ingredient.

15. A mood-stabilizing drug for bipolar disorder comprising the compound of any one of claims 1-12 or a hydrate or a solvate thereof as an active ingredient.

16. Use of the compound of any one of claims 1-12 or a hydrate or a solvate thereof for the preparation of an antiepileptic drug.
